# EUROPEAN PATENT APPLICATION

(11) **EP 2 083 079 A1**
(43) Date of publication of application: **29.07.2009**
(21) Application number: 09000133.0
(22) Date of filing: 12.06.1998
(51) Int. Cl.: C12N 15/12, C12N 15/62, C12N 5/12, C07K 14/715, C07K 16/28, A01K 67/027, A61K 38/17, G01N 33/50

(54) **Apo-2DcR**

(30) Priority: 18.06.1997 US 878168
(62) Divisional of application: 98931285.5
(71) Applicant: Genentech, Inc., South San Francisco CA 94080-4990 (US)
(72) Inventor: Ashkenazi, Avi J., San Mateo, CA 94401 (US); Baker, Kevin P., Burlingame, CA 94010 (US); Chuntharapai, Anan, Colma, CA 94015 (US); Gurney, Austin, San Francisco, CA 94114 (US); Kim, Kyung Jin, Los Altos, CA 94024 (US); Wood, William I., San Mateo, CA 94402 (US)
(74) Representative: Kiddle, Simon John

(57) **Abstract**

Novel polypeptides, designated Apo-2DcR, which are capable of binding Apo-2 ligand are provided. Compositions including Apo-2DcR chimeras, nucleic acid encoding Apo-2DcR, and antibodies to Apo-2DcR are also provided.

SEQUENCE LISTING

<110>  Genentech, Inc.

<120>  Apo-2DcR

<130>  SJK/FP6607055

<140>  EP 09000133.0
<141>  2009-01-15

<150>  EP 98931285.5
<151>  1998-06-12

<150>  PCT/US1998/12456
<151>  1998-06-12

<150>  US 08/878,168
<151>  1997-06-18

<160>  19

<170>  PatentIn version 3.3

<210>  1
<211>  259
<212>  PRT
<213>  Homo sapiens

<400>  1

Met Ala Arg Ile Pro Lys Thr Leu Lys Phe Val Val Val Ile Val Ala
1               5                   10                  15

Val Leu Leu Pro Val Leu Ala Tyr Ser Ala Thr Thr Ala Arg Gln Glu
            20                  25                  30

Glu Val Pro Gln Gln Thr Val Ala Pro Gln Gln Gln Arg His Ser Phe
        35                  40                  45

Lys Gly Glu Glu Cys Pro Ala Gly Ser His Arg Ser Glu His Thr Gly
    50                  55                  60

Ala Cys Asn Pro Cys Thr Glu Gly Val Asp Tyr Thr Asn Ala Ser Asn
65                  70                  75                  80

Asn Glu Pro Ser Cys Phe Pro Cys Thr Val Cys Lys Ser Asp Gln Lys
                85                  90                  95

His Lys Ser Ser Cys Thr Met Thr Arg Asp Thr Val Cys Gln Cys Lys
            100                 105                 110

Glu Gly Thr Phe Arg Asn Glu Asn Ser Pro Glu Met Cys Arg Lys Cys
        115                 120                 125

Ser Arg Cys Pro Ser Gly Glu Val Gln Val Ser Asn Cys Thr Ser Trp
    130                 135                 140

Asp Asp Ile Gln Cys Val Glu Glu Phe Gly Ala Asn Ala Thr Val Glu
145                 150                 155                 160

Thr Pro Ala Ala Glu Glu Thr Met Asn Thr Ser Pro Gly Thr Pro Ala
                165                 170                 175

Pro Ala Ala Glu Glu Thr Met Asn Thr Ser Pro Gly Thr Pro Ala Pro
            180                 185                 190

Ala Ala Glu Glu Thr Met Thr Thr Ser Pro Gly Thr Pro Ala Pro Ala
        195                 200                 205

Ala Glu Glu Thr Met Thr Thr Ser Pro Gly Thr Pro Ala Pro Ala Ala
    210                 215                 220

Glu Glu Thr Met Thr Thr Ser Pro Gly Thr Pro Ala Ser Ser His Tyr
225                 230                 235                 240

Leu Ser Cys Thr Ile Val Gly Ile Ile Val Leu Ile Val Leu Leu Ile
                245                 250                 255

Val Phe Val

<210>  2
<211>  1180
<212>  DNA
<213>  Homo sapiens

<220>
<221>  CDS
<222>  (193)..(972)

<400>  2
gctgtgggaa cctctccacg cgcacgaact cagccaacga tttctgatag atttttggga      60

gtttgaccag agatgcaagg ggtgaaggag cgcttcctac cgttagggaa ctctggggac     120

agagcgcccc ggccgcctga tggccgaggc agggtgcgac ccaggaccca ggacggcgtc     180

gggaaccata cc atg gcc cgg atc ccc aag acc cta aag ttc gtc gtc gtc     231
              Met Ala Arg Ile Pro Lys Thr Leu Lys Phe Val Val Val
              1               5                   10

atc gtc gcg gtc ctg ctg cca gtc cta gct tac tct gcc acc act gcc 279
Ile Val Ala Val Leu Leu Pro Val Leu Ala Tyr Ser Ala Thr Thr Ala
    15                  20                  25

cgg cag gag gaa gtt ccc cag cag aca gtg gcc cca cag caa cag agg 327
Arg Gln Glu Glu Val Pro Gln Gln Thr Val Ala Pro Gln Gln Gln Arg
30                  35                  40                  45

cac agc ttc aag ggg gag gag tgt cca gca gga tct cat aga tca gaa 375
His Ser Phe Lys Gly Glu Glu Cys Pro Ala Gly Ser His Arg Ser Glu
                50                  55                  60

cat act gga gcc tgt aac ccg tgc aca gag ggt gtg gat tac acc aac 423
His Thr Gly Ala Cys Asn Pro Cys Thr Glu Gly Val Asp Tyr Thr Asn
            65                  70                  75

gct tcc aac aat gaa cct tct tgc ttc cca tgt aca gtt tgt aaa tca 471
Ala Ser Asn Asn Glu Pro Ser Cys Phe Pro Cys Thr Val Cys Lys Ser
        80                  85                  90

gat caa aaa cat aaa agt tcc tgc acc atg acc aga gac aca gtg tgt 519
Asp Gln Lys His Lys Ser Ser Cys Thr Met Thr Arg Asp Thr Val Cys
    95                  100                 105

cag tgt aaa gaa ggc acc ttc cgg aat gaa aac tcc cca gag atg tgc 567
Gln Cys Lys Glu Gly Thr Phe Arg Asn Glu Asn Ser Pro Glu Met Cys
110                 115                 120                 125

cgg aag tgt agc agg tgc cct agt ggg gaa gtc caa gtc agt aat tgt 615
Arg Lys Cys Ser Arg Cys Pro Ser Gly Glu Val Gln Val Ser Asn Cys
                130                 135                 140

acg tcc tgg gat gat atc cag tgt gtt gaa gaa ttt ggt gcc aat gcc 663
Thr Ser Trp Asp Asp Ile Gln Cys Val Glu Glu Phe Gly Ala Asn Ala
            145                 150                 155

act gtg gaa acc cca gct gct gaa gag aca atg aac acc agc ccg ggg 711
Thr Val Glu Thr Pro Ala Ala Glu Glu Thr Met Asn Thr Ser Pro Gly
        160                 165                 170

act cct gcc cca gct gct gaa gag aca atg aac acc agc cca ggg act     759
Thr Pro Ala Pro Ala Ala Glu Glu Thr Met Asn Thr Ser Pro Gly Thr
    175                 180                 185

cct gcc cca gct gct gaa gag aca atg acc acc agc ccg ggg act cct     807
Pro Ala Pro Ala Ala Glu Glu Thr Met Thr Thr Ser Pro Gly Thr Pro
190                 195                 200                 205

gcc cca gct gct gaa gag aca atg acc acc agc ccg ggg act cct gcc     855
Ala Pro Ala Ala Glu Glu Thr Met Thr Thr Ser Pro Gly Thr Pro Ala
                210                 215                 220

cca gct gct gaa gag aca atg acc acc agc ccg ggg act cct gcc tct     903
Pro Ala Ala Glu Glu Thr Met Thr Thr Ser Pro Gly Thr Pro Ala Ser
            225                 230                 235

tct cat tac ctc tca tgc acc atc gta ggg atc ata gtt cta att gtg     951
Ser His Tyr Leu Ser Cys Thr Ile Val Gly Ile Ile Val Leu Ile Val
        240                 245                 250

ctt ctg att gtg ttt gtt tga aagacttcac tgtggaagaa attccttcct     1002
Leu Leu Ile Val Phe Val
    255

tacctgaaag gttcaggtag gcgctggctg agggcggggg gcgctggaca ctctctgccc     1062

tgcctccctc tgctgtgttc ccacagacag aaacgcctgc ccctgcccca aaaaaaaaaa     1122

aaaaaaaaaa aaaaaaaaaa aaaaaaaaaa aaaaaaaaaa aaaaaaaaaa aaaaaaaa     1180

<210>  3
<211>  299
<212>  PRT
<213>  Homo sapiens

<400>  3

<210> 4
<211> 1180
<212> DNA
<213> Homo sapiens

<220>
<221> CDS
<222> (73)..(969)

<220>
<221> mat_peptide
<222> (193)..(969)

<400> 4
gctgtgggaa cctctccacg cgcacgaact cagccaacga tttctgatag atttttggga 60
gtttgaccag ag atg caa ggg gtg aag gag cgc ttc cta ccg tta ggg aac 111
tct ggg gac aga gcg ccc cgg ccg cct gat ggc cga ggc agg gtg cga 159
ccc agg acc cag gac ggc gtc ggg aac cat acc atg gcc cgg atc ccc 207
aag acc cta aag ttc gtc gtc gtc atc gtc gcg gtc ctg ctg cca gtc 255
cta gct tac tct gcc acc act gcc cgg cag gag gaa gtt ccc cag cag 303
aca gtg gcc cca cag caa cag agg cac agc ttc aag ggg gag gag tgt 351
cca gca gga tct cat aga tca gaa cat act gga gcc tgt aac ccg tgc 399
aca gag ggt gtg gat tac acc aac gct tcc aac aat gaa cct tct tgc 447
ttc cca tgt aca gtt tgt aaa tca gat caa aaa cat aaa agt tcc tgc 495
acc atg acc aga gac aca gtg tgt cag tgt aaa gaa ggc acc ttc cgg 543
aat gaa aac tcc cca gag atg tgc cgg aag tgt agc agg tgc cct agt 591
ggg gaa gtc caa gtc agt aat tgt acg tcc tgg gat gat atc cag tgt 639
gtt gaa gaa ttt ggt gcc aat gcc act gtg gaa acc cca gct gct gaa 687
gag aca atg aac acc agc ccg ggg act cct gcc cca gct gct gaa gag 735
aca atg aac acc agc cca ggg act cct gcc cca gct gct gaa gag aca 783
atg acc acc agc ccg ggg act cct gcc cca gct gct gaa gag aca atg 831
acc acc agc ccg ggg act cct gcc cca gct gct gaa gag aca atg acc 879
acc agc ccg ggg act cct gcc tct tct cat tac ctc tca tgc acc atc 927
gta ggg atc ata gtt cta att gtg ctt ctg att gtg ttt gtt 969
tgaaagactt cactgtggaa gaaattcctt ccttacctga aaggttcagg taggcgctgg 1029
ctgagggcgg ggggcgctgg acactctctg ccctgcctcc ctctgctgtg ttcccacaga 1089
cagaaacgcc tgcccctgcc ccaaaaaaaa aaaaaaaaaa aaaaaaaaaa aaaaaaaaaa 1149
aaaaaaaaaa aaaaaaaaaa aaaaaaaaaa a 1180

<210> 5
<211> 43
<212> DNA
<213> Artificial sequence

<220>
<221> source
<223> /note= "Description of artificial sequence: Forward
oligonucleotide 1"

<400> 5
tgtaaaacga cggccagtta aatagacctg caattattaa tct 43

<210> 6
<211> 41
<212> DNA
<213> Artificial sequence

<220>
<221> source
<223> /note= "Description of artificial sequence: Reverse
      oligonucleotide 2"

<400> 6
caggaaacag ctatgaccac ctgcacacct gcaaatccat t                41

<210> 7
<211> 49
<212> PRT
<213> Homo sapiens

<400> 7

Cys Arg Glu Cys Glu Ser Gly Ser Phe Thr Ala Ser Glu Asn His Leu
1               5                   10                  15

Arg His Cys Leu Ser Cys Ser Lys Cys Arg Lys Glu Met Gly Gln Val
            20                  25                  30

Glu Ile Ser Ser Cys Thr Val Asp Arg Asp Thr Val Cys Gly Cys Arg
        35                  40                  45

Lys

<210> 8
<211> 48
<212> PRT
<213> Homo sapiens

<400> 8

Cys Asn Pro Cys Thr Glu Gly Val Asp Tyr Thr Asn Ala Ser Asn Asn
1               5                   10                  15

Glu Pro Ser Cys Phe Pro Cys Thr Val Cys Lys Ser Asp Gln Lys His
            20                  25                  30

Lys Ser Ser Cys Thr Met Thr Arg Asp Thr Val Cys Gln Cys Lys Glu
        35                  40                  45

<210> 9
<211> 70
<212> DNA
<213> Artificial sequence

<220>
<221> source
<223> /note= "Description of artificial sequence: Synthetic
      oligonucleotide probe"

<400> 9
gggagccgct catgaggaag ttgggcctca tggacaatga gataaaggtg gctaaagctg      60

aggcagcggg                                                              70

<210> 10
<211> 1799
<212> DNA
<213> Homo sapiens

<220>
<221> CDS
<222> (140)..(1375)

<220>
<221> misc_feature
<222> 1367..1369
<223> wtg translates as Xaa, which stands for Met, or Leu.

<400> 10
cccacgcgtc cgcataaatc agcacgcggc cggagaaccc cgcaatctct gcgcccacaa 60

aatacaccga cgatgcccga tctactttaa gggctgaaac ccacgggcct gagagactat 120

aagagcgttc cctaccgcc atg gaa caa cgg gga cag aac gcc ccg gcc gct 172
Met Glu Gln Arg Gly Gln Asn Ala Pro Ala Ala
1               5                   10

tcg ggg gcc cgg aaa agg cac ggc cca gga ccc agg gag gcg cgg gga 220
Ser Gly Ala Arg Lys Arg His Gly Pro Gly Pro Arg Glu Ala Arg Gly
            15                  20                  25

gcc agg cct ggg ctc cgg gtc ccc aag acc ctt gtg ctc gtt gtc gcc 268
Ala Arg Pro Gly Leu Arg Val Pro Lys Thr Leu Val Leu Val Val Ala
        30                  35                  40

gcg gtc ctg ctg ttg gtc tca gct gag tct gct ctg atc acc caa caa 316
Ala Val Leu Leu Leu Val Ser Ala Glu Ser Ala Leu Ile Thr Gln Gln
    45                  50                  55

gac cta gct ccc cag cag aga gcg gcc cca caa caa aag agg tcc agc 364
Asp Leu Ala Pro Gln Gln Arg Ala Ala Pro Gln Gln Lys Arg Ser Ser
60                  65                  70                  75

ccc tca gag gga ttg tgt cca cct gga cac cat atc tca gaa gac ggt 412
Pro Ser Glu Gly Leu Cys Pro Pro Gly His His Ile Ser Glu Asp Gly
                80                  85                  90

aga gat tgc atc tcc tgc aaa tat gga cag gac tat agc act cac tgg 460
Arg Asp Cys Ile Ser Cys Lys Tyr Gly Gln Asp Tyr Ser Thr His Trp
            95                  100                 105

aat gac ctc ctt ttc tgc ttg cgc tgc acc agg tgt gat tca ggt gaa 508
Asn Asp Leu Leu Phe Cys Leu Arg Cys Thr Arg Cys Asp Ser Gly Glu
        110                 115                 120

gtg gag cta agt ccc tgc acc acg acc aga aac aca gtg tgt cag tgc 556
Val Glu Leu Ser Pro Cys Thr Thr Thr Arg Asn Thr Val Cys Gln Cys
    125                 130                 135

gaa gaa ggc acc ttc cgg gaa gaa gat tct cct gag atg tgc cgg aag 604
Glu Glu Gly Thr Phe Arg Glu Glu Asp Ser Pro Glu Met Cys Arg Lys
140                 145                 150                 155

tgc cgc aca ggg tgt ccc aga ggg atg gtc aag gtc ggt gat tgt aca 652
Cys Arg Thr Gly Cys Pro Arg Gly Met Val Lys Val Gly Asp Cys Thr
                160                 165                 170

ccc tgg agt gac atc gaa tgt gtc cac aaa gaa tca ggc atc atc ata 700
Pro Trp Ser Asp Ile Glu Cys Val His Lys Glu Ser Gly Ile Ile Ile
            175                 180                 185

gga gtc aca gtt gca gcc gta gtc ttg att gtg gct gtg ttt gtt tgc 748
Gly Val Thr Val Ala Ala Val Val Leu Ile Val Ala Val Phe Val Cys
        190                 195                 200

aag tct tta ctg tgg aag aaa gtc ctt cct tac ctg aaa ggc atc tgc 796
Lys Ser Leu Leu Trp Lys Lys Val Leu Pro Tyr Leu Lys Gly Ile Cys
    205                 210                 215

tca ggt ggt ggt ggg gac cct gag cgt gtg gac aga agc tca caa cga 844
Ser Gly Gly Gly Gly Asp Pro Glu Arg Val Asp Arg Ser Ser Gln Arg
220                 225                 230                 235

cct ggg gct gag gac aat gtc ctc aat gag atc gtg agt atc ttg cag 892
Pro Gly Ala Glu Asp Asn Val Leu Asn Glu Ile Val Ser Ile Leu Gln
                240                 245                 250

ccc acc cag gtc cct gag cag gaa atg gaa gtc cag gag cca gca gag 940
Pro Thr Gln Val Pro Glu Gln Glu Met Glu Val Gln Glu Pro Ala Glu
            255                 260                 265

cca aca ggt gtc aac atg ttg tcc ccc ggg gag tca gag cat ctg ctg 988
Pro Thr Gly Val Asn Met Leu Ser Pro Gly Glu Ser Glu His Leu Leu
        270                 275                 280

gaa ccg gca gaa gct gaa agg tct cag agg agg agg ctg ctg gtt cca 1036
Glu Pro Ala Glu Ala Glu Arg Ser Gln Arg Arg Arg Leu Leu Val Pro
    285                 290                 295

gca aat gaa ggt gat ccc act gag act ctg aga cag tgc ttc gat gac 1084
Ala Asn Glu Gly Asp Pro Thr Glu Thr Leu Arg Gln Cys Phe Asp Asp
300                 305                 310                 315

ttt gca gac ttg gtg ccc ttt gac tcc tgg gag ccg ctc atg agg aag 1132
Phe Ala Asp Leu Val Pro Phe Asp Ser Trp Glu Pro Leu Met Arg Lys
                320                 325                 330

ttg ggc ctc atg gac aat gag ata aag gtg gct aaa gct gag gca gcg 1180
Leu Gly Leu Met Asp Asn Glu Ile Lys Val Ala Lys Ala Glu Ala Ala
            335                 340                 345

ggc cac agg gac acc ttg tac acg atg ctg ata aag tgg gtc aac aaa 1228
Gly His Arg Asp Thr Leu Tyr Thr Met Leu Ile Lys Trp Val Asn Lys
        350                 355                 360

acc ggg cga gat gcc tct gtc cac acc ctg ctg gat gcc ttg gag acg 1276
Thr Gly Arg Asp Ala Ser Val His Thr Leu Leu Asp Ala Leu Glu Thr
    365                 370                 375

ctg gga gag aga ctt gcc aag cag aag att gag gac cac ttg ttg agc 1324
Leu Gly Glu Arg Leu Ala Lys Gln Lys Ile Glu Asp His Leu Leu Ser
380                 385                 390                 395

tct gga aag ttc atg tat cta gaa ggt aat gca gac tct gcc wtg tcc     1372
Ser Gly Lys Phe Met Tyr Leu Glu Gly Asn Ala Asp Ser Ala Xaa Ser
                400                 405                 410

taa gtgtgattct cttcaggaag tgagaccttc cctggtttac cttttttctg          1425

gaaaaagccc aactggactc cagtcagtag gaaagtgcca caattgtcac atgaccggta   1485

ctggaagaaa ctctcccatc caacatcacc cagtggatgg aacatcctgt aacttttcac   1545

tgcacttggc attattttta taagctgaat gtgataataa ggacactatg gaaatgtctg   1605

gatcattccg tttgtgcgta ctttgagatt tggtttggga tgtcattgtt ttcacagcac   1665

ttttttatcc taatgtaaat gctttattta tttatttggg ctacattgta agatccatct   1725

acaaaaaaaa aaaaaaaaaa aaaagggcgg ccgcgactct agagtcgacc tgcagaagct   1785

tggccgccat ggcc                                                    1799

<210>  11
<211>  411
<212>  PRT
<213>  Homo sapiens

<220>
<221>  misc_feature
<222>  (410)..(410)
<223>  The 'Xaa' at location 410 stands for Met, or Leu.

<400>  11

Met Glu Gln Arg Gly Gln Asn Ala Pro Ala Ala Ser Gly Ala Arg Lys
1               5                   10                  15

Arg His Gly Pro Gly Pro Arg Glu Ala Arg Gly Ala Arg Pro Gly Leu
            20                  25                  30

Arg Val Pro Lys Thr Leu Val Leu Val Val Ala Ala Val Leu Leu Leu
        35                  40                  45

Val Ser Ala Glu Ser Ala Leu Ile Thr Gln Gln Asp Leu Ala Pro Gln
    50                  55                  60

Gln Arg Ala Ala Pro Gln Gln Lys Arg Ser Ser Pro Ser Glu Gly Leu
65                  70                  75                  80

Cys Pro Pro Gly His His Ile Ser Glu Asp Gly Arg Asp Cys Ile Ser
                85                  90                  95

Cys Lys Tyr Gly Gln Asp Tyr Ser Thr His Trp Asn Asp Leu Leu Phe
            100                 105                 110

Cys Leu Arg Cys Thr Arg Cys Asp Ser Gly Glu Val Glu Leu Ser Pro
        115                 120                 125

Cys Thr Thr Thr Arg Asn Thr Val Cys Gln Cys Glu Glu Gly Thr Phe
    130                 135                 140

Arg Glu Glu Asp Ser Pro Glu Met Cys Arg Lys Cys Arg Thr Gly Cys
145                 150                 155                 160

Pro Arg Gly Met Val Lys Val Gly Asp Cys Thr Pro Trp Ser Asp Ile
                165                 170                 175

Glu Cys Val His Lys Glu Ser Gly Ile Ile Ile Gly Val Thr Val Ala
            180                 185                 190

Ala Val Val Leu Ile Val Ala Val Phe Val Cys Lys Ser Leu Leu Trp
        195                 200                 205

Lys Lys Val Leu Pro Tyr Leu Lys Gly Ile Cys Ser Gly Gly Gly Gly
    210                 215                 220

Asp Pro Glu Arg Val Asp Arg Ser Ser Gln Arg Pro Gly Ala Glu Asp
225                 230                 235                 240

Asn Val Leu Asn Glu Ile Val Ser Ile Leu Gln Pro Thr Gln Val Pro
                245                 250                 255

Glu Gln Glu Met Glu Val Gln Glu Pro Ala Glu Pro Thr Gly Val Asn
            260                 265                 270

Met Leu Ser Pro Gly Glu Ser Glu His Leu Leu Glu Pro Ala Glu Ala
        275                 280                 285

Glu Arg Ser Gln Arg Arg Arg Leu Leu Val Pro Ala Asn Glu Gly Asp
    290                 295                 300

Pro Thr Glu Thr Leu Arg Gln Cys Phe Asp Asp Phe Ala Asp Leu Val
305                 310                 315                 320

Pro Phe Asp Ser Trp Glu Pro Leu Met Arg Lys Leu Gly Leu Met Asp
                325                 330                 335

Asn Glu Ile Lys Val Ala Lys Ala Glu Ala Ala Gly His Arg Asp Thr
            340                 345                 350

Leu Tyr Thr Met Leu Ile Lys Trp Val Asn Lys Thr Gly Arg Asp Ala
        355                 360                 365

Ser Val His Thr Leu Leu Asp Ala Leu Glu Thr Leu Gly Glu Arg Leu
    370                 375                 380

Ala Lys Gln Lys Ile Glu Asp His Leu Leu Ser Ser Gly Lys Phe Met
385                 390                 395                 400

Tyr Leu Glu Gly Asn Ala Asp Ser Ala Xaa Ser
                405                 410

<210>  12
<211>  29
<212>  DNA
<213>  Artificial sequence

<220>
<221>  source
<223>  /note= "Description of artificial sequence: Synthetic
       oligonucleotide probe"

<400>  12
atcagggact ttccgctggg gactttccg                                    29

<210>  13
<211>  30
<212>  DNA
<213>  Artificial sequence

<220>
<221>  source
<223>  /note= "Description of artificial sequence: Synthetic
       oligonucleotide probe"

<400>  13
aggatgggaa gtgtgtgata tatccttgat                                   30

<210> 14
<211> 1180
<212> DNA
<213> Homo sapiens

<400> 14
tttttttttt tttttttttt tttttttttt tttttttttt tttttttttt tttttttttt 60

tttttttttg gggcaggggc aggcgtttct gtctgtggga acacagcaga gggaggcagg 120

gcagagagtg tccagcgccc cccgccctca gccagcgcct acctgaacct ttcaggtaag 180

gaaggaattt cttccacagt gaagtctttc aaacaaacac aatcagaagc acaattagaa 240

ctatgatccc tacgatggtg catgagaggt aatgagaaga ggcaggagtc cccgggctgg 300

tggtcattgt ctcttcagca gctggggcag gagtccccgg gctggtggtc attgtctctt 360

cagcagctgg ggcaggagtc cccgggctgg tggtcattgt ctcttcagca gctggggcag 420

gagtccctgg gctggtgttc attgtctctt cagcagctgg ggcaggagtc cccgggctgg 480

tgttcattgt ctcttcagca gctggggttt ccacagtggc attggcacca aattcttcaa 540

cacactggat atcatcccag gacgtacaat tactgacttg gacttcccca ctagggcacc 600

tgctacactt ccggcacatc tctggggagt tttcattccg gaaggtgcct tctttacact 660

gacacactgt gtctctggtc atggtgcagg aacttttatg tttttgatct gatttacaaa 720

ctgtacatgg gaagcaagaa ggttcattgt tggaagcgtt ggtgtaatcc acaccctctg 780

tgcacgggtt acaggctcca gtatgttctg atctatgaga tcctgctgga cactcctccc 840

ccttgaagct gtgcctctgt tgctgtgggg ccactgtctg ctggggaact tcctcctgcc 900

gggcagtggt ggcagagtaa gctaggactg gcagcaggac cgcgacgatg acgacgacga 960

actttagggt cttggggatc cgggccatgg tatggttccc gacgccgtcc tgggtcctgg 1020

gtcgcaccct gcctcggcca tcaggcggcc ggggcgctct gtccccagag ttccctaacg 1080

gtaggaagcg ctccttcacc ccttgcatct ctggtcaaac tcccaaaaat ctatcagaaa 1140

tcgttggctg agttcgtgcg cgtggagagg ttcccacagc 1180

<210> 15
<211> 418
<212> PRT
<213> Homo sapiens

<400> 15

Gly Arg Gly Ala Leu Pro Thr Ser Met Gly Gln His Gly Pro Ser Ala
1               5                   10                  15

Arg Ala Arg Ala Gly Arg Ala Pro Gly Pro Arg Pro Ala Arg Glu Ala
            20                  25                  30

Ser Pro Arg Leu Arg Val His Lys Thr Phe Lys Phe Val Val Val Gly
        35                  40                  45

Val Leu Leu Gln Val Val Pro Ser Ser Ala Ala Thr Ile Lys Leu His
    50                  55                  60

Asp Gln Ser Ile Gly Thr Gln Gln Trp Glu His Ser Pro Leu Gly Glu
65                  70                  75                  80

Leu Cys Pro Pro Gly Ser His Arg Ser Glu His Pro Gly Ala Cys Asn
                85                  90                  95

Arg Cys Thr Glu Gly Val Gly Tyr Thr Asn Ala Ser Asn Asn Leu Phe
            100                 105                 110

Ala Cys Leu Pro Cys Thr Ala Cys Lys Ser Asp Glu Glu Glu Arg Ser
        115                 120                 125

Pro Cys Thr Thr Thr Arg Asn Thr Ala Cys Gln Cys Lys Pro Gly Thr
    130                 135                 140

Phe Arg Asn Asp Asn Ser Ala Glu Met Cys Arg Lys Cys Ser Arg Gly
145                 150                 155                 160

Cys Pro Arg Gly Met Val Lys Val Lys Asp Cys Thr Pro Trp Ser Asp
                165                 170                 175

Ile Glu Cys Val His Lys Glu Ser Gly Asn Gly His Asn Ile Trp Val
            180                 185                 190

Ile Leu Val Val Thr Leu Val Val Pro Leu Leu Leu Val Ala Val Leu
        195                 200                 205

Ile Val Cys Cys Cys Ile Gly Ser Gly Cys Gly Gly Asp Pro Lys Cys
    210                 215                 220

Met Asp Arg Val Cys Phe Trp Arg Leu Gly Leu Leu Arg Gly Pro Gly
225                 230                 235                 240

Ala Glu Asp Asn Ala His Asn Glu Ile Leu Ser Asn Ala Asp Ser Leu
                245                 250                 255

Ser Thr Phe Val Ser Glu Gln Gln Met Glu Ser Gln Glu Pro Ala Asp
            260                 265                 270

Leu Thr Gly Val Thr Val Gln Ser Pro Gly Glu Ala Gln Cys Leu Leu
        275                 280                 285

Gly Pro Ala Glu Ala Glu Gly Ser Gln Arg Arg Arg Leu Leu Val Pro
    290                 295                 300

Ala Asn Gly Ala Asp Pro Thr Glu Thr Leu Met Leu Phe Phe Asp Lys
305                 310                 315                 320

Phe Ala Asn Ile Val Pro Phe Asp Ser Trp Asp Gln Leu Met Arg Gln
                325                 330                 335

Leu Asp Leu Thr Lys Asn Glu Ile Asp Val Val Arg Ala Gly Thr Ala
            340                 345                 350

Gly Pro Gly Asp Ala Leu Tyr Ala Met Leu Met Lys Trp Val Asn Lys
        355                 360                 365

Thr Gly Arg Asn Ala Ser Ile His Thr Leu Leu Asp Ala Leu Glu Arg
    370                 375                 380

Met Glu Glu Arg His Ala Lys Glu Lys Ile Gln Asp Leu Leu Val Asp
385                 390                 395                 400

Ser Gly Lys Phe Ile Tyr Leu Glu Asp Gly Thr Gly Ser Ala Val Ser
                405                 410                 415

Leu Glu

<210>  16
<211>  74
<212>  PRT
<213>  Homo sapiens

<400>  16

Val Met Asp Ala Val Pro Ala Arg Arg Trp Lys Glu Phe Val Arg Thr
1               5                   10                  15

Leu Gly Leu Arg Glu Ala Glu Ile Glu Ala Val Glu Val Glu Ile Gly
            20                  25                  30

Arg Phe Arg Asp Gln Gln Tyr Glu Met Leu Lys Arg Trp Arg Gln Gln
        35                  40                  45

Gln Pro Ala Gly Leu Gly Ala Val Tyr Ala Ala Leu Glu Arg Met Gly
    50                  55                  60

Leu Asp Gly Cys Val Glu Asp Leu Arg Ser
65                  70

<210>  17
<211>  78
<212>  PRT
<213>  Homo sapiens

<400>  17

Val Val Glu Asn Val Pro Pro Leu Arg Trp Lys Glu Phe Val Arg Arg
1               5                   10                  15

Leu Gly Leu Ser Asp His Glu Ile Asp Arg Leu Glu Leu Gln Asn Gly

Arg Cys Leu Arg Glu Ala Gln Tyr Ser Met Leu Ala Thr Trp Arg Arg
        35                  40                  45

Arg Thr Pro Arg Arg Glu Ala Thr Leu Glu Leu Leu Gly Arg Val Leu
    50                  55                  60

Arg Asp Met Asp Leu Leu Gly Cys Leu Glu Asp Ile Glu Glu
65                  70                  75

<210>  18
<211>  77
<212>  PRT
<213>  Homo sapiens

<400>  18

Ile Ala Gly Val His Thr Leu Ser Gln Val Lys Gly Phe Val Arg Lys
1               5                   10                  15

Asn Gly Val Asn Glu Ala Lys Ile Asp Glu Ile Lys Asn Asp Asn Val
            20                  25                  30

Gln Asp Thr Ala Glu Gln Lys Val Gln Leu Leu Arg Asn Trp His Gln
        35                  40                  45

Leu His Gly Lys Lys Glu Ala Tyr Asp Thr Leu Ile Lys Asp Leu Lys
    50                  55                  60

Lys Ala Asn Leu Cys Thr Leu Ala Glu Lys Ile Gln Thr
65                  70                  75

<210> 19
<211> 1799
<212> DNA
<213> Homo sapiens

<400> 19
ggccatggcg gccaagcttc tgcaggtcga ctctagagtc gcggccgccc tttttttttt 60

tttttttttt ttgtagatgg atcttacaat gtagcccaaa taaataaata aagcatttac 120

attaggataa aaaagtgctg tgaaaacaat gacatcccaa accaaatctc aaagtacgca 180

caaacggaat gatccagaca tttccatagt gtccttatta tcacattcag cttataaaaa 240

taatgccaag tgcagtgaaa agttacagga tgttccatcc actgggtgat gttggatggg 300

agagtttctt ccagtaccgg tcatgtgaca attgtggcac tttcctactg actggagtcc 360

agttgggctt tttccagaaa aaaggtaaac cagggaaggt ctcacttcct gaagagaatc 420

acacttagga cawggcagag tctgcattac cttctagata catgaacttt ccagagctca 480

acaagtggtc ctcaatcttc tgcttggcaa gtctctctcc cagcgtctcc aaggcatcca 540

gcagggtgtg gacagaggca tctcgcccgg ttttgttgac ccactttatc agcatcgtgt 600

acaaggtgtc cctgtggccc gctgcctcag ctttagccac ctttatctca ttgtccatga 660

ggcccaactt cctcatgagc ggctcccagg agtcaaaggg caccaagtct gcaaagtcat 720

cgaagcactg tctcagagtc tcagtgggat caccttcatt tgctggaacc agcagcctcc 780

tcctctgaga cctttcagct tctgccggtt ccagcagatg ctctgactcc ccgggggaca 840

acatgttgac acctgttggc tctgctggct cctggacttc catttcctgc tcagggacct 900

gggtgggctg caagatactc acgatctcat tgaggacatt gtcctcagcc ccaggtcgtt 960

gtgagcttct gtccacacgc tcaggtcccc caccaccacc tgagcagatg cctttcaggt 1020

aaggaaggac tttcttccac agtaaagact tgcaaacaaa cacagccaca atcaagacta 1080

cggctgcaac tgtgactcct atgatgatgc ctgattcttt gtggacacat tcgatgtcac 1140

tccagggtgt acaatcaccg accttgacca tccctctggg acaccctgtg cggcacttcc 1200

ggcacatctc aggagaatct tcttcccgga aggtgccttc ttcgcactga cacactgtgt 1260

ttctggtcgt ggtgcaggga cttagctcca cttcacctga atcacacctg gtgcagcgca 1320

agcagaaaag gaggtcattc cagtgagtgc tatagtcctg tccatatttg caggagatgc 1380

aatctctacc gtcttctgag atatggtgtc caggtggaca caatccctct gaggggctgg 1440

acctcttttg ttgtggggcc gctctctgct ggggagctag gtcttgttgg gtgatcagag 1500

cagactcagc tgagaccaac agcaggaccg cggcgacaac gagcacaagg gtcttgggga 1560

cccggagccc aggcctggct ccccgcgcct ccctgggtcc tgggccgtgc cttttccggg 1620

cccccgaagc ggccggggcg ttctgtcccc gttgttccat ggcggtaggg aacgctctta 1680

tagtctctca ggcccgtggg tttcagccct taaagtagat cgggcatcgt cggtgtattt 1740

tgtgggcgca gagattgcgg ggttctccgg ccgcgtgctg atttatgcgg acgcgtggg 1799

SEQUENCE LISTING

<110>  Genentech, Inc.

<120>  Apo-2DcR

<130>  SJK/FP6607055

<140>  EP 09000133.0
<141>  2009-01-15

<150>  EP 98931285.5
<151>  1998-06-12

<150>  PCT/US1998/12456
<151>  1998-06-12

<150>  US 08/878,168
<151>  1997-06-18

<160>  19

<170>  PatentIn version 3.3

<210>  1
<211>  259
<212>  PRT
<213>  Homo sapiens

<400>  1

Met Ala Arg Ile Pro Lys Thr Leu Lys Phe Val Val Val Ile Val Ala
1               5                   10                  15

Val Leu Leu Pro Val Leu Ala Tyr Ser Ala Thr Thr Ala Arg Gln Glu
            20                  25                  30

Glu Val Pro Gln Gln Thr Val Ala Pro Gln Gln Gln Arg His Ser Phe
        35                  40                  45

Lys Gly Glu Glu Cys Pro Ala Gly Ser His Arg Ser Glu His Thr Gly
    50                  55                  60

Ala Cys Asn Pro Cys Thr Glu Gly Val Asp Tyr Thr Asn Ala Ser Asn
65                  70                  75                  80

Asn Glu Pro Ser Cys Phe Pro Cys Thr Val Cys Lys Ser Asp Gln Lys
                85                  90                  95

His Lys Ser Ser Cys Thr Met Thr Arg Asp Thr Val Cys Gln Cys Lys
            100                 105                 110

Glu Gly Thr Phe Arg Asn Glu Asn Ser Pro Glu Met Cys Arg Lys Cys
        115                 120                 125

Ser Arg Cys Pro Ser Gly Glu Val Gln Val Ser Asn Cys Thr Ser Trp
    130                 135                 140

Asp Asp Ile Gln Cys Val Glu Glu Phe Gly Ala Asn Ala Thr Val Glu
145                 150                 155                 160

Thr Pro Ala Ala Glu Glu Thr Met Asn Thr Ser Pro Gly Thr Pro Ala
                165                 170                 175

Pro Ala Ala Glu Glu Thr Met Asn Thr Ser Pro Gly Thr Pro Ala Pro
            180                 185                 190

Ala Ala Glu Glu Thr Met Thr Thr Ser Pro Gly Thr Pro Ala Pro Ala
        195                 200                 205

Ala Glu Glu Thr Met Thr Thr Ser Pro Gly Thr Pro Ala Pro Ala Ala
    210                 215                 220

Glu Glu Thr Met Thr Thr Ser Pro Gly Thr Pro Ala Ser Ser His Tyr
225                 230                 235                 240

Leu Ser Cys Thr Ile Val Gly Ile Ile Val Leu Ile Val Leu Leu Ile
                245                 250                 255

Val Phe Val

<210>  2
<211>  1180
<212>  DNA
<213>  Homo sapiens

<220>
<221>  CDS
<222>  (193)..(972)

<400>  2
gctgtgggaa cctctccacg cgcacgaact cagccaacga tttctgatag atttttggga      60

gtttgaccag agatgcaagg ggtgaaggag cgcttcctac cgttagggaa ctctggggac     120

agagcgcccc ggccgcctga tggccgaggc agggtgcgac ccaggaccca ggacggcgtc     180

gggaaccata cc atg gcc cgg atc ccc aag acc cta aag ttc gtc gtc gtc     231
              Met Ala Arg Ile Pro Lys Thr Leu Lys Phe Val Val Val
              1               5                   10

atc gtc gcg gtc ctg ctg cca gtc cta gct tac tct gcc acc act gcc     279
Ile Val Ala Val Leu Leu Pro Val Leu Ala Tyr Ser Ala Thr Thr Ala
    15                  20                  25

cgg cag gag gaa gtt ccc cag cag aca gtg gcc cca cag caa cag agg     327
Arg Gln Glu Glu Val Pro Gln Gln Thr Val Ala Pro Gln Gln Gln Arg
30                  35                  40                  45

cac agc ttc aag ggg gag gag tgt cca gca gga tct cat aga tca gaa     375
His Ser Phe Lys Gly Glu Glu Cys Pro Ala Gly Ser His Arg Ser Glu
                50                  55                  60

cat act gga gcc tgt aac ccg tgc aca gag ggt gtg gat tac acc aac     423
His Thr Gly Ala Cys Asn Pro Cys Thr Glu Gly Val Asp Tyr Thr Asn
            65                  70                  75

gct tcc aac aat gaa cct tct tgc ttc cca tgt aca gtt tgt aaa tca     471
Ala Ser Asn Asn Glu Pro Ser Cys Phe Pro Cys Thr Val Cys Lys Ser
        80                  85                  90

gat caa aaa cat aaa agt tcc tgc acc atg acc aga gac aca gtg tgt     519
Asp Gln Lys His Lys Ser Ser Cys Thr Met Thr Arg Asp Thr Val Cys
    95                  100                 105

cag tgt aaa gaa ggc acc ttc cgg aat gaa aac tcc cca gag atg tgc     567
Gln Cys Lys Glu Gly Thr Phe Arg Asn Glu Asn Ser Pro Glu Met Cys
110                 115                 120                 125

cgg aag tgt agc agg tgc cct agt ggg gaa gtc caa gtc agt aat tgt     615
Arg Lys Cys Ser Arg Cys Pro Ser Gly Glu Val Gln Val Ser Asn Cys
                130                 135                 140

acg tcc tgg gat gat atc cag tgt gtt gaa gaa ttt ggt gcc aat gcc     663
Thr Ser Trp Asp Asp Ile Gln Cys Val Glu Glu Phe Gly Ala Asn Ala
            145                 150                 155

act gtg gaa acc cca gct gct gaa gag aca atg aac acc agc ccg ggg     711
Thr Val Glu Thr Pro Ala Ala Glu Glu Thr Met Asn Thr Ser Pro Gly
        160                 165                 170

act cct gcc cca gct gct gaa gag aca atg aac acc agc cca ggg act     759
Thr Pro Ala Pro Ala Ala Glu Glu Thr Met Asn Thr Ser Pro Gly Thr
    175                 180                 185

cct gcc cca gct gct gaa gag aca atg acc acc agc ccg ggg act cct     807
Pro Ala Pro Ala Ala Glu Glu Thr Met Thr Thr Ser Pro Gly Thr Pro
190                 195                 200                 205

gcc cca gct gct gaa gag aca atg acc acc agc ccg ggg act cct gcc     855
Ala Pro Ala Ala Glu Glu Thr Met Thr Thr Ser Pro Gly Thr Pro Ala
                210                 215                 220

cca gct gct gaa gag aca atg acc acc agc ccg ggg act cct gcc tct     903
Pro Ala Ala Glu Glu Thr Met Thr Thr Ser Pro Gly Thr Pro Ala Ser
            225                 230                 235

tct cat tac ctc tca tgc acc atc gta ggg atc ata gtt cta att gtg     951
Ser His Tyr Leu Ser Cys Thr Ile Val Gly Ile Ile Val Leu Ile Val
        240                 245                 250

ctt ctg att gtg ttt gtt tga aagacttcac tgtggaagaa attccttcct       1002
Leu Leu Ile Val Phe Val
    255

tacctgaaag gttcaggtag gcgctggctg agggcggggg gcgctggaca ctctctgccc   1062

tgcctccctc tgctgtgttc ccacagacag aaacgcctgc ccctgcccca aaaaaaaaaa   1122

aaaaaaaaaa aaaaaaaaaa aaaaaaaaaa aaaaaaaaaa aaaaaaaaaa aaaaaaaa     1180


<210>  3
<211>  299
<212>  PRT
<213>  Homo sapiens

<400>  3

<210>  4
<211>  1180
<212>  DNA
<213>  Homo sapiens

<220>
<221>  CDS
<222>  (73)..(969)

<220>
<221>  mat_peptide
<222>  (193)..(969)

<400>  4
gctgtgggaa cctctccacg cgcacgaact cagccaacga tttctgatag atttttggga      60
gtttgaccag ag atg caa ggg gtg aag gag cgc ttc cta ccg tta ggg aac      111
tct ggg gac aga gcg ccc cgg ccg cct gat ggc cga ggc agg gtg cga      159
ccc agg acc cag gac ggc gtc ggg aac cat acc atg gcc cgg atc ccc      207
aag acc cta aag ttc gtc gtc gtc atc gtc gcg gtc ctg ctg cca gtc      255
cta gct tac tct gcc acc act gcc cgg cag gag gaa gtt ccc cag cag      303
aca gtg gcc cca cag caa cag agg cac agc ttc aag ggg gag gag tgt      351
cca gca gga tct cat aga tca gaa cat act gga gcc tgt aac ccg tgc 399
aca gag ggt gtg gat tac acc aac gct tcc aac aat gaa cct tct tgc 447
ttc cca tgt aca gtt tgt aaa tca gat caa aaa cat aaa agt tcc tgc 495
acc atg acc aga gac aca gtg tgt cag tgt aaa gaa ggc acc ttc cgg 543
aat gaa aac tcc cca gag atg tgc cgg aag tgt agc agg tgc cct agt 591
ggg gaa gtc caa gtc agt aat tgt acg tcc tgg gat gat atc cag tgt 639
gtt gaa gaa ttt ggt gcc aat gcc act gtg gaa acc cca gct gct gaa 687
gag aca atg aac acc agc ccg ggg act cct gcc cca gct gct gaa gag 735
aca atg aac acc agc cca ggg act cct gcc cca gct gct gaa gag aca 783
atg acc acc agc ccg ggg act cct gcc cca gct gct gaa gag aca atg 831
acc acc agc ccg ggg act cct gcc cca gct gct gaa gag aca atg acc      879
acc agc ccg ggg act cct gcc tct tct cat tac ctc tca tgc acc atc      927
gta ggg atc ata gtt cta att gtg ctt ctg att gtg ttt gtt              969
tgaaagactt cactgtggaa gaaattcctt ccttacctga aaggttcagg taggcgctgg   1029
ctgagggcgg ggggcgctgg acactctctg ccctgcctcc ctctgctgtg ttcccacaga   1089
cagaaacgcc tgcccctgcc ccaaaaaaaa aaaaaaaaaa aaaaaaaaaa aaaaaaaaaa   1149
aaaaaaaaaa aaaaaaaaaa aaaaaaaaaa a                                  1180

<210>  5
<211>  43
<212>  DNA
<213>  Artificial sequence

<220>
<221>  source
<223>  /note= "Description of artificial sequence: Forward
       oligonucleotide 1"

<400>  5
tgtaaaacga cggccagtta aatagacctg caattattaa tct                      43

<210>  6
<211>  41
<212>  DNA
<213>  Artificial sequence

<220>
<221>  source
<223>  /note= "Description of artificial sequence: Reverse
       oligonucleotide 2"

<400>  6
caggaaacag ctatgaccac ctgcacacct gcaaatccat t                    41

<210>  7
<211>  49
<212>  PRT
<213>  Homo sapiens

<400>  7

Cys Arg Glu Cys Glu Ser Gly Ser Phe Thr Ala Ser Glu Asn His Leu
1               5                   10                  15

Arg His Cys Leu Ser Cys Ser Lys Cys Arg Lys Glu Met Gly Gln Val
            20                  25                  30

Glu Ile Ser Ser Cys Thr Val Asp Arg Asp Thr Val Cys Gly Cys Arg
        35                  40                  45

Lys

<210>  8
<211>  48
<212>  PRT
<213>  Homo sapiens

<400>  8

Cys Asn Pro Cys Thr Glu Gly Val Asp Tyr Thr Asn Ala Ser Asn Asn
1               5                   10                  15

Glu Pro Ser Cys Phe Pro Cys Thr Val Cys Lys Ser Asp Gln Lys His
            20                  25                  30

Lys Ser Ser Cys Thr Met Thr Arg Asp Thr Val Cys Gln Cys Lys Glu
        35                  40                  45

<210>  9
<211>  70
<212>  DNA
<213>  Artificial sequence

<220>
<221>  source
<223>  /note= "Description of artificial sequence: Synthetic
       oligonucleotide probe"

<400>  9
gggagccgct catgaggaag ttgggcctca tggacaatga gataaaggtg gctaaagctg      60

aggcagcggg                                                             70

<210>  10
<211>  1799
<212>  DNA
<213>  Homo sapiens

<220>
<221>  CDS
<222>  (140)..(1375)

<220>
<221>  misc_feature
<222>  1367..1369
<223>  wtg translates as Xaa, which stands for Met, or Leu.

<400>  10
cccacgcgtc cgcataaatc agcacgcggc cggagaaccc cgcaatctct gcgcccacaa      60

aatacaccga cgatgcccga tctactttaa gggctgaaac ccacgggcct gagagactat     120

aagagcgttc cctaccgcc atg gaa caa cgg gga cag aac gcc ccg gcc gct      172
                     Met Glu Gln Arg Gly Gln Asn Ala Pro Ala Ala
                     1               5                   10

tcg ggg gcc cgg aaa agg cac ggc cca gga ccc agg gag gcg cgg gga      220
Ser Gly Ala Arg Lys Arg His Gly Pro Gly Pro Arg Glu Ala Arg Gly
            15                  20                  25

gcc agg cct ggg ctc cgg gtc ccc aag acc ctt gtg ctc gtt gtc gcc      268
Ala Arg Pro Gly Leu Arg Val Pro Lys Thr Leu Val Leu Val Val Ala
        30                  35                  40

gcg gtc ctg ctg ttg gtc tca gct gag tct gct ctg atc acc caa caa      316
Ala Val Leu Leu Leu Val Ser Ala Glu Ser Ala Leu Ile Thr Gln Gln
    45                  50                  55

gac cta gct ccc cag cag aga gcg gcc cca caa caa aag agg tcc agc      364
Asp Leu Ala Pro Gln Gln Arg Ala Ala Pro Gln Gln Lys Arg Ser Ser
60                  65                  70                  75

ccc tca gag gga ttg tgt cca cct gga cac cat atc tca gaa gac ggt 412
Pro Ser Glu Gly Leu Cys Pro Pro Gly His His Ile Ser Glu Asp Gly
                80                  85                  90

aga gat tgc atc tcc tgc aaa tat gga cag gac tat agc act cac tgg 460
Arg Asp Cys Ile Ser Cys Lys Tyr Gly Gln Asp Tyr Ser Thr His Trp
            95                  100                 105

aat gac ctc ctt ttc tgc ttg cgc tgc acc agg tgt gat tca ggt gaa 508
Asn Asp Leu Leu Phe Cys Leu Arg Cys Thr Arg Cys Asp Ser Gly Glu
        110                 115                 120

gtg gag cta agt ccc tgc acc acg acc aga aac aca gtg tgt cag tgc 556
Val Glu Leu Ser Pro Cys Thr Thr Thr Arg Asn Thr Val Cys Gln Cys
    125                 130                 135

gaa gaa ggc acc ttc cgg gaa gaa gat tct cct gag atg tgc cgg aag 604
Glu Glu Gly Thr Phe Arg Glu Glu Asp Ser Pro Glu Met Cys Arg Lys
140                 145                 150                 155

tgc cgc aca ggg tgt ccc aga ggg atg gtc aag gtc ggt gat tgt aca 652
Cys Arg Thr Gly Cys Pro Arg Gly Met Val Lys Val Gly Asp Cys Thr
                160                 165                 170

ccc tgg agt gac atc gaa tgt gtc cac aaa gaa tca ggc atc atc ata 700
Pro Trp Ser Asp Ile Glu Cys Val His Lys Glu Ser Gly Ile Ile Ile
            175                 180                 185

gga gtc aca gtt gca gcc gta gtc ttg att gtg gct gtg ttt gtt tgc 748
Gly Val Thr Val Ala Ala Val Val Leu Ile Val Ala Val Phe Val Cys
        190                 195                 200

aag tct tta ctg tgg aag aaa gtc ctt cct tac ctg aaa ggc atc tgc 796
Lys Ser Leu Leu Trp Lys Lys Val Leu Pro Tyr Leu Lys Gly Ile Cys
    205                 210                 215

tca ggt ggt ggt ggg gac cct gag cgt gtg gac aga agc tca caa cga 844
Ser Gly Gly Gly Gly Asp Pro Glu Arg Val Asp Arg Ser Ser Gln Arg
220                 225                 230                 235

cct ggg gct gag gac aat gtc ctc aat gag atc gtg agt atc ttg cag 892
Pro Gly Ala Glu Asp Asn Val Leu Asn Glu Ile Val Ser Ile Leu Gln
                240                 245                 250

ccc acc cag gtc cct gag cag gaa atg gaa gtc cag gag cca gca gag 940
Pro Thr Gln Val Pro Glu Gln Glu Met Glu Val Gln Glu Pro Ala Glu
            255                 260                 265

cca aca ggt gtc aac atg ttg tcc ccc ggg gag tca gag cat ctg ctg 988
Pro Thr Gly Val Asn Met Leu Ser Pro Gly Glu Ser Glu His Leu Leu
        270                 275                 280

gaa ccg gca gaa gct gaa agg tct cag agg agg agg ctg ctg gtt cca 1036
Glu Pro Ala Glu Ala Glu Arg Ser Gln Arg Arg Arg Leu Leu Val Pro
    285                 290                 295

gca aat gaa ggt gat ccc act gag act ctg aga cag tgc ttc gat gac 1084
Ala Asn Glu Gly Asp Pro Thr Glu Thr Leu Arg Gln Cys Phe Asp Asp
300                 305                 310                 315

ttt gca gac ttg gtg ccc ttt gac tcc tgg gag ccg ctc atg agg aag 1132
Phe Ala Asp Leu Val Pro Phe Asp Ser Trp Glu Pro Leu Met Arg Lys
                320                 325                 330

ttg ggc ctc atg gac aat gag ata aag gtg gct aaa gct gag gca gcg 1180
Leu Gly Leu Met Asp Asn Glu Ile Lys Val Ala Lys Ala Glu Ala Ala
            335                 340                 345

ggc cac agg gac acc ttg tac acg atg ctg ata aag tgg gtc aac aaa 1228
Gly His Arg Asp Thr Leu Tyr Thr Met Leu Ile Lys Trp Val Asn Lys
        350                 355                 360

acc ggg cga gat gcc tct gtc cac acc ctg ctg gat gcc ttg gag acg 1276
Thr Gly Arg Asp Ala Ser Val His Thr Leu Leu Asp Ala Leu Glu Thr
    365                 370                 375

ctg gga gag aga ctt gcc aag cag aag att gag gac cac ttg ttg agc 1324
Leu Gly Glu Arg Leu Ala Lys Gln Lys Ile Glu Asp His Leu Leu Ser
380                 385                 390                 395

tct gga aag ttc atg tat cta gaa ggt aat gca gac tct gcc wtg tcc     1372
Ser Gly Lys Phe Met Tyr Leu Glu Gly Asn Ala Asp Ser Ala Xaa Ser
                400                 405                 410

taa gtgtgattct cttcaggaag tgagaccttc cctggtttac cttttttctg          1425

gaaaaagccc aactggactc cagtcagtag gaaagtgcca caattgtcac atgaccggta   1485

ctggaagaaa ctctcccatc caacatcacc cagtggatgg aacatcctgt aacttttcac   1545

tgcacttggc attattttta taagctgaat gtgataataa ggacactatg gaaatgtctg   1605

gatcattccg tttgtgcgta ctttgagatt tggtttggga tgtcattgtt ttcacagcac   1665

ttttttatcc taatgtaaat gctttattta tttatttggg ctacattgta agatccatct   1725

acaaaaaaaa aaaaaaaaaa aaaagggcgg ccgcgactct agagtcgacc tgcagaagct   1785

tggccgccat ggcc                                                      1799

<210>  11
<211>  411
<212>  PRT
<213>  Homo sapiens

<220>
<221>  misc_feature
<222>  (410)..(410)
<223>  The 'Xaa' at location 410 stands for Met, or Leu.

<400>  11

Met Glu Gln Arg Gly Gln Asn Ala Pro Ala Ala Ser Gly Ala Arg Lys
1               5                   10                  15

Arg His Gly Pro Gly Pro Arg Glu Ala Arg Gly Ala Arg Pro Gly Leu
            20                  25                  30

Arg Val Pro Lys Thr Leu Val Leu Val Val Ala Ala Val Leu Leu Leu
        35                  40                  45

Val Ser Ala Glu Ser Ala Leu Ile Thr Gln Gln Asp Leu Ala Pro Gln
    50                  55                  60

Gln Arg Ala Ala Pro Gln Gln Lys Arg Ser Ser Pro Ser Glu Gly Leu
65                  70                  75                  80

Cys Pro Pro Gly His His Ile Ser Glu Asp Gly Arg Asp Cys Ile Ser
                85                  90                  95

Cys Lys Tyr Gly Gln Asp Tyr Ser Thr His Trp Asn Asp Leu Leu Phe
            100                 105                 110

Cys Leu Arg Cys Thr Arg Cys Asp Ser Gly Glu Val Glu Leu Ser Pro
        115                 120                 125

Cys Thr Thr Thr Arg Asn Thr Val Cys Gln Cys Glu Glu Gly Thr Phe
    130                 135                 140

Arg Glu Glu Asp Ser Pro Glu Met Cys Arg Lys Cys Arg Thr Gly Cys
145                 150                 155                 160

Pro Arg Gly Met Val Lys Val Gly Asp Cys Thr Pro Trp Ser Asp Ile
                165                 170                 175

Glu Cys Val His Lys Glu Ser Gly Ile Ile Ile Gly Val Thr Val Ala
            180                 185                 190

Ala Val Val Leu Ile Val Ala Val Phe Val Cys Lys Ser Leu Leu Trp
        195                 200                 205

Lys Lys Val Leu Pro Tyr Leu Lys Gly Ile Cys Ser Gly Gly Gly Gly
    210                 215                 220

Asp Pro Glu Arg Val Asp Arg Ser Ser Gln Arg Pro Gly Ala Glu Asp
225                 230                 235                 240

Asn Val Leu Asn Glu Ile Val Ser Ile Leu Gln Pro Thr Gln Val Pro
                245                 250                 255

Glu Gln Glu Met Glu Val Gln Glu Pro Ala Glu Pro Thr Gly Val Asn
            260                 265                 270

Met Leu Ser Pro Gly Glu Ser Glu His Leu Leu Glu Pro Ala Glu Ala
        275                 280                 285

Glu Arg Ser Gln Arg Arg Arg Leu Leu Val Pro Ala Asn Glu Gly Asp
    290                 295                 300

Pro Thr Glu Thr Leu Arg Gln Cys Phe Asp Asp Phe Ala Asp Leu Val
305                 310                 315                 320

Pro Phe Asp Ser Trp Glu Pro Leu Met Arg Lys Leu Gly Leu Met Asp
                325                 330                 335

Asn Glu Ile Lys Val Ala Lys Ala Glu Ala Ala Gly His Arg Asp Thr
            340                 345                 350

Leu Tyr Thr Met Leu Ile Lys Trp Val Asn Lys Thr Gly Arg Asp Ala
        355                 360                 365

Ser Val His Thr Leu Leu Asp Ala Leu Glu Thr Leu Gly Glu Arg Leu
    370                 375                 380

Ala Lys Gln Lys Ile Glu Asp His Leu Leu Ser Ser Gly Lys Phe Met
385                 390                 395                 400

Tyr Leu Glu Gly Asn Ala Asp Ser Ala Xaa Ser
                405                 410

<210>  12
<211>  29
<212>  DNA
<213>  Artificial sequence

<220>
<221>  source
<223>  /note= "Description of artificial sequence: Synthetic
       oligonucleotide probe"

<400>  12
atcagggact ttccgctggg gactttccg                                          29

<210>  13
<211>  30
<212>  DNA
<213>  Artificial sequence

<220>
<221>  source
<223>  /note= "Description of artificial sequence: Synthetic
       oligonucleotide probe"

<400>  13
aggatgggaa gtgtgtgata tatccttgat                                         30

<210> 14
<211> 1180
<212> DNA
<213> Homo sapiens

<400> 14
tttttttttt tttttttttt tttttttttt tttttttttt tttttttttt tttttttttt 60

tttttttttg gggcaggggc aggcgtttct gtctgtggga acacagcaga gggaggcagg 120

gcagagagtg tccagcgccc cccgccctca gccagcgcct acctgaacct ttcaggtaag 180

gaaggaattt cttccacagt gaagtctttc aaacaaacac aatcagaagc acaattagaa 240

ctatgatccc tacgatggtg catgagaggt aatgagaaga ggcaggagtc cccgggctgg 300

tggtcattgt ctcttcagca gctggggcag gagtccccgg gctggtggtc attgtctctt 360

cagcagctgg ggcaggagtc cccgggctgg tggtcattgt ctcttcagca gctggggcag 420

gagtccctgg gctggtgttc attgtctctt cagcagctgg ggcaggagtc cccgggctgg 480

tgttcattgt ctcttcagca gctggggttt ccacagtggc attggcacca aattcttcaa 540

cacactggat atcatcccag gacgtacaat tactgacttg gacttcccca ctagggcacc 600

tgctacactt ccggcacatc tctggggagt tttcattccg gaaggtgcct tctttacact 660

gacacactgt gtctctggtc atggtgcagg aacttttatg tttttgatct gatttacaaa 720

ctgtacatgg gaagcaagaa ggttcattgt tggaagcgtt ggtgtaatcc acaccctctg 780

tgcacgggtt acaggctcca gtatgttctg atctatgaga tcctgctgga cactcctccc 840

ccttgaagct gtgcctctgt tgctgtgggg ccactgtctg ctggggaact tcctcctgcc 900

gggcagtggt ggcagagtaa gctaggactg gcagcaggac cgcgacgatg acgacgacga 960

actttagggt cttggggatc cgggccatgg tatggttccc gacgccgtcc tgggtcctgg 1020

gtcgcaccct gcctcggcca tcaggcggcc ggggcgctct gtccccagag ttccctaacg    1080

gtaggaagcg ctccttcacc ccttgcatct ctggtcaaac tcccaaaaat ctatcagaaa    1140

tcgttggctg agttcgtgcg cgtggagagg ttcccacagc                          1180

<210>  15
<211>  418
<212>  PRT
<213>  Homo sapiens

<400>  15

Gly Arg Gly Ala Leu Pro Thr Ser Met Gly Gln His Gly Pro Ser Ala
1               5                   10                  15

Arg Ala Arg Ala Gly Arg Ala Pro Gly Pro Arg Pro Ala Arg Glu Ala
            20                  25                  30

Ser Pro Arg Leu Arg Val His Lys Thr Phe Lys Phe Val Val Val Gly
        35                  40                  45

Val Leu Leu Gln Val Val Pro Ser Ser Ala Ala Thr Ile Lys Leu His
    50                  55                  60

Asp Gln Ser Ile Gly Thr Gln Gln Trp Glu His Ser Pro Leu Gly Glu
65                  70                  75                  80

Leu Cys Pro Pro Gly Ser His Arg Ser Glu His Pro Gly Ala Cys Asn
                85                  90                  95

Arg Cys Thr Glu Gly Val Gly Tyr Thr Asn Ala Ser Asn Asn Leu Phe
            100                 105                 110

Ala Cys Leu Pro Cys Thr Ala Cys Lys Ser Asp Glu Glu Glu Arg Ser
        115                 120                 125

Pro Cys Thr Thr Thr Arg Asn Thr Ala Cys Gln Cys Lys Pro Gly Thr
    130                 135                 140

Phe Arg Asn Asp Asn Ser Ala Glu Met Cys Arg Lys Cys Ser Arg Gly
145                 150                 155                 160

Cys Pro Arg Gly Met Val Lys Val Lys Asp Cys Thr Pro Trp Ser Asp
                165                 170                 175

Ile Glu Cys Val His Lys Glu Ser Gly Asn Gly His Asn Ile Trp Val
            180                 185                 190

Ile Leu Val Val Thr Leu Val Val Pro Leu Leu Leu Val Ala Val Leu
        195                 200                 205

Ile Val Cys Cys Cys Ile Gly Ser Gly Cys Gly Gly Asp Pro Lys Cys
    210                 215                 220

Met Asp Arg Val Cys Phe Trp Arg Leu Gly Leu Leu Arg Gly Pro Gly
225                 230                 235                 240

Ala Glu Asp Asn Ala His Asn Glu Ile Leu Ser Asn Ala Asp Ser Leu
                245                 250                 255

Ser Thr Phe Val Ser Glu Gln Gln Met Glu Ser Gln Glu Pro Ala Asp
            260                 265                 270

Leu Thr Gly Val Thr Val Gln Ser Pro Gly Glu Ala Gln Cys Leu Leu
        275                 280                 285

Gly Pro Ala Glu Ala Glu Gly Ser Gln Arg Arg Arg Leu Leu Val Pro
    290                 295                 300

Ala Asn Gly Ala Asp Pro Thr Glu Thr Leu Met Leu Phe Phe Asp Lys
305                 310                 315                 320

Phe Ala Asn Ile Val Pro Phe Asp Ser Trp Asp Gln Leu Met Arg Gln
                325                 330                 335

Leu Asp Leu Thr Lys Asn Glu Ile Asp Val Val Arg Ala Gly Thr Ala
            340                 345                 350

Gly Pro Gly Asp Ala Leu Tyr Ala Met Leu Met Lys Trp Val Asn Lys
        355                 360                 365

Thr Gly Arg Asn Ala Ser Ile His Thr Leu Leu Asp Ala Leu Glu Arg
    370                 375                 380

Met Glu Glu Arg His Ala Lys Glu Lys Ile Gln Asp Leu Leu Val Asp
385                 390                 395                 400

Ser Gly Lys Phe Ile Tyr Leu Glu Asp Gly Thr Gly Ser Ala Val Ser
                405                 410                 415

Leu Glu

<210>  16
<211>  74
<212>  PRT
<213>  Homo sapiens

<400>  16

Val Met Asp Ala Val Pro Ala Arg Arg Trp Lys Glu Phe Val Arg Thr
1               5                   10                  15

Leu Gly Leu Arg Glu Ala Glu Ile Glu Ala Val Glu Val Glu Ile Gly
            20                  25                  30

Arg Phe Arg Asp Gln Gln Tyr Glu Met Leu Lys Arg Trp Arg Gln Gln
        35                  40                  45

Gln Pro Ala Gly Leu Gly Ala Val Tyr Ala Ala Leu Glu Arg Met Gly
    50                  55                  60

Leu Asp Gly Cys Val Glu Asp Leu Arg Ser
65                  70

<210>  17
<211>  78
<212>  PRT
<213>  Homo sapiens

<400>  17

Val Val Glu Asn Val Pro Pro Leu Arg Trp Lys Glu Phe Val Arg Arg
1               5                   10                  15

Leu Gly Leu Ser Asp His Glu Ile Asp Arg Leu Glu Leu Gln Asn Gly
            20                  25                  30

Arg Cys Leu Arg Glu Ala Gln Tyr Ser Met Leu Ala Thr Trp Arg Arg
        35                  40                  45

Arg Thr Pro Arg Arg Glu Ala Thr Leu Glu Leu Leu Gly Arg Val Leu
    50                  55                  60

Arg Asp Met Asp Leu Leu Gly Cys Leu Glu Asp Ile Glu Glu
65                  70                  75

<210>  18
<211>  77
<212>  PRT
<213>  Homo sapiens

<400>  18

Ile Ala Gly Val His Thr Leu Ser Gln Val Lys Gly Phe Val Arg Lys
1               5                   10                  15

Asn Gly Val Asn Glu Ala Lys Ile Asp Glu Ile Lys Asn Asp Asn Val
            20                  25                  30

Gln Asp Thr Ala Glu Gln Lys Val Gln Leu Leu Arg Asn Trp His Gln
        35                  40                  45

Leu His Gly Lys Lys Glu Ala Tyr Asp Thr Leu Ile Lys Asp Leu Lys
    50                  55                  60

Lys Ala Asn Leu Cys Thr Leu Ala Glu Lys Ile Gln Thr
65                  70                  75

<210>  19
<211>  1799
<212>  DNA
<213>  Homo sapiens

<400>  19
ggccatggcg gccaagcttc tgcaggtcga ctctagagtc gcggccgccc tttttttttt      60

tttttttttt ttgtagatgg atcttacaat gtagcccaaa taaataaata aagcatttac     120

attaggataa aaaagtgctg tgaaaacaat gacatcccaa accaaatctc aaagtacgca     180

caaacggaat gatccagaca tttccatagt gtccttatta tcacattcag cttataaaaa     240

taatgccaag tgcagtgaaa agttacagga tgttccatcc actgggtgat gttggatggg     300

agagtttctt ccagtaccgg tcatgtgaca attgtggcac tttcctactg actggagtcc     360

agttgggctt tttccagaaa aaaggtaaac cagggaaggt ctcacttcct gaagagaatc     420

acacttagga cawggcagag tctgcattac cttctagata catgaacttt ccagagctca     480

acaagtggtc ctcaatcttc tgcttggcaa gtctctctcc cagcgtctcc aaggcatcca     540

gcagggtgtg gacagaggca tctcgcccgg ttttgttgac ccactttatc agcatcgtgt     600

acaaggtgtc cctgtggccc gctgcctcag ctttagccac ctttatctca ttgtccatga     660

ggcccaactt cctcatgagc ggctcccagg agtcaaaggg caccaagtct gcaaagtcat     720

cgaagcactg tctcagagtc tcagtgggat caccttcatt tgctggaacc agcagcctcc     780

tcctctgaga cctttcagct tctgccggtt ccagcagatg ctctgactcc ccgggggaca     840

acatgttgac acctgttggc tctgctggct cctggacttc catttcctgc tcagggacct     900

gggtgggctg caagatactc acgatctcat tgaggacatt gtcctcagcc ccaggtcgtt     960

gtgagcttct gtccacacgc tcagggtccc caccaccacc tgagcagatg cctttcaggt    1020

aaggaaggac tttcttccac agtaaagact tgcaaacaaa cacagccaca atcaagacta 1080

cggctgcaac tgtgactcct atgatgatgc ctgattcttt gtggacacat tcgatgtcac 1140

tccagggtgt acaatcaccg accttgacca tccctctggg acaccctgtg cggcacttcc 1200

ggcacatctc aggagaatct tcttcccgga aggtgccttc ttcgcactga cacactgtgt 1260

ttctggtcgt ggtgcaggga cttagctcca cttcacctga atcacacctg gtgcagcgca 1320

agcagaaaag gaggtcattc cagtgagtgc tatagtcctg tccatatttg caggagatgc 1380

aatctctacc gtcttctgag atatggtgtc caggtggaca caatccctct gaggggctgg 1440

acctcttttg ttgtggggcc gctctctgct ggggagctag gtcttgttgg gtgatcagag 1500

cagactcagc tgagaccaac agcaggaccg cggcgacaac gagcacaagg gtcttgggga 1560

cccggagccc aggcctggct ccccgcgcct ccctgggtcc tgggccgtgc cttttccggg 1620

cccccgaagc ggccggggcg ttctgtcccc gttgttccat ggcggtaggg aacgctctta 1680

tagtctctca ggcccgtggg tttcagccct taaagtagat cgggcatcgt cggtgtattt 1740

tgtgggcgca gagattgcgg ggttctccgg ccgcgtgctg atttatgcgg acgcgtggg 1799

SEQUENCE LISTING

(1) GENERAL INFORMATION:

(i) APPLICANT: Genentech, Inc.

(ii) TITLE OF INVENTION: Apo-2DcR

(iii) NUMBER OF SEQUENCES: 13

(iv) CORRESPONDENCE ADDRESS:
(A) ADDRESSEE: Genentech, Inc.
(B) STREET: 1 DNA Way
(C) CITY: South San Francisco
(D) STATE: California
(E) COUNTRY: USA
(F) ZIP: 94080

(v) COMPUTER READABLE FORM:
(A) MEDIUM TYPE: 3.5 inch, 1.44 Mb floppy disk
(B) COMPUTER: IBM PC compatible
(C) OPERATING SYSTEM: PC-DOS/MS-DOS
(D) SOFTWARE: WinPatin (Genentech)

(vi) CURRENT APPLICATION DATA:
(A) APPLICATION NUMBER:
(B) FILING DATE: 12-Jun-1998
(C) CLASSIFICATION:

(vii) PRIOR APPLICATION DATA:
(A) APPLICATION NUMBER: 08/878168
(B) FILING DATE: 18-JUN-1997

(viii) ATTORNEY/AGENT INFORMATION:
(A) NAME: Marschang, Diane L.
(B) REGISTRATION NUMBER: 35,600
(C) REFERENCE/DOCKET NUMBER: P1110P1PCT

(ix) TELECOMMUNICATION INFORMATION:
(A) TELEPHONE: 650/225-5416
(B) TELEFAX: 650/952-9881
(2) INFORMATION FOR SEQ ID NO:1:

(i) SEQUENCE CHARACTERISTICS:
(A) LENGTH: 259 amino acids
(B) TYPE: Amino Acid
(D) TOPOLOGY: Linear

(xi) SEQUENCE DESCRIPTION: SEQ ID NO:1:

Met Ala Arg Ile Pro Lys Thr Leu Lys Phe Val Val Val Ile Val
1               5                   10                  15

Ala Val Leu Leu Pro Val Leu Ala Tyr Ser Ala Thr Thr Ala Arg
                20                  25                  30

Gln Glu Glu Val Pro Gln Gln Thr Val Ala Pro Gln Gln Gln Arg
                35                  40                  45

His Ser Phe Lys Gly Glu Glu Cys Pro Ala Gly Ser His Arg Ser
                50                  55                  60

Glu His Thr Gly Ala Cys Asn Pro Cys Thr Glu Gly Val Asp Tyr
                65                  70                  75

Thr Asn Ala Ser Asn Asn Glu Pro Ser Cys Phe Pro Cys Thr Val
                80                  85                  90

Cys Lys Ser Asp Gln Lys His Lys Ser Ser Cys Thr Met Thr Arg
                95                  100                 105

Asp Thr Val Cys Gln Cys Lys Glu Gly Thr Phe Arg Asn Glu Asn
                110                 115                 120

Ser Pro Glu Met Cys Arg Lys Cys Ser Arg Cys Pro Ser Gly Glu
                125                 130                 135

Val Gln Val Ser Asn Cys Thr Ser Trp Asp Asp Ile Gln Cys Val
                140                 145                 150

Glu Glu Phe Gly Ala Asn Ala Thr Val Glu Thr Pro Ala Ala Glu
                155                 160                 165

Glu Thr Met Asn Thr Ser Pro Gly Thr Pro Ala Pro Ala Ala Glu
                170                 175                 180

Glu Thr Met Asn Thr Ser Pro Gly Thr Pro Ala Pro Ala Ala Glu
                185                 190                 195

Glu Thr Met Thr Thr Ser Pro Gly Thr Pro Ala Pro Ala Ala Glu
                200                 205                 210

Glu Thr Met Thr Thr Ser Pro Gly Thr Pro Ala Pro Ala Ala Glu
                215                 220                 225

Glu Thr Met Thr Thr Ser Pro Gly Thr Pro Ala Ser Ser His Tyr
                230                 235                 240

Leu Ser Cys Thr Ile Val Gly Ile Ile Val Leu Ile Val Leu Leu
                245                 250                 255

Ile Val Phe Val
            259

(2) INFORMATION FOR SEQ ID NO:2:

   (i) SEQUENCE CHARACTERISTICS:
       (A) LENGTH: 1180 base pairs
       (B) TYPE: Nucleic Acid
       (C) STRANDEDNESS: Single
       (D) TOPOLOGY: Linear

  (xi) SEQUENCE DESCRIPTION: SEQ ID NO:2:

GCTGTGGGAA CCTCTCCACG CGCACGAACT CAGCCAACGA TTTCTGATAG 50

ATTTTTGGGA GTTTGACCAG AGATGCAAGG GGTGAAGGAG CGCTTCCTAC 100

CGTTAGGGAA CTCTGGGGAC AGAGCGCCCC GGCCGCCTGA TGGCCGAGGC 150

AGGGTGCGAC CCAGGACCCA GGACGGCGTC GGGAACCATA CC ATG 195
Met
1

GCC CGG ATC CCC AAG ACC CTA AAG TTC GTC GTC GTC ATC 234
Ala Arg Ile Pro Lys Thr Leu Lys Phe Val Val Val Ile
            5                   10

GTC GCG GTC CTG CTG CCA GTC CTA GCT TAC TCT GCC ACC 273
Val Ala Val Leu Leu Pro Val Leu Ala Tyr Ser Ala Thr
15                  20                  25

ACT GCC CGG CAG GAG GAA GTT CCC CAG CAG ACA GTG GCC 312
Thr Ala Arg Gln Glu Glu Val Pro Gln Gln Thr Val Ala
        30                  35                  40

CCA CAG CAA CAG AGG CAC AGC TTC AAG GGG GAG GAG TGT 351
Pro Gln Gln Gln Arg His Ser Phe Lys Gly Glu Glu Cys
                45                  50

CCA GCA GGA TCT CAT AGA TCA GAA CAT ACT GGA GCC TGT 390
Pro Ala Gly Ser His Arg Ser Glu His Thr Gly Ala Cys
    55                  60                  65

AAC CCG TGC ACA GAG GGT GTG GAT TAC ACC AAC GCT TCC 429
Asn Pro Cys Thr Glu Gly Val Asp Tyr Thr Asn Ala Ser
            70                  75

AAC AAT GAA CCT TCT TGC TTC CCA TGT ACA GTT TGT AAA 468
Asn Asn Glu Pro Ser Cys Phe Pro Cys Thr Val Cys Lys
80                  85                  90

TCA GAT CAA AAA CAT AAA AGT TCC TGC ACC ATG ACC AGA 507
Ser Asp Gln Lys His Lys Ser Ser Cys Thr Met Thr Arg
        95                  100                 105

GAC ACA GTG TGT CAG TGT AAA GAA GGC ACC TTC CGG AAT 546
Asp Thr Val Cys Gln Cys Lys Glu Gly Thr Phe Arg Asn
                110                 115

GAA AAC TCC CCA GAG ATG TGC CGG AAG TGT AGC AGG TGC 585
Glu Asn Ser Pro Glu Met Cys Arg Lys Cys Ser Arg Cys
    120                 125                 130

CCT AGT GGG GAA GTC CAA GTC AGT AAT TGT ACG TCC TGG 624
Pro Ser Gly Glu Val Gln Val Ser Asn Cys Thr Ser Trp
            135                 140

GAT GAT ATC CAG TGT GTT GAA GAA TTT GGT GCC AAT GCC 663
Asp Asp Ile Gln Cys Val Glu Glu Phe Gly Ala Asn Ala
145                 150                 155

ACT GTG GAA ACC CCA GCT GCT GAA GAG ACA ATG AAC ACC 702
Thr Val Glu Thr Pro Ala Ala Glu Glu Thr Met Asn Thr

        160                 165                 170

AGC CCG GGG ACT CCT GCC CCA GCT GCT GAA GAG ACA ATG 741
Ser Pro Gly Thr Pro Ala Pro Ala Ala Glu Glu Thr Met
                175                 180

AAC ACC AGC CCA GGG ACT CCT GCC CCA GCT GCT GAA GAG 780
Asn Thr Ser Pro Gly Thr Pro Ala Pro Ala Ala Glu Glu
    185                 190                 195

ACA ATG ACC ACC AGC CCG GGG ACT CCT GCC CCA GCT GCT 819
Thr Met Thr Thr Ser Pro Gly Thr Pro Ala Pro Ala Ala
            200                 205

GAA GAG ACA ATG ACC ACC AGC CCG GGG ACT CCT GCC CCA 858
Glu Glu Thr Met Thr Thr Ser Pro Gly Thr Pro Ala Pro
210                 215                 220

GCT GCT GAA GAG ACA ATG ACC ACC AGC CCG GGG ACT CCT 897
Ala Ala Glu Glu Thr Met Thr Thr Ser Pro Gly Thr Pro
        225                 230                 235

GCC TCT TCT CAT TAC CTC TCA TGC ACC ATC GTA GGG ATC 936
Ala Ser Ser His Tyr Leu Ser Cys Thr Ile Val Gly Ile
                240                 245

ATA GTT CTA ATT GTG CTT CTG ATT GTG TTT GTT T 970
Ile Val Leu Ile Val Leu Leu Ile Val Phe Val
250 255 259

GAAAGACTTC ACTGTGGAAG AAATTCCTTC CTTACCTGAA AGGTTCAGGT 1020

AGGCGCTGGC TGAGGGCGGG GGGCGCTGGA CACTCTCTGC CCTGCCTCCC 1070

TCTGCTGTGT TCCCACAGAC AGAAACGCCT GCCCCTGCCC CAAAAAAAAA 1120

AAAAAAAAAA AAAAAAAAAA AAAAAAAAAA AAAAAAAAAA AAAAAAAAAA 1170

AAAAAAAAAA 1180

(2) INFORMATION FOR SEQ ID NO:3:

(i) SEQUENCE CHARACTERISTICS:
(A) LENGTH: 299 amino acids
(B) TYPE: Amino Acid
(D) TOPOLOGY: Linear

(xi) SEQUENCE DESCRIPTION: SEQ ID NO:3:

(2) INFORMATION FOR SEQ ID NO:4:

   (i) SEQUENCE CHARACTERISTICS:
      (A) LENGTH: 1180 base pairs
      (B) TYPE: Nucleic Acid
      (C) STRANDEDNESS: Single
      (D) TOPOLOGY: Linear

(xi) SEQUENCE DESCRIPTION: SEQ ID NO:4:
GCTGTGGGAA CCTCTCCACG CGCACGAACT CAGCCAACGA TTTCTGATAG 50
ATTTTTGGGA GTTTGACCAG AG ATG CAA GGG GTG AAG GAG 90
CGC TTC CTA CCG TTA GGG AAC TCT GGG GAC AGA GCG CCC 129
CGG CCG CCT GAT GGC CGA GGC AGG GTG CGA CCC AGG ACC 168
CAG GAC GGC GTC GGG AAC CAT ACC ATG GCC CGG ATC CCC 207
AAG ACC CTA AAG TTC GTC GTC GTC ATC GTC GCG GTC CTG 246
CTG CCA GTC CTA GCT TAC TCT GCC ACC ACT GCC CGG CAG 285
GAG GAA GTT CCC CAG CAG ACA GTG GCC CCA CAG CAA CAG 324
AGG CAC AGC TTC AAG GGG GAG GAG TGT CCA GCA GGA TCT 363
CAT AGA TCA GAA CAT ACT GGA GCC TGT AAC CCG TGC ACA 402
GAG GGT GTG GAT TAC ACC AAC GCT TCC AAC AAT GAA CCT 441
TCT TGC TTC CCA TGT ACA GTT TGT AAA TCA GAT CAA AAA 480
CAT AAA AGT TCC TGC ACC ATG ACC AGA GAC ACA GTG TGT 519
CAG TGT AAA GAA GGC ACC TTC CGG AAT GAA AAC TCC CCA 558
GAG ATG TGC CGG AAG TGT AGC AGG TGC CCT AGT GGG GAA 597
GTC CAA GTC AGT AAT TGT ACG TCC TGG GAT GAT ATC CAG 636
TGT GTT GAA GAA TTT GGT GCC AAT GCC ACT GTG GAA ACC 675
CCA GCT GCT GAA GAG ACA ATG AAC ACC AGC CCG GGG ACT 714
CCT GCC CCA GCT GCT GAA GAG ACA ATG AAC ACC AGC CCA 753
GGG ACT CCT GCC CCA GCT GCT GAA GAG ACA ATG ACC ACC 792
AGC CCG GGG ACT CCT GCC CCA GCT GCT GAA GAG ACA ATG 831
ACC ACC AGC CCG GGG ACT CCT GCC CCA GCT GCT GAA GAG 870
ACA ATG ACC ACC AGC CCG GGG ACT CCT GCC TCT TCT CAT 909
TAC CTC TCA TGC ACC ATC GTA GGG ATC ATA GTT CTA ATT 948
GTG CTT CTG ATT GTG TTT GTT T GAAAGACTTC ACTGTGGAAG 990
AAATTCCTTC CTTACCTGAA AGGTTCAGGT AGGCGCTGGC TGAGGGCGGG 1040
GGGCGCTGGA CACTCTCTGC CCTGCCTCCC TCTGCTGTGT TCCCACAGAC 1090
AGAAACGCCT GCCCCTGCCC CAAAAAAAAA AAAAAAAAAA AAAAAAAAAA 1140
AAAAAAAAAA AAAAAAAAAA AAAAAAAAAA AAAAAAAAAA 1180

(2) INFORMATION FOR SEQ ID NO:5:

   (i) SEQUENCE CHARACTERISTICS:
       (A) LENGTH: 43 base pairs
       (B) TYPE: Nucleic Acid
       (C) STRANDEDNESS: Single
       (D) TOPOLOGY: Linear

(xi) SEQUENCE DESCRIPTION: SEQ ID NO:5:

TGTAAAACGA CGGCCAGTTA AATAGACCTG CAATTATTAA TCT 43

(2) INFORMATION FOR SEQ ID NO:6:

(i) SEQUENCE CHARACTERISTICS:
(A) LENGTH: 41 base pairs
(B) TYPE: Nucleic Acid
(C) STRANDEDNESS: Single
(D) TOPOLOGY: Linear

(xi) SEQUENCE DESCRIPTION: SEQ ID NO:6:

CAGGAAACAG CTATGACCAC CTGCACACCT GCAAATCCAT T 41

(2) INFORMATION FOR SEQ ID NO:7:

(i) SEQUENCE CHARACTERISTICS:
(A) LENGTH: 49 amino acids
(B) TYPE: Amino Acid
(D) TOPOLOGY: Linear

(xi) SEQUENCE DESCRIPTION: SEQ ID NO:7:

Cys Arg Glu Cys Glu Ser Gly Ser Phe Thr Ala Ser Glu Asn His
1               5                   10                  15

Leu Arg His Cys Leu Ser Cys Ser Lys Cys Arg Lys Glu Met Gly
                20                  25                  30

Gln Val Glu Ile Ser Ser Cys Thr Val Asp Arg Asp Thr Val Cys
                35                  40                  45

Gly Cys Arg Lys
49

(2) INFORMATION FOR SEQ ID NO:8:

(i) SEQUENCE CHARACTERISTICS:
(A) LENGTH: 48 amino acids
(B) TYPE: Amino Acid
(D) TOPOLOGY: Linear

(xi) SEQUENCE DESCRIPTION: SEQ ID NO:8:

Cys Asn Pro Cys Thr Glu Gly Val Asp Tyr Thr Asn Ala Ser Asn
1               5                   10                  15

Asn Glu Pro Ser Cys Phe Pro Cys Thr Val Cys Lys Ser Asp Gln
                20                  25                  30

Lys His Lys Ser Ser Cys Thr Met Thr Arg Asp Thr Val Cys Gln
                35                  40                  45

Cys Lys Glu

48

(2) INFORMATION FOR SEQ ID NO:9:

(i) SEQUENCE CHARACTERISTICS:
(A) LENGTH: 70 base pairs
(B) TYPE: Nucleic Acid
(C) STRANDEDNESS: Single
(D) TOPOLOGY: Linear

(xi) SEQUENCE DESCRIPTION: SEQ ID NO:9:

GGGAGCCGCT CATGAGGAAG TTGGGCCTCA TGGACAATGA GATAAAGGTG 50

GCTAAAGCTG AGGCAGCGGG 70

(2) INFORMATION FOR SEQ ID NO:10:

(i) SEQUENCE CHARACTERISTICS:
(A) LENGTH: 1799 base pairs
(B) TYPE: Nucleic Acid
(C) STRANDEDNESS: Single
(D) TOPOLOGY: Linear

(xi) SEQUENCE DESCRIPTION: SEQ ID NO:10:

CCCACGCGTC CGCATAAATC AGCACGCGGC CGGAGAACCC CGCAATCTCT 50

GCGCCCACAA AATACACCGA CGATGCCCGA TCTACTTTAA GGGCTGAAAC 100

CCACGGGCCT GAGAGACTAT AAGAGCGTTC CCTACCGCC ATG GAA 145
Met Glu
1

CAA CGG GGA CAG AAC GCC CCG GCC GCT TCG GGG GCC CGG 184
Gln Arg Gly Gln Asn Ala Pro Ala Ala Ser Gly Ala Arg
        5                   10                  15

AAA AGG CAC GGC CCA GGA CCC AGG GAG GCG CGG GGA GCC 223
Lys Arg His Gly Pro Gly Pro Arg Glu Ala Arg Gly Ala
                20                  25

AGG CCT GGG CTC CGG GTC CCC AAG ACC CTT GTG CTC GTT 262
Arg Pro Gly Leu Arg Val Pro Lys Thr Leu Val Leu Val
    30                  35                  40

GTC GCC GCG GTC CTG CTG TTG GTC TCA GCT GAG TCT GCT 301
Val Ala Ala Val Leu Leu Leu Val Ser Ala Glu Ser Ala
            45                  50

CTG ATC ACC CAA CAA GAC CTA GCT CCC CAG CAG AGA GCG 340
Leu Ile Thr Gln Gln Asp Leu Ala Pro Gln Gln Arg Ala
55                  60                  65

GCC CCA CAA CAA AAG AGG TCC AGC CCC TCA GAG GGA TTG 379
Ala Pro Gln Gln Lys Arg Ser Ser Pro Ser Glu Gly Leu

        70                  75                  80

TGT CCA CCT GGA CAC CAT ATC TCA GAA GAC GGT AGA GAT 418
Cys Pro Pro Gly His His Ile Ser Glu Asp Gly Arg Asp
                85                  90

TGC ATC TCC TGC AAA TAT GGA CAG GAC TAT AGC ACT CAC 457
Cys Ile Ser Cys Lys Tyr Gly Gln Asp Tyr Ser Thr His
    95                  100                 105

TGG AAT GAC CTC CTT TTC TGC TTG CGC TGC ACC AGG TGT 496
Trp Asn Asp Leu Leu Phe Cys Leu Arg Cys Thr Arg Cys
            110                 115

GAT TCA GGT GAA GTG GAG CTA AGT CCC TGC ACC ACG ACC 535
Asp Ser Gly Glu Val Glu Leu Ser Pro Cys Thr Thr Thr
120                 125                 130

AGA AAC ACA GTG TGT CAG TGC GAA GAA GGC ACC TTC CGG 574
Arg Asn Thr Val Cys Gln Cys Glu Glu Gly Thr Phe Arg
        135                 140                 145

GAA GAA GAT TCT CCT GAG ATG TGC CGG AAG TGC CGC ACA 613
Glu Glu Asp Ser Pro Glu Met Cys Arg Lys Cys Arg Thr
                150                 155

GGG TGT CCC AGA GGG ATG GTC AAG GTC GGT GAT TGT ACA 652
Gly Cys Pro Arg Gly Met Val Lys Val Gly Asp Cys Thr
    160                 165                 170

CCC TGG AGT GAC ATC GAA TGT GTC CAC AAA GAA TCA GGC 691
Pro Trp Ser Asp Ile Glu Cys Val His Lys Glu Ser Gly
            175                 180

ATC ATC ATA GGA GTC ACA GTT GCA GCC GTA GTC TTG ATT 730
Ile Ile Ile Gly Val Thr Val Ala Ala Val Val Leu Ile
185                 190                 195

GTG GCT GTG TTT GTT TGC AAG TCT TTA CTG TGG AAG AAA 769
Val Ala Val Phe Val Cys Lys Ser Leu Leu Trp Lys Lys
        200                 205                 210

GTC CTT CCT TAC CTG AAA GGC ATC TGC TCA GGT GGT GGT 808
Val Leu Pro Tyr Leu Lys Gly Ile Cys Ser Gly Gly Gly
                215                 220

GGG GAC CCT GAG CGT GTG GAC AGA AGC TCA CAA CGA CCT 847
Gly Asp Pro Glu Arg Val Asp Arg Ser Ser Gln Arg Pro
    225                 230                 235

GGG GCT GAG GAC AAT GTC CTC AAT GAG ATC GTG AGT ATC 886
Gly Ala Glu Asp Asn Val Leu Asn Glu Ile Val Ser Ile
            240                 245

TTG CAG CCC ACC CAG GTC CCT GAG CAG GAA ATG GAA GTC 925
Leu Gln Pro Thr Gln Val Pro Glu Gln Glu Met Glu Val
250                 255                 260

CAG GAG CCA GCA GAG CCA ACA GGT GTC AAC ATG TTG TCC 964
Gln Glu Pro Ala Glu Pro Thr Gly Val Asn Met Leu Ser
        265                 270                 275

CCC GGG GAG TCA GAG CAT CTG CTG GAA CCG GCA GAA GCT 1003
Pro Gly Glu Ser Glu His Leu Leu Glu Pro Ala Glu Ala
                280                 285

GAA AGG TCT CAG AGG AGG AGG CTG CTG GTT CCA GCA AAT 1042
Glu Arg Ser Gln Arg Arg Arg Leu Leu Val Pro Ala Asn
    290                 295                 300

GAA GGT GAT CCC ACT GAG ACT CTG AGA CAG TGC TTC GAT 1081
Glu Gly Asp Pro Thr Glu Thr Leu Arg Gln Cys Phe Asp
            305                 310

GAC TTT GCA GAC TTG GTG CCC TTT GAC TCC TGG GAG CCG 1120
Asp Phe Ala Asp Leu Val Pro Phe Asp Ser Trp Glu Pro
315                 320                 325

CTC ATG AGG AAG TTG GGC CTC ATG GAC AAT GAG ATA AAG 1159
Leu Met Arg Lys Leu Gly Leu Met Asp Asn Glu Ile Lys
        330                 335                 340

GTG GCT AAA GCT GAG GCA GCG GGC CAC AGG GAC ACC TTG 1198
Val Ala Lys Ala Glu Ala Ala Gly His Arg Asp Thr Leu
                345                 350

TAC ACG ATG CTG ATA AAG TGG GTC AAC AAA ACC GGG CGA 1237
Tyr Thr Met Leu Ile Lys Trp Val Asn Lys Thr Gly Arg
    355                 360                 365

GAT GCC TCT GTC CAC ACC CTG CTG GAT GCC TTG GAG ACG 1276
Asp Ala Ser Val His Thr Leu Leu Asp Ala Leu Glu Thr
            370                 375

CTG GGA GAG AGA CTT GCC AAG CAG AAG ATT GAG GAC CAC 1315
Leu Gly Glu Arg Leu Ala Lys Gln Lys Ile Glu Asp His
380                 385                 390

TTG TTG AGC TCT GGA AAG TTC ATG TAT CTA GAA GGT AAT 1354
Leu Leu Ser Ser Gly Lys Phe Met Tyr Leu Glu Gly Asn
        395                 400                 405

GCA GAC TCT GCC WTG TCC TAAGTGTG ATTCTCTTCA GGAAGTGAGA 1400
Ala Asp Ser Ala Xaa Ser
410 411

CCTTCCCTGG TTTACCTTTT TTCTGGAAAA AGCCCAACTG GACTCCAGTC 1450

AGTAGGAAAG TGCCACAATT GTCACATGAC CGGTACTGGA AGAAACTCTC 1500

CCATCCAACA TCACCCAGTG GATGGAACAT CCTGTAACTT TTCACTGCAC 1550

TTGGCATTAT TTTTATAAGC TGAATGTGAT AATAAGGACA CTATGGAAAT 1600

GTCTGGATCA TTCCGTTTGT GCGTACTTTG AGATTTGGTT TGGGATGTCA 1650

TTGTTTTCAC AGCACTTTTT TATCCTAATG TAAATGCTTT ATTTATTTAT 1700

TTGGGCTACA TTGTAAGATC CATCTACAAA AAAAAAAAAA AAAAAAAAAG 1750

GGCGGCCGCG ACTCTAGAGT CGACCTGCAG AAGCTTGGCC GCCATGGCC 1799

(2) INFORMATION FOR SEQ ID NO:11:

(i) SEQUENCE CHARACTERISTICS:
(A) LENGTH: 411 amino acids
(B) TYPE: Amino Acid
(D) TOPOLOGY: Linear

(xi) SEQUENCE DESCRIPTION: SEQ ID NO:11:

Met Glu Gln Arg Gly Gln Asn Ala Pro Ala Ala Ser Gly Ala Arg
1               5                   10                  15

Lys Arg His Gly Pro Gly Pro Arg Glu Ala Arg Gly Ala Arg Pro
                20                  25                  30

Gly Leu Arg Val Pro Lys Thr Leu Val Leu Val Val Ala Ala Val
                35                  40                  45

Leu Leu Leu Val Ser Ala Glu Ser Ala Leu Ile Thr Gln Gln Asp
                50                  55                  60

Leu Ala Pro Gln Gln Arg Ala Ala Pro Gln Gln Lys Arg Ser Ser
                65                  70                  75

Pro Ser Glu Gly Leu Cys Pro Pro Gly His His Ile Ser Glu Asp
                80                  85                  90

Gly Arg Asp Cys Ile Ser Cys Lys Tyr Gly Gln Asp Tyr Ser Thr
                95                  100                 105

His Trp Asn Asp Leu Leu Phe Cys Leu Arg Cys Thr Arg Cys Asp
                110                 115                 120

Ser Gly Glu Val Glu Leu Ser Pro Cys Thr Thr Thr Arg Asn Thr
                125                 130                 135

Val Cys Gln Cys Glu Glu Gly Thr Phe Arg Glu Glu Asp Ser Pro
                140                 145                 150

Glu Met Cys Arg Lys Cys Arg Thr Gly Cys Pro Arg Gly Met Val
                155                 160                 165

Lys Val Gly Asp Cys Thr Pro Trp Ser Asp Ile Glu Cys Val His
                170                 175                 180

Lys Glu Ser Gly Ile Ile Ile Gly Val Thr Val Ala Ala Val Val
                185                 190                 195

Leu Ile Val Ala Val Phe Val Cys Lys Ser Leu Leu Trp Lys Lys
                200                 205                 210

Val Leu Pro Tyr Leu Lys Gly Ile Cys Ser Gly Gly Gly Gly Asp

                215                 220                 225

Pro Glu Arg Val Asp Arg Ser Ser Gln Arg Pro Gly Ala Glu Asp
                230                 235                 240

Asn Val Leu Asn Glu Ile Val Ser Ile Leu Gln Pro Thr Gln Val
                245                 250                 255

Pro Glu Gln Glu Met Glu Val Gln Glu Pro Ala Glu Pro Thr Gly
                260                 265                 270

Val Asn Met Leu Ser Pro Gly Glu Ser Glu His Leu Leu Glu Pro
                275                 280                 285

Ala Glu Ala Glu Arg Ser Gln Arg Arg Arg Leu Leu Val Pro Ala
                290                 295                 300

Asn Glu Gly Asp Pro Thr Glu Thr Leu Arg Gln Cys Phe Asp Asp
                305                 310                 315

Phe Ala Asp Leu Val Pro Phe Asp Ser Trp Glu Pro Leu Met Arg
                320                 325                 330

Lys Leu Gly Leu Met Asp Asn Glu Ile Lys Val Ala Lys Ala Glu
                335                 340                 345

Ala Ala Gly His Arg Asp Thr Leu Tyr Thr Met Leu Ile Lys Trp
                350                 355                 360

Val Asn Lys Thr Gly Arg Asp Ala Ser Val His Thr Leu Leu Asp
                365                 370                 375

Ala Leu Glu Thr Leu Gly Glu Arg Leu Ala Lys Gln Lys Ile Glu
                380                 385                 390

Asp His Leu Leu Ser Ser Gly Lys Phe Met Tyr Leu Glu Gly Asn
                395                 400                 405

Ala Asp Ser Ala Xaa Ser
410 411

(2) INFORMATION FOR SEQ ID NO:12:

(i) SEQUENCE CHARACTERISTICS:
(A) LENGTH: 29 base pairs
(B). TYPE: Nucleic Acid
(C) STRANDEDNESS: Single
(D) TOPOLOGY: Linear

(xi) SEQUENCE DESCRIPTION: SEQ ID NO:12:

ATCAGGGACT TTCCGCTGGG GACTTTCCG 29

(2) INFORMATION FOR SEQ ID NO:13:

(i) SEQUENCE CHARACTERISTICS:
(A) LENGTH: 30 base pairs

(B) TYPE: Nucleic Acid
(C) STRANDEDNESS: Single
(D) TOPOLOGY: Linear

(xi) SEQUENCE DESCRIPTION: SEQ ID NO:13:

AGGATGGGAA GTGTGTGATA TATCCTTGAT 30

## Description

### FIELD OF THE INVENTION

The present invention relates generally to the identification, isolation, and recombinant production of novel polypeptides, designated herein as "Apo-2DcR" and to anti-Apo-2DcR antibodies.

### BACKGROUND OF THE INVENTION

### Apoptosis or "Programmed Cell Death"

Control of cell numbers in mammals is believed to be determined, in part, by a balance between cell proliferation and cell death. One form of cell death, sometimes referred to as necrotic cell death, is typically characterized as a pathologic form of cell death resulting from some trauma or cellular injury. In contrast, there is another, "physiologic" form of cell death which usually proceeds in an orderly or controlled manner. This orderly or controlled form of cell death is often referred to as "apoptosis" [see. *e.g.*, Barr et al., Bio/Technology, 12:487-493 (1994); Steller et al., Science, 267:1445-1449 (1995)]. Apoptotic cell death naturally occurs in many physiological processes, including embryonic development and clonal selection in the immune system [Itoh et al., Cell, 66:233-243 (1991)]. Decreased levels of apoptotic cell death have been associated with a variety of pathological conditions, including cancer, lupus, and herpes virus infection [Thompson, Science, 267:1456-1462 (1995)]. Increased levels of apoptotic cell death may be associated with a variety of other pathological conditions, including AIDS, Alzheimer's disease, Parkinson's disease, amyotrophic lateral sclerosis, multiple sclerosis, retinitis pigmentosa, cerebellar degeneration, aplastic anemia, myocardial infarction, stroke, reperfusion injury, and toxin-induced liver disease [see, Thompson, supra].

Apoptotic cell death is typically accompanied by one or more characteristic morphological and biochemical changes in cells, such as condensation of cytoplasm, loss of plasma membrane microvilli, segmentation of the nucleus, degradation of chromosomal DNA or loss of mitochondrial function. A variety of extrinsic and intrinsic signals are believed to trigger or induce such morphological and biochemical cellular changes [Raff, Nature, 356:397-400 (1992); Steller, supra; Sachs et al., Blood, 82:15 (1993)]. For instance, they can be triggered by hormonal stimuli, such as glucocorticoid hormones for immature thymocytes, as well as withdrawal of certain growth factors [Watanabe-Fukunagaet al., Nature, 356:314-317 (1992)]. Also, some identified oncogenes such as *myc, rel*, and *E1A*, and tumor suppressors, like *p53*, have been reported to have a role in inducing apoptosis. Certain chemotherapy drugs and some forms of radiation have likewise been observed to have apoptosis-inducing activity [Thompson, supra].

### TNF Family of Cytokines

Various molecules, such as tumor necrosis factor-α ("TNF-α"), tumor necrosis factor-β ("TNF-β" or "lymphotoxin"),CD30 ligand, CD27 ligand, CD40 ligand, OX-40 ligand, 4-1 BB ligand, Apo-1 ligand (also referred to as Fas ligand or CD95 ligand), and Apo-2 ligand (also referred to as TRAIL) have been identified as members of the tumor necrosis factor ("TNF") family of cytokines [See, e.g., Gruss and Dower, Blood 85:3378-3404 (1995); Wiley et al., Immunity, 3:673-682 (1995); Pitti et al., J. Biol. Chem., 271:12687-12690 (1996)]. Among these molecules, TNF-α, TNF-β, CD30 ligand, 4-1BB ligand, Apo-1 ligand, and Apo-2 ligand (TRAIL) have been reported to be involved in apoptotic cell death. Both TNF-α and TNF-β have been reported to induce apoptotic death in susceptible tumor cells [Schmid et al., Proc. Natl. Acad. Sci., 83:1881 (1986); Dealtry et al., Eur. J. Immunol., 17:689 (1987)]. Zheng et al. have reported that TNF-α is involved in post-stimulation apoptosis of CD8-positive T cells [Zheng et al., Nature, 377:348-351 (1995)]. Other investigators have reported that CD30 ligand may be involved in deletion of self-reactive T cells in the thymus [Amakawa et al., Cold Spring Harbor Laboratory Symposium on Programmed Cell Death, Abstr. No. 10, (1995)].

Mutations in the mouse Fas/Apo-1 receptor or ligand genes (called *lpr* and *gld*, respectively) have been associated with some autoimmune disorders, indicating that Apo-1 ligand may play a role in regulating the clonal deletion of self-reactive lymphocytes in the periphery [Krammeret al., Curr. Op. Immunol., 6:279-289 (1994); Nagata et al., Science, 267:1449-1456 (1995)]. Apo-1 ligand is also reported to induce post-stimulation apoptosis in CD4-positive T lymphocytes and in B lymphocytes, and may be involved in the elimination of activated lymphocytes when their function is no longer needed [Krammer et al., supra; Nagata et al., supra]. Agonist mouse monoclonal antibodies specifically binding to the Apo-1 receptor have been reported to exhibit cell killing activity that is comparable to or similar to that of TNF-α [Yonehara et al., J. Exp. Med., 169:1747-1756 (1989)].

### TNF Family of Receptors

Induction of various cellular responses mediated by such TNF family cytokines is believed to be initiated by their binding to specific cell receptors. Two distinct TNF receptors of approximately 55-kDa (TNFR1) and 75-kDa (TNFR2) have been identified[Hohman et al., J. Biol. Chem., 264:14927-14934(1989); Brockhaus et al., Proc. Natl. Acad. Sci., 87:3127-3131 (1990): EP 417.563, published March 20, 1991] and human and mouse cDNAs corresponding to both receptor types have been isolated and characterized [Loetscheret al., Cell, 61:351 (1990); Schall et al., Cell, 61:361 (1990); Smith et al., Science, 248:1019-1023 (1990), Lewis et al., Proc. Natl. Acad. Sci., 88:2830-2834 (1991); Goodwin et al., Mol. Cell. Biol., 11:3020-3026 (1991)]. Extensive polymorphisms have been associated with both TNF receptor genes [see, e.g., Takao et al., Immunogenetics, 37:199-203 (1993)]. Both TNFRs share the typical structure of cell surface receptors including extracellular, transmembrane and intracellularregions. The extracellular portions of both receptors are found naturally also as soluble TNF-binding proteins [Nophar, Y. et al., EMBO J., 9:3269 (1990); and Kohno, T. et al., Proc. Natl. Acad. Sci. U.S.A., 87:8331 (1990)]. More recently, the cloning of recombinant soluble TNF receptors was reported by Hale et al. [J. Cell. Biochem. Supplement 15F, 1991, p. 113 (P424)].

The extracellular portion of type 1 and type 2 TNFRs (TNFR1 and TNFR2) contains a repetitive amino acid sequence pattern of four cysteine-rich domains (CRDs) designated 1 through 4, starting from the NH₂-terminus. Each CRD is about 40 amino acids long and contains 4 to 6 cysteine residues at positions which are well conserved [Schall et al., supra; Loetscheret al., supra; Smith et al., supra; Nophar et al., supra; Kohno et al.. supra]. In TNFR1, the approximate boundaries of the four CRDs are as follows: CRD1- amino acids 14 to about 53; CRD2- amino acids from about 54 to about 97; CRD3- amino acids from about 98 to about 138; CRD4- amino acids from about 139 to about 167. In TNFR2, CRD1 includes amino acids 17 to about 54; CRD2-amino acids from about 55 to about 97; CRD3- amino acids from about 98 to about 140; and CRD4- amino acids from about 141 to about 179 [Banner et al., Cell, 73:431-435 (1993)]. The potential role of the CRDs in ligand binding is also described by Banner et al., supra.

A similar repetitive pattern of CRDs exists in several other cell-surface proteins, including the p75 nerve growth factor receptor (NGFR) [Johnson et al., Cell, 47:545 (1986); Radeke et al., Nature, 325:593 (1987)], the B cell antigen CD40 [Stamenkovic et al., EMBO J., 8:1403 (1989)], the T cell antigen OX40 [Mallet et al., EMBO J., 9:1063 (1990)] and the Fas antigen [Yonehara et al., supra and Itoh et al., supra]. CRDs are also found in the soluble TNFR (sTNFR)-like T2 proteins of the Shope and myxoma poxviruses [Upton et al., Virology, 160:20-29 (1987); Smith et al., Biochem. Biophys. Res. Commun., 176:335 (1991); Upton et al., Virology, 184:370 (1991)]. Optimal alignment of these sequences indicates that the positions of the cysteine residues are well conserved. These receptors are sometimes collectively referred to as members of the TNF/NGF receptor superfamily. Recent studies on p75NGFR showed that the deletion of CRD1 [Welcher, A.A. et al., Proc. Natl. Acad. Sci. USA, 88:159-163 (1991)] or a 5-amino acid insertion in this domain [Yan, H. and Chao. M.V., J. Biol. Chem., 266:12099-12104(1991)] had little or no effect on NGF binding [Yan. H. and Chao. M.V., supra]. p75 NGFR contains a proline-rich stretch of about 60 amino acids, between its CRD4 and transmembrane region, which is not involved in NGF binding [Peetre, C. et al., Eur. J. Hematol., 41:414-419 (1988); Seckinger, P. et al., J. Biol. Chem., 264:11966-11973 (1989); Yan, H. and Chao, M.V.. supra]. A similar proline-rich region is found in TNFR2 but not in TNFR1.

Itoh et al. disclose that the Apo-1 receptor can signal an apoptotic cell death similar to that signaled by the 55-kDa TNFR1 [Itoh et al., supra]. Expression of the Apo-1 antigen has also been reported to be down-regulated along with that of TNFR1 when cells are treated with either TNF-α or anti-Apo-1 mouse monoclonal antibody [Krammer et al., supra: Nagata et al., supra]. Accordingly, some investigators have hypothesized that cell lines that co-express both Apo-1 and TNFR1 receptors may mediate cell killing through common signaling pathways [Id.].

The TNF family ligands identified to date, with the exception of lymphotoxin-α, are type II transmembrane proteins, whose C-terminus is extracellular. In contrast, the receptors in the TNF receptor (TNFR) family identified to date are type I transmembrane proteins. In both the TNF ligand and receptor families, however, horology identified between family members has been found mainly in the extracellular domain ("ECD"). Several of the TNF family cytokines, including TNF-α, Apo-1 ligand and CD40 ligand, are cleaved proteolytically at the cell surface; the resulting protein in each case typically forms a homotrimeric molecule that functions as a soluble cytokine. TNF receptor family proteins are also usually cleaved proteolytically-to release soluble receptor ECDs that can function as inhibitors of the cognate cytokines.

Recently, other members of the TNFR family have been identified. In Marsters et al., Curr. Biol., 6:750 (1996), investigators describe a full length native sequence human polypeptide, called Apo-3, which exhibits similarity to the TNFR family in its extracellular cysteine-rich repeats and resembles TNFR1 and CD95 in that it contains a cytoplasmic death domain sequence [see also Marsters et al., Curr. Biol., 6:1669 (1996)]. Apo-3 has also been referred to by other investigators as DR3, wsl-1 and TRAMP [Chinnaiyan et al., Science, 274:990 (1996); Kitson et al., Nature, 384:372 (1996); Bodmer et al., Immunity, 6:79 (1997)].

Pan et al. have disclosed another TNF receptor family member referred to as "DR4" [Pan et al., Science, 276:111-113 (1997)]. The DR4 was reported to contain a cytoplasmic death domain capable of engaging the cell suicide apparatus. Pan et al. disclose that DR4 is believed to be a receptor for the ligand known as Apo-2 ligand or TRAIL.

### The Apoptosis-Inducing Signaling Complex

As presently understood, the cell death program contains at least three important elements - activators, inhibitors, and effectors; in *C. elegans,* these elements are encoded respectively by three genes, *Ced-4*, *Ced*-*9* and *Ced-3* [Steller, Science, 267:1445 (1995); Chinnaiyan et al., Science, 275:1122-1126 (1997)]. Two of the TNFR family members, TNFR1 and Fas/Apo1 (CD95), can activate apoptotic cell death [Chinnaiyan and Dixit, Current Biology, 6:555-562 (1996); Fraser and Evan, Cell; 85:781-784 (1996)]. TNFR1 is also known to mediate activation of the transcription factor, NF-κB [Tartaglia et al., Cell, 74:845-853 (1993); Hsu et al., Cell, 84:299-308 (1996)]. In addition to some ECD homology, these two receptors share homology in their intracellulardomain (ICD) in an oligomerization interface known as the death domain [Tartaglia et al., supra; Nagata, Cell, 88:355 (1997)]. Death domains are also found in several metazoan proteins that regulate apoptosis, namely, the Drosophilaprotein, Reaper, and the mammalian proteins referred to as FADD/MORT1, TRADD, and RIP [Cleavelandand Ihle, Cell, 81:479-482 (1995)]. Using the yeast-two hybrid system. Raven et al. report the identification of protein, wsl-1, which binds to the TNFR1 death domain [Raven et al., Programmed Cell Death Meeting, September 20-24, 1995, Abstract at page 127; Raven et al., European Cytokine Network, 7:Abstr. 82 at page 210 (April-June 1996)]. The wsl-1 protein is described as being homologous to TNFR1 (48% identity) and having a restricted tissue distribution. According to Raven et al., the tissue distribution of wsl-1 is significantly different from the TNFR1 binding protein, TRADD.

Upon ligand binding and receptor clustering, TNFR1 and CD95 are believed to recruit FADD into a death-inducing signalling complex. CD95 purportedly binds FADD directly, while TNFR1 binds FADD indirectly via TRADD [Chinnaiyan et al., Cell, 81:505-512 (1995); Boldin et al.. J. Biol. Chem., 270:387-391 (1995); Hsu et al., supra; Chinnaiyan et al., J. Biol. Chem., 271:4961-4965 (1996)]. It has been reported that FADD serves as an adaptor protein which recruits the Ced-3-related protease, MACHα/FLICE (caspase 8), into the death signalling complex [Boldin et al., Cell, 85:803-81 (1996); Muzio et al., Cell, 85:817-827 (1996)]. MACHα/FLICE appears to be the trigger that sets off a cascade of apoptotic proteases, including the interleukin-1β converting enzyme (ICE) and CPP32/Yama, which may execute some critical aspects of the cell death programme [Fraser and Evan, supra].

It was recently disclosed that programmed cell death involves the activity of members of a family of cysteine proteases related to the *C. elegans* cell death gene, ced-3. and to the mammalian IL-1-converting enzyme, ICE. The activity of the ICE and CPP32/Yama proteases can be inhibited by the product of the cowpox virus gene, *crmA* [Ray et al., Cell, 69:597-604 (1992); Tewari et al., Cell, 81:801-809 (1995)]. Recent studies show that CrmA can inhibit TNFR1- and CD95-induced cell death [Enari et al., Nature, 375:78-81 (1995); Tewari et al., J. Biol. Chem., 270:3255-3260 (1995)].

As reviewed recently by Tewari et al., TNFR1, TNFR2 and CD40 modulate the expression of proinflammatory and costimulatory cytokines, cytokine receptors, and cell adhesion molecules through activation of the transcription factor, NF-κB [Tewari et al., Curr. Op. Genet, Develop., 6:39-44 (1996)]. NF-κB is the prototype of a family of dimeric transcription factors whose subunits contain conserved Rel regions [Verma et al., Genes Develop., 9:2723-2735 (1996); Baldwin, Ann. Rev. Immunol., 14:649-681 (1996)]. In its latent form, NF-κB is complexed with members of the IκB inhibitor family; upon inactivation of the IκB in response to certain stimuli, released NF-κB translocates to the nucleus where it binds to specific DNA sequences and activates gene transcription.

For a review of the TNF family of cytokines and their receptors, see Gruss and Dower, supra.

### SUMMARY OF THE INVENTION

Applicants have identified cDNA clones that encode novel polypeptides, designated in the present application as "Apo-2DcR." It is believed that Apo-2DcR is a member of the TNFR family; full-length native sequence human Apo-2DcR polypeptide exhibits similarity to the TNFR family in its extracellular cysteine-rich repeats. Applicants found that Apo-2DcR binds Apo-2 ligand (Apo-2L).

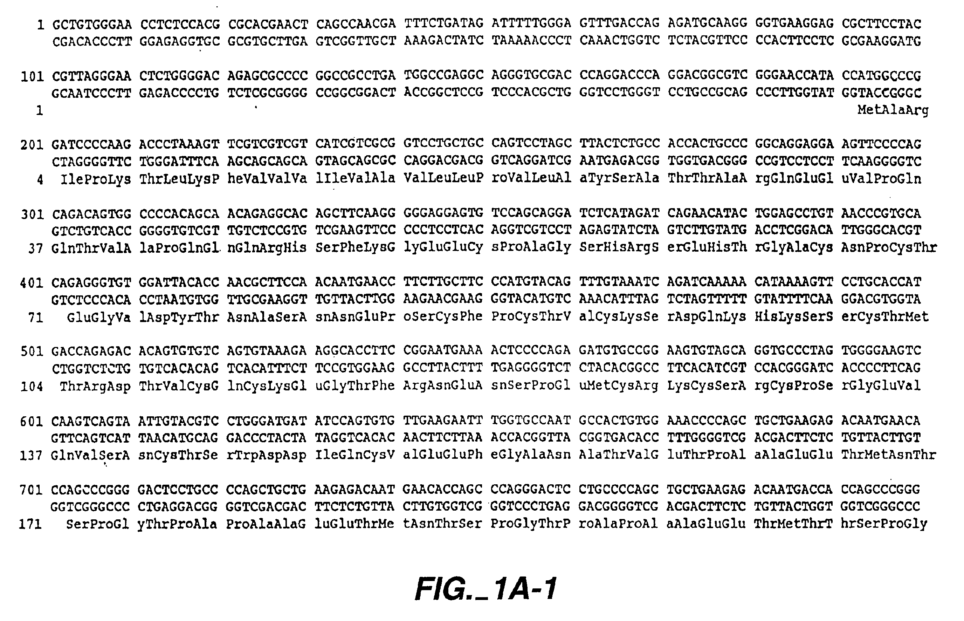

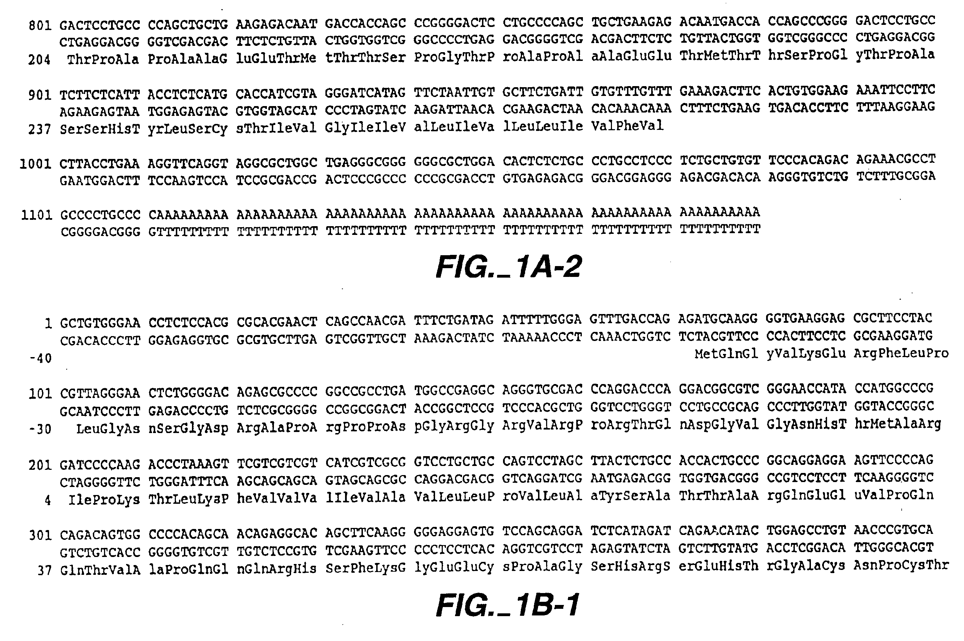

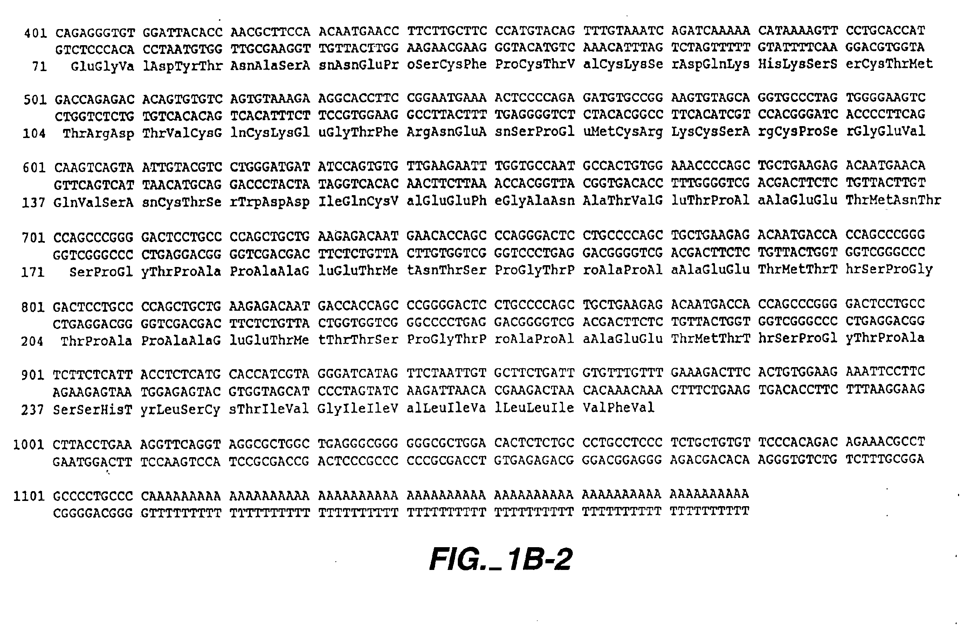

In one embodiment, the invention provides isolated Apo-2DcR polypeptide. In particular, the invention provides isolated native sequence Apo-2DcR polypeptide, which in one embodiment, includes an amino acid sequence comprising residues 1 to 259 of Figure 1A (SEQ ID NO:1). In other embodiments, the isolated Apo-2DcR polypeptide comprises at least about 80% amino acid sequence identity with native sequence Apo-2DcR polypeptide comprising residues 1 to 259 of Figure 1A (SEQ ID NO:1). Optionally, the isolated Apo-2DcR polypeptide includes an amino acid sequence comprising residues identified in Figure 1B as -40 to 259 (SEQ ID NO:3 Optionally, the Apo-2DcR polypeptide is obtained or obtainable by expressing the polypeptide encoded by the cDNA insert of the vector deposited as ATCC 209087.

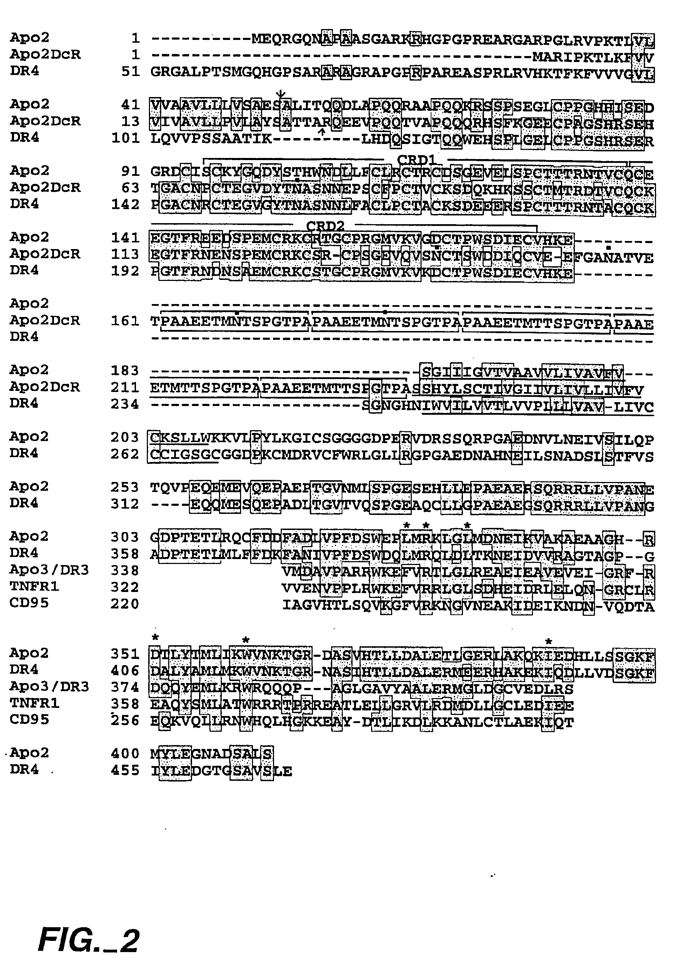

In another embodiment, the invention provides an isolated extracellular domain (ECD) sequence of Apo-2DcR. Optionally, the isolated extracellular domain sequence comprises amino acid residues 1 to 236 of Fig. 1A (SEQ ID NO:1) or residues 1 to 161 of Fig. 1A (SEQ ID NO:1). Optionally, the isolated extracellular domain sequence comprises an amino acid sequence wherein one or more of the amino acids identified in any of the Apo-2DcR pseudorepeats identified herein (See, Figure 2) have been deleted. Such isolated extracellular domain sequences may include polypeptides comprising a sequence of amino acid residues 1 to X. wherein X is any one of amino acid residues 161 to 236 of Figure 1A (SEQ ID NO:1).

In another embodiment, the invention provides chimeric molecules comprising Apo-2DcR polypeptide fused to a heterologous polypeptide or amino acid sequence. An example of such a chimeric molecule comprises an Apo-2DcR fused to an immunoglobulin sequence. Another example comprises an extracellular domain sequence of Apo-2DcR fused to a heterologouspolypeptideor amino acid sequence, such as an immunoglobulin sequence.

In another embodiment, the invention provides an isolated nucleic acid molecule encoding Apo-2DcR polypeptide. In one aspect, the nucleic acid molecule is RNA or DNA that encodes an Apo-2DcR polypeptide or a particular domain of Apo-2DcR, or is complementary to such encoding nucleic acid sequence, and remains stably bound to it under at least moderate, and optionally, under high stringency conditions. In one embodiment, the nucleic acid sequence is selected from:
(a) the coding region of the nucleic acid sequence of Figure 1A (SEQ ID NO:2) that codes for residue 1 to residue 259 (i.e., nucleotides 193-195 through 967-969), inclusive;
(b) the coding region of the nucleic acid sequence of Figure 1A (SEQ ID NO:2) that codes for residue 1 to residue 236 (i.e., nucleotides 193-195 through 898-900), inclusive;
(c) the coding region of the nucleic acid sequence of Figure 1B (SEQ ID NO:4) that codes for residue -40 to residue 259 (i.e., nucleotides 73-75 through 967-969), inclusive;
(d) a sequence correspondingto the sequence of (a), (b) or (c) within the scope of degeneracy of the genetic code.

In a further embodiment, the invention provides a vector comprising the nucleic acid molecule encoding the Apo-2DcR polypeptide or particular domain of Apo-2DcR. A host cell comprising the vector or the nucleic acid molecule is also provided. A method of producing Apo-2DcR is further provided.

In another embodiment, the invention provides an antibody which binds to Apo-2DcR. The antibody may be an agonistic, blocking or neutralizing antibody.

In another embodiment, the invention provides non-human, transgenic or knock-out animals.

A further embodiment of the invention provides articles of manufacture and kits that include Apo-2DcR or Apo-2DcR antibodies.

### BRIEF DESCRIPTION OF THE DRAWINGS

Figure 1A shows the nucleotide sequence of a native sequence human Apo-2DcR cDNA and its derived amino acid sequence (initiation site assigned at residue 1 (nucleotides 193-195)).
Figure 1B shows the nucleotide sequence of a native sequence human Apo-2DcR cDNA and its derived amino acid sequence (initiation site assigned at residue -40 (nucleotides 73-75)).
Figure 2 shows the primary structure and mRNA expression of Apo-2 and Apo-2DcR. The figure depicts the deduced amino acid sequences of human Apo-2 and Apo-2DcR aligned with full-length DR4. The death domain of Apo-2 is aligned with those of DR4, Apo-3/DR3, TNFR I, and CD95; asterisks indicate residues that are essential for death signaling by TNFR1 [Tartaglia et al., supra]. Indicated are the predicted signal peptide cleavage sites (arrows), the two cysteine-rich domains (CRD1, 2) and the transmembrane domain of Apo-2 and DR4 or the hydrophobic C-terminus of Apo-2DcR (underlined). Also indicated are the five potential N-linked glycosylation sites (black boxes) and the five sequence pseudo-repeats (brackets) of Apo-2DcR.
Figure 3 shows hydropathy plots of Apo-2 and Apo-2DcR. Numbers at the top indicate amino acid positions.
Figure 4 shows binding of radioiodinated Apo-2L to Apo-2DcR-transfected cells and its inhibition by pre-treatment of cells with PI-PLC.
Figure 5 shows inhibition of Apo-2L induction of apoptosis by Apo-2DcR.
Figure 6 shows inhibition of Apo-2L activation of NF-κB by Apo-2DcR.
Figure 7A shows expression of Apo-2DcR mRNA in human tissues as analyzed by Northern hybridization of human tissue poly A RNA blots.
Figure 7B shows (lack of) expression of Apo-2DcR mRNA in human cancer cell lines as analyzed by Northern hybridization of human cancer cell line poly A RNA blots.
Figure 8 shows the nucleotide sequence of a native sequence human Apo-2 cDNA and its derived amino acid sequence.
Figure 9 shows the derived amino acid sequence of a native sequence human Apo-2 - the putative signal sequence is underlined, the putative transmembrane domain is boxed, and the putative death domain sequence is dash underlined. The cysteines of the two cysteine-rich domains are individually underlined.
Figure 10 shows the interaction of the Apo-2 ECD with Apo-2L. Supernatants from mock-transfected 293 cells or from 293 cells transfected with Flag epitope-tagged Apo-2 ECD were incubated with poly-His-tagged Apo-2L and subjected to immuno precipitation with anti-Flag conjugated or Nickel conjugated agarose beads. The precipitated proteins were resolved by electrophoresis on polyacrylamide gels, and detected by immunoblot with anti-Apo-2L or anti-Flag antibody.

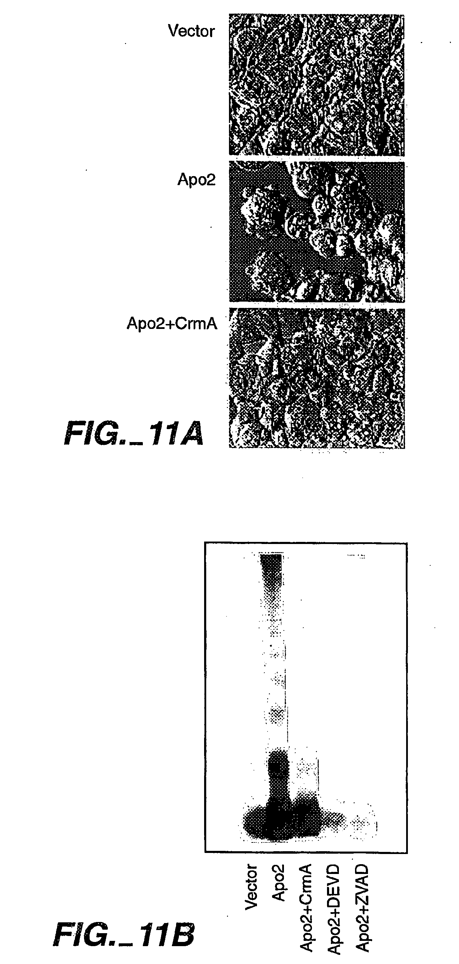

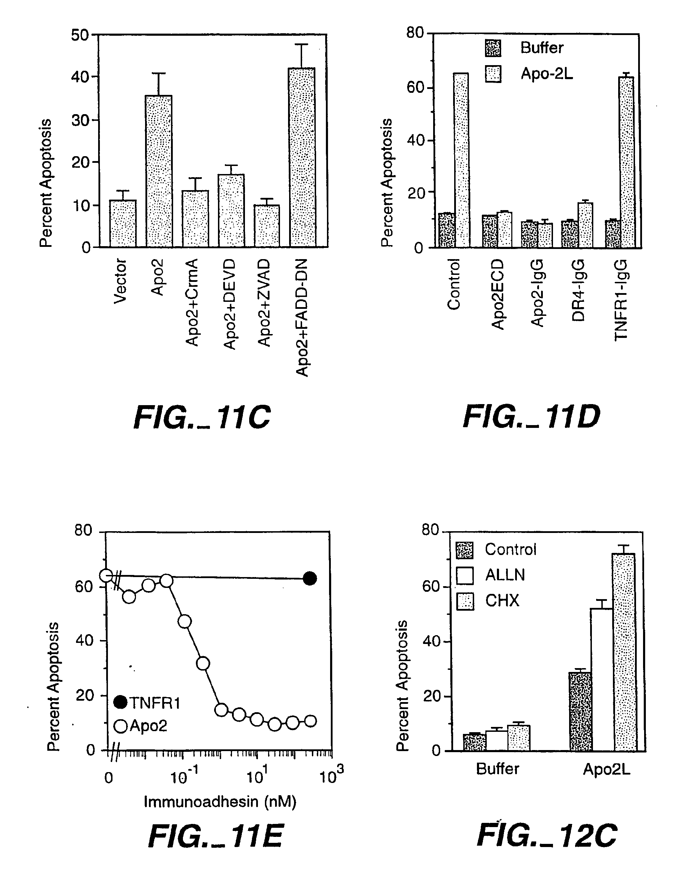

Figure 11 shows the induction of apoptosis by Apo-2 and inhibition of Apo-2L activity by soluble Apo-2 ECD. Human 293 cells (A, B) or HeLa cells (C) were transfected by pRK5 vector or by pRK5-based plasmids encoding Apo-2 and/or CrmA. Apoptosiswas assessed by morphology (A), DNA fragmentation(B), or by FACS (C-E). Soluble Apo-2L was pre-incubated with buffer or affinity-purified Apo-2 ECD together with anti-Flag antibody or Apo-2 ECD immunoadhesin or DR4 or TNFR1 immunoadhesins and added to HeLa cells. The cells were later analyzed for apoptosis(D). Dose-responseanalysis using Apo-2L with Apo-2 ECD immunoadhesin was also determined (E).
Figure 12 shows activation of NF-κB by Apo-2, DR4, and Apo-2L. (A) HeLa cells were transfected with expression plasmids encoding the indicated proteins. Nuclear extracts were prepared and analyzed by an electrophoretic mobility shift assay. (B) HeLa cells or MCF7 cells were treated with buffer, Apo-2L or TNF-alpha and assayed for NF-κB activity. (C) HeLa cells were preincubated with buffer, ALLN or cyclohexamide before addition of Apo-2L. Apoptosis was later analyzed by FACS.
Figure 13 shows expression of Apo-2 mRNA in human tissues as analyzed by Northern hybridization of human tissue poly A RNA blots.
Figure 14 shows the FACS analysis of Apo-2DcR antibodies (illustrated by the bold lines) as compared to IgG controls (dotted lines). The antibodies (4G3.9.9; 6D10.9.7; and 1C5.24.1 respectively) recognized the Apo-2DcR receptor expressed in HUMEC cells.
Figure 15 contains graphs showing results of ELISAs testing binding of Apo-2DcR antibodies 4G3.9.9; 6D 10.9.7; and IC5.24.1 respectively, to Apo-2DcR and to other known Apo-2L receptors referred to as DR4, Apo-2 and DcR2.
Figure 16 is a table providing a summary of isotype and cross-reactivity properties of antibodies IC5.24.1; 4G3.9.9; and 6D10.9.7.

### DETAILED DESCRIPTION OF THE PREFERRED EMBODIMENTS

### 1. Definitions

The terms "Apo-2DcR polypeptide" and "Apo-2DcR" when used herein encompass native sequence Apo-2DcR and Apo-2DcR variants (which are further defined herein). These terms encompass Apo-2DcR from a variety of mammals, including humans. The Apo-2DcR may be isolated from a variety of sources, such as from human tissue types or from another source, or prepared by recombinant or synthetic methods.

A "native sequence Apo-2DcR" comprises a polypeptide having the same amino acid sequence as an Apo-2DcR derived from nature. Thus, a native sequence Apo-2DcR can have the amino acid sequence of naturally-occurring Apo-2DcR from any mammal. Such native sequence Apo-2DcR can be isolated from nature or can be produced by recombinant or synthetic means. The term "native sequence Apo-2DcR" specifically encompasses naturally-occurringtruncated, secreted, or soluble forms of the Apo-2DcR (*e.g.*, an extracellular domain sequence), naturally-occurring variant forms (*e.g.*, alternatively spliced forms) and naturally-occurring allelic variants of the Apo-2DcR. In one embodiment of the invention, the native sequence Apo-2DcR is a mature or full-length native sequence Apo-2DcR comprising amino acids 1 to 259 of Fig. 1A (SEQ ID NO:1) or amino acids -40 to 259 of Figure 1B (SEQ ID NO:3). Optionally, the Apo-2DcR polypeptide is obtained or obtainable by expressing the polypeptide encoded by the cDNA insert of the vector deposited as ATCC 209087.

The "Apo-2DcR extracellular domain" or "Apo-2DcR ECD" refers to a form of Apo-2DcR which is essentially free of transmembrane and cytoplasmic domains. Ordinarily, Apo-2DcR ECD will have less than 1% of such transmembrane and cytoplasmic domains and preferably, will have less than 0.5% of such domains. Optionally, Apo-2DcR ECD will comprise amino acid residues 1 to 236 of Fig. 1A (SEQ ID NO:1) or amino acid residues 1 to 161 of Fig. 1A (SEQ ID NO:1). Optionally, the isolated extracellular domain sequence comprises an amino acid sequence wherein one or more of the amino acids identified in any of the Apo-2DcR pseudorepeats identified herein (See, Figure 2) have been deleted. Such isolated extracellular domain sequences may include polypeptides comprising a sequence of amino acid residues 1 to X, wherein X is any one of amino acid residues 161 to 236 of Figure 1A (SEQ ID NO:1).

"Apo-2DcR variant" means a biologically active Apo-2DcR as defined below having at least about 80% amino acid sequence identity with the Apo-2DcR having the deduced amino acid sequence shown in Fig. 1A (SEQ ID NO:1) for a full-length native sequence human Apo-2DcR or the sequences identified herein for Apo-2DcR ECD. Such Apo-2DcR variants include, for instance. Apo-2DcR polypeptides wherein one or more amino acid residues are added, or deleted, at the N- or C-terminus of the sequence of Fig. 1A (SEQ ID NO:1) or the sequences identified herein for Apo-2DcR ECD. Ordinarily, an Apo-2DcR variant will have at least about 80% amino acid sequence identity, more preferably at least about 90% amino acid sequence identity, and even more preferably at least about 95% amino acid sequence identity with the amino acid sequence of Fig. 1A (SEQ ID NO: 1).

"Percent (%) amino acid sequence identity" with respect to the Apo-2DcR sequences identified herein is defined as the percentage of amino acid residues in a candidate sequence that are identical with the amino acid residues in the Apo-2DcR sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as ALIGN or Megalign (DNASTAR) software. Those skilled in the art can determine appropri ate parameters for measuring alignment, including any algorithms needed to achieve maximal alignment over the full length of the sequences being compared.

The term "epitope tagged" when used herein refers to a chimeric polypeptide comprising Apo-2DcR, or a domain sequence thereof, fused to a "tag polypeptide". The tag polypeptide has enough residues to provide an epitope against which an antibody can be made, yet is short enough such that it does not interfere with activity of the Apo-2DcR. The tag polypeptide preferably also is fairly unique so that the antibody does not substantiallycross-react with other epitopes. Suitable tag polypeptides generally have at least six amino acid residues and usually between about 8 to about 50 amino acid residues (preferably, between about 10 to about 20 residues).

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or nonproteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducingor reducing conditions using Coomassie blue or, preferably, silver stain. Isolated polypeptide includes polypeptide *in situ* within recombinant cells, since at least one component of the Apo-2DcR natural environment will not be present. Ordinarily, however, isolated polypeptide will be prepared by at least one purification step.

An "isolated" Apo-2DcR nucleic acid molecule is a nucleic acid molecule that is identified and separated from at least one contaminant nucleic acid molecule with which it is ordinarily associated in the natural source of the Apo-2DcR nucleic acid. An isolated Apo-2DcR nucleic acid molecule is other than in the form or setting in which it is found in nature. Isolated Apo-2DcR nucleic acid molecules therefore are distinguished from the Apo-2DcR nucleic acid molecule as it exists in natural cells. However, an isolated Apo-2DcR nucleic acid molecule includes Apo-2DcR nucleic acid molecules contained in cells that ordinarily express Apo-2DcR where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadest sense and specifically covers single anti-Apo-2DcR monoclonal antibodies (including agonist, antagonist, and neutralizing antibodies) and anti-Apo-2DcR antibody compositions with polyepitopic specificity.

The term "monoclonal antibody" as used herein refers to an antibody obtained from a population of substantially homogeneous antibodies, *i.e.,* the individual antibodies comprising the population are identical except for possible naturally-occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen.

The monoclonal antibodies herein include hybrid and recombinant antibodies produced by splicing a variable (including hypervariable) domain of an anti-Apo-2DcR antibody with a constant domain (*e.g.* "humanized" antibodies), or a light chain with a heavy chain, or a chain from one species with a chain from another species, or fusions with heterologous proteins, regardless of species of origin or immunoglobulin class or subclass designation, as well as antibody fragments (*e.g.*, Fab, F(ab')₂, and Fv), so long as they exhibit the desired biological activity. See, *e.g.* U.S. Pat. No. 4,816,567 and Mage et al., in Monoclonal Antibody Production Techniques and Applications, pp.79-97 (Marcel Dekker, Inc.: New York, 1987).

Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler and Milstein, Nature, 256:495 (1975), or may be made by recombinant DNA methods such as described in U.S. Pat. No. 4,816,567. The "monoclonal antibodies" may also be isolated from phage libraries generated using the techniques described in McCafferty et al., Nature, 348:552-554 (1990), for example.

"Humanized" forms of non-human (*e.g.* murine) antibodies are specific chimeric immunoglobulins, immunoglobulin chains, or fragments thereof (such as Fv, Fab, Fab', F(ab')₂ or other antigen-binding subsequences of antibodies)which contain minimal sequence derived from non-human immunoglobulin. For the most part, humanized antibodies are human immunoglobulins(recipient antibody) in which residues from a complementary determining region (CDR) of the recipient are replaced by residues from a CDR of a non-human species (donor antibody) such as mouse, rat, or rabbit having the desired specificity, affinity, and capacity. In some instances. Fv framework region (FR) residues of the human immunoglobulin are replaced by correspondingnon-human residues. Furthermore, the humanized antibody may comprise residues which are found neither in the recipient antibody nor in the imported CDR or framework sequences. These modifications are made to further refine and optimize antibody performance. In general, the humanized antibody will comprise substantially all of at least one, and typically two, variable domains, in which all or substantially aU of the CDR regions correspond to those of a non-human immunoglobulin and all or substantially all of the FR regions are those of a human immunoglobulin consensus sequence. The humanized antibody optimally also will comprise at least a portion of an immunoglobulin constant region or domain (Fc), typically that of a human immunoglobulin.

"Biologically active" and "desired biological activity" for the purposes herein means (1) having the ability to modulate apoptosis (either in an agonistic or stimulating manner or in an antagonistic or blocking manner) in at least one type of mammalian cell *in vivo* or *ex vivo*; (2) having the ability to bind Apo-2 ligand; or (3) having the ability to modulate Apo-2 ligand signaling and Apo-2 ligand activity.

The terms "apoptosis" and "apoptotic activity" are used in a broad sense and refer to the orderly or controlled form of cell death in mammals that is typically accompanied by one or more characteristic cell changes, including condensation of cytoplasm, loss of plasma membrane microvilli, segmentation of the nucleus, degradation of chromosomal DNA or loss of mitochondrial function. This activity can be determined and measured, for instance, by cell viability assays, FACS analysis or DNA electrophoresis, all of which are known in the art.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers include squamous cell cancer, small-cell lung cancer, non-small cell lung cancer, blastoma, gastrointestinal cancer, renal cancer, pancreatic cancer, glioblastoma, neuroblastoma, cervical cancer, ovarian cancer, liver cancer, stomach cancer, bladder cancer, hepatoma, breast cancer, colon cancer, colorectal cancer, endometrial cancer, salivary gland cancer, kidney cancer, prostate cancer, vulval cancer, thyroid cancer, hepatic carcinoma, and various types of head and neck cancer.

The terms "treading." "treatment," and "therapy" as used herein refer to curative therapy, prophylactic therapy, and preventative therapy.

The term "mammal" as used herein refers to any mammal classified as a mammal, including humans, cows, horses, dogs and cats. In a preferred embodiment of the invention, the mammal is a human.

### II. Compositions and Methods of the Invention

The present invention provides newly identified and isolated Apo-2DcR polypeptides. In particular, Applicants have identified and isolated various human Apo-2DcR polypeptides. The properties and characteristics of some of these Apo-2DcR polypeptides are described in further detail in the Examples below. Based upon the properties and characteristicsof the Apo-2DcR polypeptides disclosed herein, it is Applicants' present belief that Apo-2DcR is a member of the TNFR family.

A description follows as to how Apo-2DcR, as well as Apo-2DcR chimeric molecules and anti-Apo-2DcR antibodies, may be prepared.

### A. Preparation of Apo-2DcR

The description below relates primarily to production of Apo-2DcR by culturing cells transformed or transfected with a vector containing Apo-2DcR nucleic acid. It is of course, contemplated that alternative methods, which are well known in the art, may be employed to prepare Apo-2DcR.

### 1. Isolation of DNA Encoring Apo-2DcR

The DNA encoding Apo-2DcR may be obtained from any cDNA library prepared from tissue believed to possess the Apo-2DcR mRNA and to express it at a detectable level. Accordingly, human Apo-2DcR DNA can be conveniently obtained from a cDNA library prepared from human tissues, such as libraries of human cDNA described in Example 1. The Apo-2DcR-encoding gene may also be obtained from a genomic library or by oligonucleotide synthesis.

Libraries can be screened with probes (such as antibodies to the Apo-2DcR or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning A Laboratory Manual (New York: Cold Spring Harbor z Laboratory Press, 1989). An alternative means to isolate the gene encoding Apo-2DcR is to use PCR methodology [Sambrook et al., supra: Dieffenbach et al., PCR Primer:A Laboratory Manual (Cold Spring Harbor Laboratory Press, 1995)].

One method of screening employs selected ol igonucleotide sequences to screen cDNA libraries from various human tissues. Example 1 below describes techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like ³²P-labeled ATP, biotinylation or enzyme labeling. Hybridization conditions, including moderate stringency and high stringency, are provided in Sambrook et al., supra.

Nucleic acid having all the protein coding sequence may be obtained by screening selected cDNA or genomic libraries using the deduced amino acid sequence disclosed herein for the first time, and, if necessary, using conventional primer extension procedures as described in Sambrook et al., supra, to detect precursors and processing intermediates of mRNA that may not have been reverse-transcribed into cDNA.

Apo-2DcR variants can be prepared by introducing appropriate nucleotide changes into the Apo-2DcR DNA, or by synthesis of the desired Apo-2DcRpolypeptide. Those skilled in the art will appreciate that amino acid changes may alter post-translational processes of the Apo-2DcR. such as changing the number or position of glycosylation sites or altering the membrane anchoring characteristics.

Variations in the native full-length sequence Apo-2DcR or in various domains of the Apo-2DcR described herein, can be made, for example, using any of the techniques and guidelines for conservative and non-conservative mutations set forth, for instance, in U.S. Pat. No. 5,364,934. Variations may be a substitution, deletion or insertion of one or more codons encoding the Apo-2DcR that results in a change in the amino acid sequence of the Apo-2DcRas compared with the native sequence Apo-2DcR. Optionally the variation is by substitution of at least one amino acid with any other amino acid in one or more of the domains of the Apo-2DcR molecule. The variations can be made using methods known in the art such as oligonucleotide-mediated(site-directed)mutagenesis,alanine scanning, and PCR mutagenesis. Site-directed mutagenesis [Carter et al., Nucl. Acids Res., 13:4331 (1986), Zoller et al., Nucl. Acids Res., 10:6487 (1982)], cassette mutagenesis [Wells et all, 34:315 (1985)], restriction selection mutagenesis [Wells et al., Philos. Trans. R. Soc. London SerA, 317:415 (1986)] or other known techniques can be performed on the cloned DNA to produce the Apo-2DcR variant DNA.

Scanning amino acid analysis can also be employed to identify one or more amino acids along a contiguous sequence which are involved in the interaction with a particular ligand or receptor. Among the preferred scanning amino acids are relatively small, neutral amino acids. Such amino acids include alanine, glycine, serine, and cysteine. Alanine is the preferred scanning amino acid among this group because it eliminates the side-chain beyond the beta-carbon and is less likely to alter the main-chain conformation of the variant. Alanine is also preferred because it is the most common amino acid. Further, it is frequently found in both buried and exposed positions [Creighton, The Proteins, (W.H. Freeman & Co., N.Y.); Chothia, J. Mol, Biol., 105:1 (1976)]. If alanine substitution does not yield adequate amounts of variant, an isoteric amino acid can be used.

Once selected Apo-2DcR variants are produced, they can be contacted with, for instance, Apo-2L, and the interaction, if any, can be determined. The interaction between the Apo-2DcR variant and Apo-2L can be measured by an *in vitro* assay, such as described in the Examples below. While any number of analytical measurements can be used to compare activities and properties between a native sequence Apo-2DcR and an Apo-2DcR variant, a convenient one for binding is the dissociation constant K_{d} of the complex formed between the Apo-2DcR variant and Apo-2L as compared to the K_{d} for the native sequence Apo-2DcR. Generally, a ≥ 3-fold increase or decrease in K_{d} per substituted residue indicates that the substituted residue(s) is active in the interaction of the native sequence Apo-2DcR with the Apo-2L.

Optionally, representativesites in the Apo-2DcR sequence suitable for mutagenesis (such as deletion of one or more amino acids) would include sites within the extracellular domain, and particularly, within one or more of the cysteine-rich domains or within one or more of the pseudorepeats. Such variations can be accomplished using the methods described above.

### 2. Insertion of Nucleic Acid into A Replicable Vector

The nucleic acid (*e.g*., cDNA or genomic DNA) encoding Apo-2DcR may be inserted into a replicable vector for further cloning (amplification of the DNA) or for expression. Various vectors are publicly available. The vector components generally include, but are not limited to, one or more of the following: a signal sequence, an origin of replication, one or more marker genes, an enhancer element, a promoter, and a transcription termination sequence, each of which is described below.

### (i) Signal Sequence Component

The Apo-2DcR may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the Apo-2DcR DNA that is inserted into the vector. The heterologous signal sequence selected preferably is one that is recognized and processed (i.e., cleaved by a signal peptidase) by the host cell. The signal sequence may be a prokaryotic signal sequence selected, for example, from the group of the alkaline phosphatase, penicillinase, lpp, or heat-stable enterotoxin II leaders. For yeast secretion the signal sequence may be, *e.g*., the yeast invertase leader, alpha factor leader (including *Saccharomyces* and *Kluyveromyces* α-factor leaders, the latter described in U.S. Pat. No. 5,010,182), or acid phosphatase leader, the *C. albicans* glucoamylase leader (EP 362,179 published 4 April 1990), or the signal described in WO 90/13646 published 15 November 1990. In mammalian cell expression the native Apo-2DcR presequencethat normally directs insertion of Apo-2DcR in the cell membrane of human cells *in vivo* is satisfactory, although other mammalian signal sequences may be used to direct secretion of the protein, such as signal sequences from secreted polypeptides of the same or related species, as well as viral secretory leaders, for example, the herpes simplex glycoprotein D signal.

The DNA for such precursor region is preferably ligated in reading frame to DNA encoding Apo-2DcR.

### (ii) Origin of Replication Component

Both expression and cloning vectors contain a nucleic acid sequence that enables the vector to replicate in one or more selected host cells. Generally, in cloning vectors this sequence is one that enables the vector to replicate independently of the host chromosomal DNA, and includes origins of replication or autonomously replicating sequences. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells. Generally, the origin of replication component is not needed for mammalian expression vectors (the SV40 origin may typically be used because it contains the early promoter).

Most expression vectors are "shuttle" vectors, i.e., they are capable of replication in at least one class of organisms but can be transfected into another organism for expression. For example, a vector is cloned in *E. coli* and then the same vector is transfected into yeast or mammalian cells for expression even though it is not capable of replicating independently of the host cell chromosome.

DNA may also be amplified by insertion into the host genome. This is readily accomplished using *Bacillus* species as hosts, for example, by including in the vector a DNA sequence that is complementary to a sequence found in *Bacillus* genomic DNA. Transfectionof *Bacillus* with this vector results in homologous recombination with the genome and insertion of Apo-2DcR DNA. However, the recovery of genomic DNA encoding Apo-2DcR is more complex than that of an exogenously replicated vector because restriction enzyme digestion is required to excise the Apo-2DcR DNA.

### (iii) Selection Gene Component

Expression and cloning vectors typically contain a selection gene, also termed a selectable marker. This gene encodes a protein necessary for the survival or growth of transformed host cells grown in a selective culture medium. Host cells not transformed with the vector containing the selection gene will not survive in the culture medium. Typical selection genes encode proteins that (a) confer resistance to antibiotics or other toxins, *e.g*., ampicillin, neomycin, methotrexate, or tetracycline, (b) complement auxotrophic deficiencies, or (c) supply critical nutrients not available from complex media, *e.g*., the gene encoding D-alanine racemase for *Bacilli.*

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin [Southern et al., J. Molec. Appl. Genet., 1:327 (1982)], mycophenolic acid (Mulligan et al., Science, 209: 1422 (1980)] or hygromycin [Sugden et al., Mol. Cell. Biol., 5:410-413 (1985)]. The three examples given above employ bacterial genes under eukaryotic control to convey resistance to the appropriate drug G418 or neomycin (geneticin), xgpt (mycophenolic acid), or hygromycin, respectively.

Another example of suitable selectable markers for mammalian cells are those that enable the identification of cells competent to take up the Apo-2DcR nucleic acid, such as DHFR or thymidine kinase. The mammalian cell transformants are placed under selection pressure that only the transformants are uniquely adapted to survive by virtue of having taken up the marker. Selection pressure is imposed by culturing the transformants under conditions in which the concentration of selection agent in the medium is successively changed, thereby leading to amplification of both the selection gene and the DNA that encodes Apo-2DcR. Amplification is the process by which genes in greater demand for the production of a protein critical for growth are reiterated in tandem within the chromosomes of successive generations of recombinant cells. Increased quantities of Apo-2DcR are synthesized from the amplified DNA. Other examples of amplifiable genes include metallothionein -I and -II, adenosine deaminase, and ornithine decarboxylase.

Cells transformed with the DHFR selection gene may first be identified by culturing all of the transformants in a culture medium that contains methotrexate (Mtx), a competitive antagonist of DHFR. An appropriate host cell when wild-type DHFR is employed is the Chinese hamster ovary (CHO) cell line deficient in DHFR activity, prepared and propagated as described by Urlaub et al., Proc. Natl. Acad. Sci. USA, 77:4216 (1980). The transformed cells are then exposed to increased levels of methotrexate. This leads to the synthesis of multiple copies of the DHFR gene, and, concomitantly, multiple copies of other DNA comprising the expression vectors, such as the DNA encoding Apo-2DcR. This amplification technique can be used with any otherwise suitable host. *e.g*., ATCC No. CCL61 CHO-K1, notwithstanding the presence of endogenous DHFR if, for example, a mutant DHFR gene that is highly resistant to Mtx is employed (EP 117,060).

Alternatively, host cells (particularly wild-type hosts that contain endogenous DHFR) transformed or co-transformed with DNA sequences encoding Apo-2DcR, wild-type DHFR protein, and another selectable marker such as aminoglycoside 3'-phosphotransferase (APH) can be selected by cell growth in medium containing selection agent for the selectable marker such as an aminoglycosidic antibiotic, *e.g.*, kanamycin, neomycin, or G418. See U.S. Patent No. 4,965,199.

A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10:157 (1980)]. The *trp*1 gene provides a selection marker for a mutant strain of yeast lacking the ability to grow in tryptophan. for example, ATCC No. 44076 or PEP4-1 [Jones, Genetics, 85:23 (1977)]. The presence of the *trp*1 lesion in the yeast host cell genome then provides an effective environment for detecting transformation by growth in the absence of tryptophan. Similarly, *Leu*2-deficient yeast strains (ATCC 20,622 or 38,626) are complemented by known plasmids bearing the Leu2 gene.

In addition, vectors derived from the 1.6 µm circular plasmid pKD1 can be used for transformation of *Kluyveromyces* yeasts [Bianchi et al., Curr. Genet., 12:185 (1987)]. More recently, an expression system for large-scale production of recombinant calf chymosin was reported for *K. lactis* [Van den Berg, Bio/Technology, 8:135 (1990)]. Stable multi-copy expression vectors for secretion of mature recombinant human serum albumin by industrial strains of *Kluyveromyces* have also been disclosed [Fleer et al., Bio/Technology, 9:968-975 (1991)].

### (iv) Promoter Component

- Expression and cloning vectors usually contain a promoter that is recognized by the host organism and is operably linked to the Apo-2DcR nucleic acid sequence. Promoters are untranslated sequences located upstream (5') to the start codon of a structural gene (generally within about 100 to 1000 bp) that control the transcription and translation of particularnucleic acid sequence, such as the Apo-2DcR nucleic acid sequence, to which they are operably linked. Such promoters typically fall into two classes, inducible and constitutive. Inducible promoters are promoters that initiate increased levels of transcription from DNA under their control in response to some change in culture conditions, *e.g*., the presence or absence of a nutrient or a change in temperature. At this time a large number of promoters recognized by a variety of potential host cells are well known. These promoters are operably linked to Apo-2DcR encoding DNA by removing the promoter from the source DNA by restriction enzyme digestion and inserting the isolated promoter sequence into the vector. Both the native Apo-2DcR promoter sequence and many heterologous promoters may be used to direct amplification and/or expression of the Apo-2DcR DNA.

Promoters suitable for use with prokaryotic hosts include the β-lactamase and lactose promoter systems [Chang et al., Nature, 275:617 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. However, other known bacterial promoters are suitable. Their nucleotide sequences have been published, thereby enabling a skilled worker operably to ligate them to DNA encoding Apo-2DcR [Siebenlist et al., Cell, 20:269 (1980)] using linkers or adaptors to supply any required restriction sites. Promoters for use in bacterial systems also will contain a Shine-Dalgarno(S.D.) sequence operably linked to the DNA encodingApo-2DcR.

Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CXCAAT region where X may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase2, isocytochromeC, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657. Yeast enhancers also are advantageously used with yeast promoters.

Apo-2DcR transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and most preferably Simian Virus 40 (SV40), from heterologous mammalianpromoters, *e.g*., the actin promoter or an immunoglobul in promoter, from heat-shock promoters, and from the promoternormally associated with the Apo-2DcR sequence, provided such promoters are compatible with the host cell systems.

The early and late promoters of the SV40 virus are conveniently obtained as an SV40 restriction fragment that also contains the SV40 viral origin of replication [Fiers et al., Nature, 273:113 (1978); Mulligan and Berg, Science, 209:1422-1427(1980); Pavlakiset al., Proc. Natl. Acad. Sci. USA, 78:7398-7402 (1981)]. The immediate early promoter of the human cytomegalovirus is conveniently obtained as a HindIII E restriction fragment [Greenaway et al., Gene, 18:355-360 (1982)]. A system for expressing DNA in mammalian hosts using the bovine papilloma virus as a vector is disclosed in U.S. Patent No. 4,419,446. A modification of this system is described in U.S. Patent No. 4,601,978 [See also Gray et al., Nature, 295:503-508 (1982) on expressing cDNA encoding immune interferon in monkey cells; Reyes et al., Nature, 297:598-601 (1982) on expression of human β-interferon cDNA in mouse cells under the control of a thymidine kinase promoter from herpes simplex virus; Canaani and Berg, Proc. Natl. Acad. Sci. USA 79:5166-5170 (1982) on expression of the human interferon β1 gene in cultured mouse and rabbit cells; and Gorman et al., Proc. Natl. Acad. Sci. USA, 79:6777-6781 (1982) on expression of bacterial CAT sequences in CV-1 monkey kidney cells, chicken embryo fibroblasts, Chinese hamster ovary cells, HeLa cells, and mouse NIH-3T3 cells using the Rous sarcoma virus long terminal repeat as a promoter].

### (v) Enhancer Element Component

Transcription of a DNA encoding the Apo-2DcR of this invention by higher eukaryotes may be increased by inserting an enhancer sequence into the vector. Enhancers are cis-acting elements of DNA. usually about from 10 to 300 bp, that act on a promoter to increase its transcription. Enhancers are relatively orientation and position independent, having been found 5' [Laimins et al., Proc. Natl. Acad. Sci. USA, 78:464 (1981]) and 3' [Lusky et al., Mol. Cell Bio., 3:1108(1983]) to the transcription unit, within an intron [Banerji et al., Cell, 33:729 (1983)], as well as within the coding sequence itself [Osborne et al., Mol. Cell Bio., 4:1293 (1984)]. Many enhancer sequences are now known from mammalian genes (globin, elastase, albumin. α-fetoprotein, and insulin). Typically,however,one will use an enhancer from a eukaryotic cell virus. Examples include the SV40 enhancer on the late side of the replication origin (bp 100-270), the cytomegalovirusearly promoter enhancer, the polyoma enhancer on the late side of the replication origin, and adenovirus enhancers. See also Yaniv, Nature, 297:17-18 (1982) on enhancing elements for activation of eukaryotic promoters. The enhancer may be spliced into the vector at a position 5' or 3' to the Apo-2DcR coding sequence, but is preferably located at a site 5' from the promoter.

### (vi) Transcription Termination Component

Expression vectors used in eukaryotic host cells (yeast, fungi, insect, plant, animal, human, or nucleated cells from other multicellular organisms) will also contain sequences necessary for the terminationoftranscriptionand for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding Apo-2DcR.

### (vii) Construction and Analysis of Vectors

Construction of suitable vectors containing one or more of the above-listed components employs standard ligation techniques. Isolated plasmids or DNA fragments are cleaved, tailored, and re-ligated in the form desired to generate the plasmids required.

For analysis to confirm correct sequences in plasmids constructed, the ligation mixtures can be used to transform *E. coli* K12 strain 294 (ATCC 31,446) and successful transformants selected by ampicillin or tetracycline resistance where appropriate. Plasmids from the transformants are prepared, analyzed by restriction endonuclease digestion, and/or sequenced by the method of Messing et al., Nucleic Acids Res., 9:309 (1981) or by the method of Maxam et al., Methods in Enzymology, 65:499 (1980).

### (viii) Transient Expression Vectors

Expression vectors that provide for the transient expression in mammalian cells of DNA encoding Apo-2DcR may be employed. In general, transient expression involves the use of an expression vector that is able to replicate efficiently in a host cell, such that the host cell accumulates many copies of the expression vector and, in turn, synthesizes high levels of a desired polypeptide encoded by the expression vector [Sambrook et al., *supra*]*.* Transient expression systems, comprising a suitable expression vector and a host cell, allow for the convenient positive identification of polypeptides encoded by cloned DNAs, as well as for the rapid screening of such polypeptides for desired biological or physiological properties. Thus, transient expression systems are particularly useful in the invention for purposes of identifying Apo-2DcR variants.

### (ix) Suitable Exemplary Vertebrate Cell Vectors

Other methods, vectors, and host cells suitable for adaptation to the synthesis of Apo-2DcR in recombinant vertebrate cell culture are described in Gething et al., Nature, 293:620-625 (1981); Mantei et al., Nature, 281:40-46 (1979); EP 117,060; and EP 117,058.

### 3. Selection and Transformation of Host Cells

Suitable host cells for cloning or expressing the DNA in the vectors herein are the prokaryote, yeast, or higher eukaryote cells described above. Suitable prokaryotes for this purpose include but are not limited to eubacteria, such as Gram-negative or Gram-positive organisms, for example, Enterobacteriaceaesuch as *Escherichia, e.g., E. coli, Enterobacter, Erwinia, Klebsiella. Proteus, Salmonella, e.g., Salmonella typhimurium, Serratia, e.g., Serratia marcescans,* and *Shigella,* as well as *Bacilli* such as *B. subtilis* and *B. licheniformis (e.g., B. licheniformis* 41P disclosed in DD 266,710 published 12 April 1989), *Pseudomonas* such as *P. aeruginosa,* and *Streptomyces.* Preferably, the host cell should secrete minimal amounts of proteolytic enzymes.

In addition to prokaryotes, eukaryotic microbes such as filamentous fungi or yeast are suitable cloning or expression hosts for Apo-2DcR-encoding vectors. *Saccharomyces cerevisiae,* or common baker's yeast, is the most commonly used among lower eukaryotic host microorganisms. However, a number of other genera, species, and strains are commonly available and useful herein.

Suitable host cells for the expression of glycosylated Apo-2DcR are derived from multicellular organisms. Such host cells are capable of complex processing and glycosylation activities. In principle, any higher eukaryotic cell culture is workable, whether from vertebrate or invertebrate culture. Examples of invertebrate cells include plant and insect cells. Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila melanogaster* (fruitfly), and *Bombyx mori* have been identified [See, *e.g*., Luckow et al., Bio/Technology, 6:47-55 (1988); Miller et al., in Genetic Engineering, Setlow et al., eds., Vol. 8 (Plenum Publishing, 1986), pp. 277-279; and Maeda et al., Nature, 315:592-594 (1985)]. A variety of viral strains for transfection are publicly available, *e.g.,* the L-1 variant of *Autographa californica* NPV and the Bm-5 strain of *Bombyx mori* NPV.

Plant cell cultures of cotton, corn, potato, soybean, petunia, tomato, and tobacco can be utilized as hosts. Typically, plant cells are transfected by incubation with certain strains of the bacterium *Agrobacterium tumefaciens.* During incubation of the plant cell culture with *A. tumefaciens*, the DNA encoding the Apo-2DcR can be transferred to the plant cell host such that it is transfected, and will, under appropriate conditions, express the Apo-2DcR-encoding DNA. In addition, regulatory and signal sequences compatible with plant cells are available, such as the nopaline synthase promoter and polyadenylation signal sequences [Depicker et al., J. Mol. Appl. Gen., 1:561 (1982)]. In addition. DNA segments isolated from the upstream region of the T-DNA *780* gene are capable of activating or increasing transcription levels of plant-expressible genes in recombinant DNA-containing plant tissue [EP 321,196 published 21 June 1989].

Propagation of vertebrate cells in culture (tissue culture) is also well known in the art [See, *e.g*., Tissue Culture, Academic Press. Kruse and Patterson, editors (1973)]. Examples of useful mammalian host cell lines are monkey kidney CV 1 line transformed by SV40 (COS-7, ATCC CRL 1651); human embryonic kidney line (293 or 293 cells subcloned for growth in suspension culture, Graham et al., J. Gen Virol., 36:59 (1977)): baby hamster kidney cells (BHK. ATCC CCL 10); Chinese hamster ovary cells/-DHFR(CHO, Urlaub and Chasin, Proc. Natl. Acad Sci. USA, 77:4216 (1980)); mouse sertoli cells (TM4, Mather. Biol. Reprod., 23:243-251 (1980)); monkey kidney cells (CV1 ATCC CCL 70); African green monkey kidney cells (VERO-76, ATCC CRL-1587); human cervical carcinomacells (HELA, ATCC CCL 2); canine kidney cells (MDCK, ATCC CCL 34): buffalo rat liver cells (BRL 3A. ATCC CRL 1442); human lung cells (W138, ATCC CCL 75); human liver cells (Hep G2. HB 8065); mouse mammary tumor (MMT 060562, ATCC CCL51); TRI cells (Mather et al., Annals N.Y. Acad. Sci., 383:44-68 (1982)); MRC 5 cells; and FS4 cells.

Host cells are transfected and preferably transformed with the above-describedexpressionor cloning vectors for Apo-2DcR production and cultured in conventional nutrient media modified as appropriate for inducing promoters, selecting transformants, or amplifying the genes encoding the desired sequences.

Transfection refers to the taking up of an expression vector by a host cell whether or not any coding sequences are in fact expressed. Numerous methods of transfection are known to the ordinarily skilled artisan, for example, CaPO4 and electroporation. Successful transfection is generally recognized when any indication of the operation of this vector occurs within the host cell.

Transformation means introducing DNA into an organism so that the DNA is replicable, either as an extrachromosomal element or by chromosomal integrant. Depending on the host cell used, transformation is done using standard techniques appropriate to such cells. The calcium treatment employing calcium chloride, as described in Sambrook et al., supra, or electroporation is generally used for prokaryotes or other cells that contain substantial cell-wall barriers. Infection with *Agrobacterium tumefaciens* is used for transformation of certain plant cells, as described by Shaw et al., Gene, 23:315 (1983) and WO 89/05859 published 29 June 1989. In addition, plants may be transfected using ultrasound treatment as described in WO 91/00358 published 10 January 1991.

For mammalian cells without such cell walls, the calcium phosphate precipitation method of Graham and van der Eb, Virology, 52:456-467 (1973) is preferred. General aspects of mammalian cell host system transformations have been described in U.S. Pat. No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, *e.g*., polybrene, polyornithine, may also be used. For various techniques for transforming mammalian cells, see Keown et al., Methods in Enzymology, 185:527-537 (1990) and Mansour et al., Nature, 336:348-352 (1988).

### 4. Culturing the Host Cells

Prokaryotic cells used to produce Apo-2DcR may be cultured in suitable media as described generally in Sambrook et al., supra.

The mammalian host cells used to produce Apo-2DcR may be cultured in a variety of media. Examples of commercially available media include Ham's F10 (Sigma), Minimal Essential Medium ("MEM", Sigma), RPMI-1640 (Sigma), and Dulbecco's Modified Eagle's Medium ("DMEM", Sigma). Any such media may be supplemented as necessary with hormones and/or other growth factors (such as insulin, transferrin, or epidermal growth factor), salts (such as sodium chloride, calcium, magnesium, and phosphate), buffers (such as HEPES), nucleosides(such as adenosine and thymidine), antibiotics (such as Gentamycin^{™} drug), trace elements (defined as inorganic compounds usually present at final concentrations in the micromolar range), and glucose or an equivalent energy source. Any other necessary supplements may also be included at appropriate concentrationsthat would be known to those skilled in the an. The culture conditions, such as temperature, pH, and the like, are those previously used with the host cell selected for expression, and will be apparent to the ordinarily skilled artisan.

In general, principles, protocols, and practical techniques for maximizing the productivity of mammalian cell cultures can be found in Mammalian Cell Biotechnology: a Practical Approach, M. Butler, ed. (IRL Press, 1991).

The host cells referred to in this disclosure encompass cells in culture as well as cells that are within a host animal.

### 5. Detecting Gene Amplification/Expression

Gene amplificationand/or expression may be measured in a sample directly, for example, by conventional Southern blotting, Northern blotting to quantitate the transcription of mRNA [Thomas, Proc. Natl. Acad. Sci. USA, 77:5201-5205 (1980)], dot blotting (DNA analysis), or *in situ* hybridization, using an appropriately labeled probe, based on the sequences provided herein. Various labels may be employed, most commonly radioisotopes, and particularly ³²P. However, other techniques may also be employed, such as using biotin-modifiednucleotides for introduction into a polynucleotide. The biotin then serves as the site for binding to avidin or antibodies, which may be labeled with a wide variety of labels, such as radionucleotides, fluorescers or enzymes. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemicalstaining of cells or tissue sections and assay of cell culture or body fluids, to quantitate directly the expression of gene product. With immunohistochemical staining techniques, a cell sample is prepared, typically by dehydration and fixation, followed by reaction with labeled antibodies specific for the gene product coupled, where the labels are usually visually detectable, such as enzymatic labels, fluorescent labels, or luminescent labels.

Antibodies useful for immunohistochemical staining and/or assay of sample fluids may be either monoclonal or polyclonal, and may be prepared in any mammal. Conveniently, the antibodies may be prepared against a native sequence Apo-2DcR polypeptide or against a synthetic peptide based on the DNA sequences provided herein or against exogenous sequence fused to Apo-2DcR DNA and encoding a specific antibody epitope.

### 6. Purification of Apo-2DcR Polypeptide

Forms of Apo-2DcR may be recovered from culture medium or from host cell lysates. If the Apo-2DcR is membrane-bound, it can be released from the membrane using a suitable detergent solution (*e.g*. Triton-X 100) or its extracellular domain may be released by enzymatic cleavage. Apo-2DcR can also be released from the cell-surface by enzymatic cleavage of its glycophospholipid membrane anchor.

When Apo-2DcR is produced in a recombinant cell other than one of human origin, the Apo-2DcR is free of proteins or polypeptides of human origin. However, it may be desired to purify Apo-2DcR from recombinant cell proteins or polypeptides to obtain preparationsthat are substantiallyhomogeneous as to Apo-2DcR. As a first step, the culture medium or lysate may be centrifugedto remove particulate cell debris. Apo-2DcR thereafter is purified from contaminant soluble proteins and polypeptides, with the following procedures being exemplary of suitable purification procedures: by fractionation on an ion-exchange column; ethanol precipitation; reverse phase HPLC; chromatography on silica or on a cation-exchange resin such as DEAE; chromatofocusing;SDS-PAGE; ammonium sulfate precipitation; gel filtration using, for example, Sephadex G-75; and protein A Sepharose columns to remove contaminants such as IgG.

Apo-2DcR variants in which residues have been deleted, inserted, or substituted can be recovered in the same fashion as native sequence Apo-2DcR, taking account of changes in properties occasioned by the variation. For example, preparation of an Apo-2DcR fusion with another protein or polypeptide, *e.g*., a bacterial or viral antigen, immunoglobulin sequence, or receptor sequence, may facilitate purification; an immunoaffinity column containing antibody to the sequence can be used to adsorb the fusion polypeptide. Other types of affinity matrices also can be used.

A protease inhibitor such as phenyl methyl sulfonyl fluoride (PMSF) also may be useful to inhibit proteolytic degradation during purification, and antibiotics may be included to prevent the growth of adventitiouscontaminants. One skilled in the art will appreciate that purification method suitable for native sequence Apo-2DcR may require modification to account for changes in the character of Apo-2DcR or its variants upon expression in recombinant cell culture.

### 7. Covalent Modifications of Apo-2DcR Polypeptides

Covalent modifications of Apo-2DcR are included within the scope of this invention. One type of covalent modification of the Apo-2DcR is introduced into the molecule by reacting targeted amino acid residues of the Apo-2DcR with an organic derivatizing agent that is capable of reacting with selected side chains or the N- or C- terminal residues of the Apo-2DcR.

Derivatization with bifunctional agents is useful for crosslinking Apo-2DcR to a water-insoluble support matrix or surface for use in the method for purifying anti-Apo-2DcR antibodies, and vice-versa. Derivatizationwith one or more bifunctional agents will also be useful for crosslinking Apo-2DcR molecules to generate Apo-2Dc Rdimers. Such dimers may increase binding avidity and extend half-life of the molecule *in vivo.* Commonly used crosslinking agents include, *e.g.*, 1,1-bis(diazoacetyl)-2-phenylethane, glutaraldehyde, N-hydroxysuccinimideesters, for example, esters with 4-azidosalicylicacid, homobifunctional imidoesters, including disuccinimidylesters such as 3,3'-dithiobis(succinimidylpropionate), and bifunctional maleimides such as bis-N-maleimido-1,8-octane. Derivatizing agents such as methyl-3-[(p-azidophenyl)-dithio]propioimidate yield photoactivatable intermediates that are capable of forming crosslinks in the presence of light. Alternatively, reactive water-insoluble matrices such as cyanogen bromide-activated carbohydrates and the reactive substrates described in U.S. Patent Nos. 3,969,287; 3,691,016; 4,195,128; 4,247,642; 4,229,537; and 4,330,440 are employed for protein immobilization.

Other modifications include deamidation of glutaminyl and asparaginyl residues to the corresponding glutamyl and aspartyl residues, respectively, hydroxylationof proline and lysine, phosphorylationof hydroxyl groups of seryl or threonyl residues, methylation of the α-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group. The modified forms of the residues fall within the scope of the present invention.

Another type of covalent modification of the Apo-2DcR polypeptide included within the scope of this invention comprises altering the native glycosylation pattern of the polypeptide. "Altering the native glycosylation pattern" is intended for purposes herein to mean deleting one or more carbohydrate moieties found in native sequence Apo-2DcR. and/or adding one or more glycosylation sites that are not present in the native sequence Apo-2DcR.

Glycosylation of polypeptides is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxylamino acid, most commonly serine or threonine, although 5-hydroxyproline or 5-hydroxylysine may also be used.

Addition of glycosylation sites to the Apo-2DcR polypeptide may be accomplished by altering the amino acid sequence such that it contains one or more of the above-described tripeptide sequences (for N-linked glycosylationsites). The alteration may also be made by the addition of, or substitution by, one or more serine or threonine residues to the native sequence Apo-2DcR (for O-linked glycosylation sites). The Apo-2DcR amino acid sequence may optionally be altered through changes at the DNA level, particularly by mutating the DNA encoding the Apo-2DcR polypeptide at preselected bases such that codons are generated that will translate into the desired amino acids. The DNA mutation(s) may be made using methods described above and in U.S. Pat. No. 5,364,934, supra.

Another means of increasing the number of carbohydrate moieties on the Apo-2DcR polypeptide is by chemical or enzymatic coupling of glycosides to the polypeptide. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine,tyrosine, or tryptophan, or (f) the amide group of glutamine. These methods are described in WO 87/05330 published 11 September 1987, and in Aplin and Wriston, CRC Crit, Rev. Biochem., pp. 259-306 (1981).

Removal of carbohydrate moieties present on the Apo-2DcR polypeptide may be accomplished chemically or enzymatically or by mutational substitution of codons encoding for amino acid residues that serve as targets for glycosylation. For instance, chemical deglycosylation by exposing the polypeptide to the compound trifluoromethanesulfonicacid, or an equivalent compound can result in the cleavage of most or all sugars except the linking sugar (N-acetylglucosamine or N-acetylgalactosamine), while leaving the polypeptide intact. Chemical deglycosylation is described by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Glycosylat ion at potential glycosylation sites may be prevented by the use of the compound tunicamycin as described by Duksin et al., J. Biol. Chem., 257:3105 (1982). Tunicamycin blocks the formation of protein-N-glycoside linkages.

Another type of covalent modification of Apo-2DcR comprises linking the Apo-2DcR polypeptide to one of a variety of nonproteinaceous polymers, *e.g*., polyethylene glycol, polypropylene glycol, or polyoxyalkylenes, in the manner set forth in U.S. Patent Nos. 4,640,835; 4,496,689; 4,301.144; 4,670,417; 4,791,192 or 4,479,337.

### 8. Apo-2DcR Chimeras

The present invention also provides chimeric molecules comprising Apo-2DcR fused to another, heterologous polypeptide or amino acid sequence.

In one embodiment, the chimeric molecule comprises a fusion of the Apo-2DcR with a tag polypeptide which provides an epitope to which an anti-tag antibody can selectively bind. The epitope tag is generally placed at the am ino- or carboxyl- terminus of the Apo-2DcR. The presence of such epitope-tagged forms of the Apo-2DcR can be detected using an antibody against the tag polypeptide. Also, provision of the epitope tag enables the Apo-2DcR to be readily purified by affinity purification using an anti-tag antibody or another type of affinity matrix that binds to the epitope tag.

Various tag polypeptides and their respective antibodies are well known in the art. Examples include the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an α-tubulin epitope peptide [Skinner et al., J. Biol, Chem., 266:14163-14166 (1991)]; and the T7 gene 10 protein peptide tag [Lutz-Freyermuth et al., Proc. Natl. Acad. Sci. USA, 87:6393-6397 (1990)]. Once the tag polypeptide has been selected, an antibody thereto can be generated using the techniques disclosed herein.

Generally, epitope-tagged Apo-2DcR may be constructed and produced according to the methods described above. Apo-2DcR-tag polypeptide fusions are preferably constructed by fusing the cDNA sequence encoding the Apo-2DcRportion in-frame to the tag polypeptide DNA sequence and expressing the resultant DNA fusion construct in appropriate host cells. Ordinarily, when preparing the Apo-2DcR-tag polypeptide chimeras of the present invention, nucleic acid encoding the Apo-2DcR will be fused at its 3' end to nucleic acid encoding the N-terminus of the tag polypeptide, however 5' fusions are also possible. For example, a polyhistidi ne sequence of about 5 to about 10 histidine residues may be fused at the N- terminus or the C-terminus and used as a purification handle in affinity chromatography.

Epitope-taggedApo-2DcR can be purified by affinity chromatography using the anti-tag antibody. The matrix to which the affinity antibody is attached may include, for instance, agarose, controlled pore glass or poly(styrenedivinyl)benzene. The epitope-tagged Apo-2DcR can then be eluted from the affinity column using techniques known in the art.

In another embodiment, the chimeric molecule comprises an Apo-2DcR polypeptide fused to an immunoglobulin sequence. The chimeric molecule may also comprise a particular domain sequence of Apo-2DcR, such as an extracellular domain sequence of Apo-2DcR fused to an immunoglobulin sequence. This includes chimeras in monomeric, homo- or heteromultimeric, and particularly homo- or heterodimeric, or - tetrameric forms; optionally, the chimeras may be in dimeric forms or homodimeric heavy chain forms. Generally, these assembled immunoglobulins will have known unit structures as represented by the following diagrams.

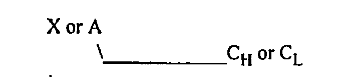

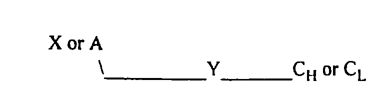

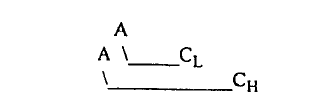

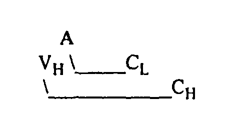

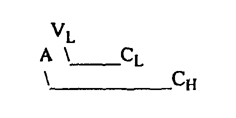

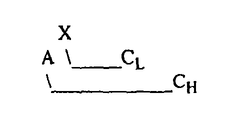

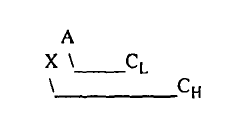

A basic four chain structural unit is the form in which IgG, IgD, and IgE exist. A four chain unit is repeated in the higher molecular weight immunoglobulins; IgM generally exists as a pentamer of basic four-chain units held together by disulfide bonds. IgA globulin, and occasionally IgG globulin, may also exist in a multimeric form in serum. In the case of multimers, each four chain unit may be the same or different.

The following diagrams depict some exemplary monomer, homo- and heterodimer and homo- and heteromultimer structures. These diagrams are merely illustrative,and the chains of the multimersare believed to be disulfide bonded in the same fashion as native immunoglobulins.
monomer: 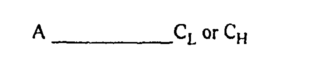 homodimer: 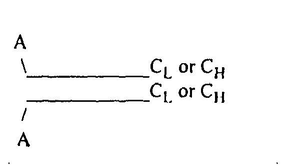 heterodimer: 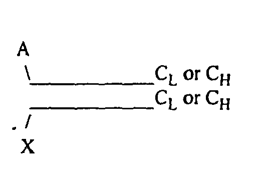 homotetramer: 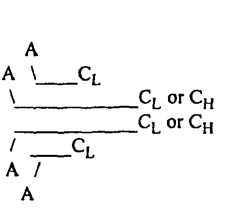 heterotetramer: 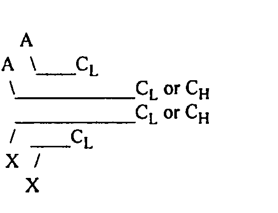 and

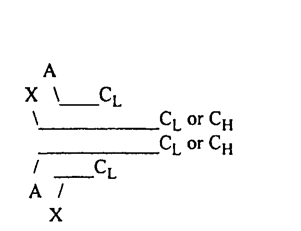

In the foregoing diagrams, "A" means an Apo-2DcR sequence or an Apo-2DcR sequence fused to a heterologous sequence: X is an additional agent, which may be the same as A or different, a portion of an immunoglobulin superfamily member such as a variable region or a variable region-like domain, including a native or chimeric immunoglobulin variable region, a toxin such a pseudomonas exotoxin or ricin, or a sequence functionally binding to another protein, such as other cytokines (i.e., IL-1, interferon-γ) or cell surface molecules (i.e., NGFR, CD40, OX40. Fas antigen, T2 proteins of Shope and myxoma poxviruses), or a polypeptide therapeutic agent not otherwise normally associated with a constant domain; Y is a linker or another receptor sequence: and V_{L}, V_{H}, C_{L} and C_{H} represent light or heavy chain variable or constant domains of an immunoglobulin. Structures comprising at least one CRD of an Apo-2DcR sequence as "A" and another cell-surface protein having a repetitive pattern of CRDs (such as TNFR) as "X" are specifically included.

It will be understood that the above diagrams are merely exemplary of the possible structures of the chimeras of the present invention, and do not encompass all possibilities. For example, there might desirably be several different "A"s, "X"s, or "Y"s in any of these constructs. Also, the heavy or light chain constant domains may be originated from the same or different immunoglobulins. All possible permutations of the illustrated and similar structures are all within the scope of the invention herein.

In general, the chimeric molecules can be constructed in a fashion similar to chimeric antibodies in which a variable domain from an antibody of one species is substituted for the variable domain of another species. See, for example, EP 0 125 023; EP 173,494; Munro, Nature, 312:597 (13 December 1984); Neuberger et al., Nature, 312:604-608 (13 December 1984); Sharon et al., Nature, 309:364-367 (24 May 1984); Morrison et al., Proc. Nat'l. Acad. Sci. USA, 81:6851-6855 (1984); Morrison et al., Science, 229:1202-1207 (1985); Boulianne et al., Nature, 312:643-646 (13 December 1984); Capon et al., Nature, 337:525-531 (1989); Traunecker et al., Nature, 339:68-70 (1989).

Alternatively,the chimeric molecules may be constructed as follows. The DNA including a region encoding the desired sequence, such as an Apo-2DcR and/or TNFR sequence, is cleaved by a restriction enzyme at or proximal to the 3' end of the DNA encoding the immunoglobulin-like domain(s) and at a point at or near the DNA encoding the N-terminal end of the Apo-2DcR or TNFR polypeptide (where use of a different leader is contemplated)or at or proximal to the N-terminal coding region for TNFR (where the native signal is employed). This DNA fragment then is readily inserted proximal to DNA encoding an immunoglobulin light or heavy chain constant region and, if necessary, the resulting construct tailored by deletional mutagenesis. Preferably, the 1g is a human immunoglobulinwhen the chimeric molecule is intended for *in vivo* therapy for humans. DNA encoding immunoglobulin light or heavy chain constant regions is known or readily available from cDNA libraries or is synthesized. See for example, Adams et al., Biochemistry, 19:2711-2719 (1980); Gough et al., Biochemistry, 19:2702-2710 (1980); Dolby et al., Proc. Natl. Acad. Sci.. USA, 77:6027-6031 (1980); Rice et al., Proc. Natl. Acad. Sci., 79:7862-7865(1982); Falkner et al., Nature, 298:286-288 (1982); and Morrison et al., Ann. Rev. Immunol., 2:239-256 (1984).

Further details of how to prepare such fusions are found in publications concerning the preparation of immunoadhesins. Immunoadhesins in general, and CD4-Ig fusion molecules specifically are disclosed in WO 89/02922, published 6 April 1989). Molecules comprising the extracellular portion of CD4, the receptor for human immunodeficiencyvirus (HIV), linked to IgG heavy chain constantregion are known in the art and have been found to have a markedly longer half life and lower clearance than the soluble extracellular portion of CD4 [Capon et al., supra; Byrn et al., Nature, 344:667 (1990)]. The construction of specific chimeric TNFR-IgG molecules is also described in Ashkenazi et al. Proc. Natl. Acad. Sci., 88:10535-10539 (1991); Lesslauer et al. [J. Cell. Biochem. Supplement 15F, 1991, p. 115 (P 432)]; and Peppel and Beutler, J, Cell. Biochem. Supplement 15F. 1991, p. 118 (P 439)].

### B. Therapeutic and Non-therapeutic Uses for Apo-2DcR

Apo-2DcR. as disclosed in the present specification, can be employed therapeutically to regulate apoptosis and/or NF-κB activation by Apo-2L or by another ligand that Apo-2DcR binds to in mammalian cells. This therapy can be accomplished for instance, using *in vivo* or *ex vivo* gene therapy techniques and includes the use of the death domain sequences disclosed herein. The Apo-2DcR chimeric molecules (including the chimeric molecules containing an extracellular domain sequence of Apo-2DcR or the Apo-2DcR immunoadhesin described in the Examples below) comprising immunoglobulin sequences can also be employed therapeutically to inhibit Apo-2L activities, for example, apoptosis or NF-κB induction or the activity of another ligand that Apo-2DcR binds to.

Suitable carriers and their formulations are described in Remington's Pharmaceutical Sciences, 16th ed., 1980, Mack Publishing Co., edited by Oslo et al. Typically, an appropriate amount of a pharmaceutically-acceptable salt is used in the formulation to render the formulation isotonic. Examples of the carrier include buffers such as saline, Ringer's solution and dextrose solution. The pH of the solution is preferably from about 5 to about 8, and more preferably from about 7.4 to about 7.8. It will be apparent to those persons skilled in the art that certain carriers may be more preferable depending upon, for instance, the route of administration.

Administration to a mammal may be accomplished by injection (*e.g*., intravenous, intraperitoneal, subcutaneous, intramuscular), or by other methods such as infusion that ensure delivery to the bloodstream in an effective form.

Effective dosages and schedules for administration may be determined empirically, and making such determinations is within the skill in the art.

It is contemplated that other, additional therapies may be administered to the mammal, and such includes but is not limited to, chemotherapy and radiation therapy, immunoadjuvants,cytokines, and antibody-based therapies. Examples include interleukins (e.g., IL-1, IL-2, IL-3, IL-6), leukemia inhibitory factor, interferons, TGF-beta, erythropoietin, thrombopoietin, and HER-2 antibody. Other agents known to induce apoptosis in mammalian cells may also employed, and such agents include TNF-α, TNF-β(lymphotoxin-α), CD30 ligand, 4-1BB ligand, and Apo-1 ligand.

Chemotherapies contemplated by the invention include chemical substances or drugs which are known in the art and are commercially available, such as Doxorubicin, 5-Fluorouracil, Cytosine arabinoside ("Ara-C"), Cyclophosphamide, Thiotepa, Busulfan, Cytoxin, Taxol, Methotrexate, Cisplatin, Melphalan, Vinblastine and Carboplatin. Preparation and dosing schedules for such chemotherapymay be used according to manufacturers'instructionsor as determined empirically by the skilled practitioner. Preparation and dosing schedules for such chemotherapy are also described in Chemotherapy Service Ed., M.C. Perry, Williams & Wilkins, Baltimore, MD (1992). The chemotherapy is preferably administered in a pharmaceutically-acceptable carrier, such as those described above.

The Apo-2DcR of the invention also has utility in non-therapeutic applications. Nucleic acid sequences encoding the Apo-2DcR may be used as a diagnostic for tissue-specific typing. For example, procedures like *in situ* hybridization, Northern and Southern blotting, and PCR analysis may be used to determine whether DNA and/or RNA encoding Apo-2DcR is present in the cell type(s) being evaluated. Apo-2DcR nucleic acid will also be useful for the preparation of Apo-2DcR by the recombinant techniques described herein.

The isolated Apo-2DcR may be used in quantitative diagnostic assays as a control against which samples containing unknown quantities of Apo-2DcR may be prepared. Apo-2DcR preparations are also useful in generating antibodies, as standards in assays for Apo-2DcR (e.g., by labeling Apo-2DcR for use as a standard in a radioimmunoassay, radioreceptor assay, or enzyme-linked immunoassay), in affinity purification techniques, and in competitive-type receptor binding assays when labeled with, for instance, radioiodine, enzymes, or fluorophores.

Isolated, native forms of Apo-2DcR, such as described in the Examples, may be employed to identify alternate forms of Apo-2DcR; for example, forms that possess cytoplasmic domain(s) which may be involved in signaling pathway(s). Modified forms of the Apo-2DcR, such as the Apo-2DcR-1gG chimeric molecules (immunoadhesins) described above, can be used as immunogens in producing anti-Apo-2DcR antibodies.

Nucleic acids which encode Apo-2DcR or its modified forms can also be used to generate either transgenic animals or "knock out" animals which, in turn, are useful in the development and screening of therapeutically useful reagents. A transgenic animal (e.g., a mouse or rat) is an animal having cells that contain a transgene, which transgene was introduced into the animal or an ancestor of the animal at a prenatal, e.g., an embryonic stage. A transgene is a DNA which is integrated into the genome of a cell from which a transgenic animal develops. In one embodiment, cDNA encoding Apo-2DcR or an appropriate sequence thereof(such as Apo-2DcR-IgG)can be used to clone genomic DNA encoding Apo-2DcR in accordance with established techniques and the genomic sequences used to generate transgenic animals that contain cells which express DNA encoding Apo-2DcR. Methods for generating transgenic animals, particularly animals such as mice or rats, have become conventional in the art and are described, for example, in U.S. Patent Nos. 4,736,866 and 4,870,009. Typically, particular cells would be targeted for Apo-2DcR transgene incorporation with tissue-specific enhancers. Transgenic animals that include a copy of a transgene encoding Apo-2DcR introduced into the germ line of the animal at an embryonic stage can be used to examine the effect of increased expression of DNA encoding Apo-2DcR. Such animals can be used as tester animals for reagents thought to confer protection from, for example, pathological conditions associated with excessive apoptosis. In accordance with this facet of the invention, an animal is treated with the reagent and a reduced incidence of the pathological condition, compared to untreated animals bearing the transgene, would indicate a potential therapeutic intervention for the pathological condition. In another embodiment, transgenic animals that carry a soluble form of Apo-2DcR such as the Apo-2DcR ECD or an immunoglobulin chimera of such form could be constructed to test the effect of chronic neutralization of Apo-2L, a ligand of Apo-2DcR.

Alternatively,non-human homologues of Apo-2DcR can be used to construct an Apo-2DcR "knock out" animal which has a defective or altered gene encoding Apo-2DcR as a result of homologous recombination between the endogenous gene encoding Apo-2DcR and altered genomic DNA encoding Apo-2DcR introduced into an embryonic cell of the animal. For example, cDNA encoding Apo-2DcR can be used to clone genomic DNA encoding Apo-2DcR in accordance with established techniques. A portion of the genomic DNA encoding Apo-2DcR can be deleted or replaced with another gene, such as a gene encoding a selectable marker which can be used to monitor integration. Typically, several kilobases of unaltered flanking DNA (both at the 5' and 3' ends) are included in the vector [see e.g., Thomas and Capecchi, Cell, 51:503 (1987) for a description of homologous recombination vectors]. The vector is introduced into an embryonic stem cell line (e.g., by electroporation) and cells in which the introduced DNA has homologously recombined with the endogenous DNA are selected [see e.g., Li et al., Cell, 69:915 (1992)]. The selected cells are then injected into a blastocyst of an animal (e.g., a mouse or rat) to form aggregation chimeras [see e.g., Bradley, in Teratocarcinomas and Embryonic Stem Cells: A Practical Approach, E. J. Robertson, ed. (IRL, Oxford, 1987), pp. 113-152]. A chimeric embryo can then be implanted into a suitable pseudopregnantfemale foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their developmentof pathological conditions due to absence of the Apo-2DcR polypeptide, including for example, development of tumors.

### C. Anti-Apo-2DcR Antibody Preparation

The present invention further provides anti-Apo-2DcR antibodies. Antibodies against Apo-2DcR may be prepared as follows. Exemplary antibodies include polyclonal, monoclonal, humanized, bispecific, and heteroconjugate antibodies.

### 1. Polyclonal Antibodies

The Apo-2DcR antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the Apo-2DcR polypeptide or a fusion protein thereof. An example of a suitable immunizing agent is a Apo-2DcR-IgG fusion protein or chimeric molecule (including an Apo-2DcR ECD-IgG fusion protein). Cells expressing Apo-2DcR at their surface may also be employed. It may be useful to conjugate the immunizing agent to a protein known to be immunogenic in the mammal being immunized. Examples of such immunogenic proteins which may be employed include but are not limited to keyhole limpet hemocyanin, serum albumin, bovine thyroglobulin, and soybean trypsin inhibitor. An aggregating agent such as alum may also be employed to enhance the mammal's immune response. Examples of adjuvants which may be employed include Freund's complete adjuvant and MPL-TDM adjuvant (monophosphorylLipid A, synthetic trehalose dicorynomycolate). The immunization protocol may be selected by one skilled in the art without undue experimentation. The mammal can then be bled, and the serum assayed for antibody titer. If desired, the mammal can be boosted until the antibody titer increases or plateaus.

### 2. Monoclonal Antibodies

The Apo-2DcR antibodies may, alternatively, be monoclonal antibodies. Monoclonal antibodies may be prepared using hybridoma methods, such as those described by Kohler and Milstein, supra. In a hybridoma method, a mouse, hamster, or other appropriate host animal, is typically immunized (such as described above) with an immunizing agent to elicit lymphocytes that produce or are capable of producing antibodies that will specifically bind to the immunizing agent. Alternatively, the lymphocytes may be immunized *in vitro.*

The immunizing agent will typically include the Apo-2DcR polypeptide or a fusion protein thereof. An example of a suitable immunizing agent is a Apo-2DcR-IgG fusion protein or chimeric molecule. A specific example of an immunogen is described in Example 13 below. Cells expressing Apo-2DcR at their surface may also be employed. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against Apo-2DcR. Preferably, the binding specificity of monoclonal antibodies produced by the hybridoma cells is determined by immunoprecipitation or by an *in vitro* binding assay, such as radioimmunoassay(RIA) or enzyme-linked immunoabsorbentassay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinantDNA methods,such as those described in U.S. Patent No. 4,816.567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamsterovary (CHO) cells, or myeloma cells that do not otherwise produce immunoglobulinprotein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

As described in the Examples below, anti-Apo-2DcR monoclonal antibodies have been prepared. Several of these antibodies, referred to as 4G3.9.9, 6D10.9.7, and 1C5.24.1 have been deposited with ATCC and have been assigned deposit accession numbers _____, ______, and ______, respectively. In one embodiment, the monoclonal antibodies of the invention will have the same biological characteristics as one or more of the antibodies secreted by the hybridoma cell lines deposited under accession numbers ___, ____, or ____. The term "biological characteristics" is used to refer to the *in vitro* and or *in vivo* activities or properties of the monoclonal antibodies, such as the ability to bind to Apo-2DcR or to substantially block, induce, or enhance Apo-2DcR activation. Optionally, the monoclonal antibody will bind to the same epitope as at least one of the three antibodies specifically referred to above. Such epitope binding can be determined by conducting various assays, like those described herein and in the examples.

The antibodies may be monovalentantibodies. Methods for preparing monovalentantibodiesare well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art. For instance, digestion can be performed using papain. Examples of papain digestion are described in WO 94/29348 published 12/22/94 and U.S. Patent No. 4,342,566. Papain digestion of antibodies typically produces two identical antigen binding fragments, called Fab fragments, each with a single antigen binding site, and a residual Fc fragment. Pepsin treatment yields an F(ab')₂ fragment that has two antigen combining sites and is still capable of cross-linking antigen.

The Fab fragments produced in the antibody digestion also contain the constant domains of the light chain and the first constant domain (CH₁) of the heavy chain. Fab' fragments differ from Fab fragments by the addition of a few residues at the carboxy terminus of the heavy chain CH₁ domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

### 3. Humanized Antibodies

The Apo-2DcR antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Reichmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important in order to reduce antigenicity. According to the "best-fit" method, the sequence of the variable domain of a rodent antibody is screened against the entire library of known human variable domain sequences. The human sequence which is closest to that of the rodent is then accepted as the human framework (FR) for the humanized antibody [Sims et al., J. Immunol., 151:2296 (1993); Chothia and Lesk, J. Mol. Biol., 196:901 (1987)]. Another method uses a particular framework derived from the consensus sequence of all human antibodies of a particular subgroup of light or heavy chains. The same framework may be used for several different humanized antibodies [Carteret al., Proc. Natl. Acad. Sci. USA, 89:4285 (1992); Presta et al., J. Immunol., 151:2623 (1993)].

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three dimensional models of the parental and humanized sequences. Three dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the consensus and import sequence so that the desired antibody characteristic, such as increased affinity for the target antigen(s), is achieved. In general, the CDR residues are directly and most substantially involved in influencing antigen binding [see, WO 94/04679 published 3 March 1994].

Transgenic animals (e.g., mice) that are capable, upon immunization, of producing a full repertoire of human antibodies in the absence of endogenous immunoglobulin production can be employed. For example, it has been described that the homozygous deletion of the antibody heavy chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge [see, e.g., Jakobovits et al., Proc. Natl. Acad. Sci. USA. 90:2551-255 (1993); Jakobovits et al., Nature, 362:255-258 (1993); Bruggemann et al., Year in Immuno., 7:33 (1993)]. Human antibodies can also be produced in phage display libraries [Hoogenboom and Winter, J. Mol. Biol., 227:381 (1992); Marks et al., J. Mol, Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)].

### 4. Bispecific Antibodies

Bispecific antibodies are monoclonal, preferably human or humanized, antibodies that have binding specificities for at least two different antigens. In the present case, one of the binding specificities is for the Apo-2DcR, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities[Miistein and Cuello, Nature, 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

According to a different and more preferred approach, antibody variable domains with the desired binding specificities (antibody-antigen combining sites) are fused to immunoglobulin constant domain sequences. The fusion preferably is with an immunoglobulinheavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfectedinto a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance. In a preferred embodiment of this approach, the bispecific antibodies are composed of a hybrid immunoglobulin heavy chain with a first binding specificity in one arm, and a hybrid immunoglobulin heavy-chain/light-chain pair (providing a second binding specificity) in the other arm. It was found that this asymmetric structure facilitates the separation of the desired bispecific compound from unwanted immunoglobulin chain combinations,as the presenceof an immunoglobulin light chain in only one half of the bispecific molecule provides for a facile way of separation. This approach is disclosed in WO 94/04690 published 3 March 1994. For further details of generating bispecific antibodies see, for example, Suresh et al., Methods in Enzymology, 121:210 (1986).

### 5. Heteroconjugate Antibodies

- Heteroconjugate antibodies are also within the scope of the present invention. Heteroconjugate antibodies are composed of two covalently joined antibodies. Such antibodies have, for example, been proposed to target immune system cells to unwanted cells [US Patent No.4,676,980], and for treatmentofHIV infection [WO 91/00360; WO 92/20373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiotate and methyl-4-mercaptobutyrimidate and those disclosed, for example, in U.S. Pat. No. 4,676,980.

### D. Therapeutic and Non-therapeutic Uses for Apo-2DcR Antibodies

The Apo-2DcR antibodies of the invention have therapeutic utility. For example, Apo-2DcR antibodieswhich cross-react with other receptors for Apo-2 ligand may be used to block excessive apoptosis (for instance in neurodegenerative disease) or to block potential autoimmune/inflammatory effects. Optionally, Apo-2DcR blocking antibodies can be used in combination with an Apo-2 ligand treatment. Such Apo-2DcR antibodies can block the Apo-2DcR receptor, and increase bioavailability of the administered Apo-2 ligand. Therapeutic compositions and modes of administration (such as described above for Apo-2DcR) may be employed.

Apo-2DcR antibodies may further be used in immunohistochemistry staining assays or diagnostic assays for Apo-2DcR, *e.g.**.*** detecting its expression in specific cells, tissues, or serum. Various diagnostic assay techniques known in the art may be used, such as competitive binding assays, direct or indirect sandwich assays and immunoprecipitationassays conducted in either heterogeneous or homogeneous phases [Zola, Monoclonal Antibodies: A Manual of Techniques, CRC Press, Inc. (1987) pp. 147-158]. The antibodies used in the diagnostic assays can be labeled with a detectable moiety. The detectable moiety should be capable of producing, either directly or indirectly, a detectable signal. For example, the detectable moiety may be a radioisotope, such as ³H, ¹⁴C, ³²P, ³⁵S, or ¹²⁵I, a fluorescent or chemiluminescent compound, such as fluoresce in isothiocyanate, rhodamine, or luciferin, or an enzyme, such as alkaline phosphatase, beta-galactosidase or horseradish peroxidase. Any method known in the art for conjugating the antibody to the detectable moiety may be employed, including those methods described by Hunter et al.. Nature, 194:495 (1963): David et al.. Biochemistry, 13:1014 (1974); Pain et al.. J. Immunol. Meth., 40:219 (1981); and Nygren. J. Histochem, and Cytochem., 30:407 (1982).

Apo-2DcR antibodies also are useful for the affinity purification of Apo-2DcR from recombinant cell culture or natural sources. In this process, the antibodies against Apo-2DcR are immobilized on a suitable support, such a Sephadex resin or filter paper, using methods well known in the art. The immobilized antibody then is contacted with a sample containing the Apo-2DcR to be purified, and thereafter the support is washed with a suitable solvent that will remove substantially all the material in the sample except the Apo-2DcR, which is bound to the immobilized antibody. Finally, the support is washed with another suitable solvent that will release the Apo-2DcR from the antibody.

### E. Kits Containing Apo-2DcR or Apo-2DcR Antibodies

In a further embodiment of the invention, there are provided articles of manufacture and kits containing Apo-2DcR or Apo-2DcR antibodies which can be used, for instance, for the therapeutic or non-therapeutic applications described above. The article of manufacture comprises a container with a label. Suitable containers include, for example, bottles, vials, and test tubes. The containers may be formed from a variety of materials such as glass or plastic. The container holds a composition which includes an active agent that is effective for therapeutic or non-therapeutic applications, such as described above. The active agent in the composition is Apo-2DcR or an Apo-2DcR antibody. The label on the container indicates that the composition is used for a specific therapy or non-therapeutic application, and may also indicate directions for either *in vivo* or *in vitro* use, such as those described above.

The kit of the invention will typically comprise the container described above and one or more other containers comprising materials desirable from a commercial and user standpoint, including buffers, diluents, filters, needles, syringes, and package inserts with instructions for use.

The following examples are offered for illustrative purposes only, and are not intended to limit the scope of the present invention in any way.

All patent and literature references cited in the present specification are hereby incorporated by reference in their entirety.

### EXAMPLES

All restriction enzymes referred to in the examples were purchased from New England Biolabs and used accordi ng to manufacturer's instructions. All other commercially available reagents referred to in the examples were used according to manufacturer's instructions unless otherwise indicated. The source of those cells identified in the following examples, and throughout the specification, by ATCC accession numbers is the American Type Culture Collection. Mannasas. Virginia.

### EXAMPLE I

### Isolation of cDNA clones Encoding Human Apo-2DcR

### 1. Preparation of oligo dT primed cDNA library ("LIB111")

mRNA was isolated from human breast carcinoma tissue using reagents and protocols from Invitrogen, San Diego, CA (Fast Track 2). This RNA was used to generate an oligo dT primed cDNA library ("LIB111") in the vector pRK5D using reagents and protocols from Life Technologies. Gaithersburg, MD (Super Script Plasmid System). In this procedure, the double stranded cDNA was sized to greater than 1000 bp and the SalI/NotI linkered cDNA was cloned into XhoI/NotI cleaved vector. pRK5D is a cloning vector that has an sp6 transcription initiation site followed by an SfiI restriction enzyme site preceding the XhoI/NotI cDNA cloning sites.

### 2. Preparation of random primed cDNA library ("LIB118")

A secondary cDNA library was generated in order to preferentially represent the 5' ends of the primary cDNA clones. Sp6 RNA was generated from the primary library (LIB111, described above), and this RNA was used to generate a random primed cDNA library ("LIB118") in the vector pSST-AMY.0 using reagents and protocols from Life Technologies (Super Script Plasmid System, referenced above). In this procedure the double stranded cDNA was sized to 500-1000 bp, linkered with blunt to NotI adaptors, cleaved with SfiI, and cloned into SfiI/NotI cleaved vector. pSST-AMY.0 is a cloning vector that has a yeast alcohol dehydrogenase promoter preceding the cDNA cloning sites and the mouse amylase sequence (the mature sequence without the secretion signal) followed by the yeast alcohol dehydrogenase terminator, after the cloning sites. Thus, cDNAs cloned into this vector that are fused in frame with amylase sequence will lead to the secretion of amylase from appropriately transfected yeast colonies.

### 3. Transformation and Detection

DNA from LIB118 was chilled on ice to which was added electrocompetent DH10B bacteria (Life Technologies, 20 ml). The bacteria vector mixture was then electroporated as recommended by the manufacturer. Subsequently, SOC media (Life Technologies, 1 ml) was added and the mixture was incubated at 37°C for 30 minutes. The transformants were then plated onto 20 standard 150 mm LB plates containing ampicillin and incubated for 16 hours (37°C). Positive colonies were scraped off the plates and the DNA was isolated from the bacterial pellet using standard protocols, e.g. CsCI-gradient. The purified DNA was then carried on to the yeast protocols below.

The yeast methods employed in the present invention were divided into three categories: (I) Transformation of yeast with the plasmid/cDNA combined vector; (2) Detection and isolation of yeast clones secreting amylase: and (3) PCR amplification of the insert directly from the yeast colony and purification of the DNA for sequencing and further analysis.

While any yeast strain containing a stable mutant ura3 is useable with the present invention, the preferable yeast strain used with the practice of the invention was HD56-5A (ATCC-90785). This strain had the following genotype: MAT alpha, ura3-52. leu2-3, leu2-112, his3-11, his3-15, MAL⁺, SUC⁺, GAL⁺.

Transformation was performed based on the protocol outlined by Gietz et al., Nucl. Acid. Res., 20:1425 (1992). With this procedure, we obtained transformation efficiencies of approximately I x 10⁵ transformants per microgram of DNA. Transformed cells were then inoculated from agar into YEPD complex media broth (100 ml) and grown overnight at 30°C. The YEPD broth was prepared as described in Kaiser et al., Methods in Yeast Genetics, Cold Spring Harbor Press. Cold Spring Harbor. N.Y, USA, p. 207 (1994). The overnight culture was then diluted to about 2 x 10⁶ cells/ml (approx. OD₆₀₀=0.1) into fresh YEPD broth (500 ml) and regrown to 1 x 10⁷ cells/ml (approx. OD₆₀₀=0.4-0.5). This usually took about 3 hours to complete.

The cells were then harvested and prepared for transformation by transfer into GS3 rotor bottles in a Sorval GS3 rotor at 5.000 rpm for 5 minutes, the supernatant discarded, and then resuspended into sterile water, and centrifuged again in 50 ml falcon tubes at 3.500 rpm in a Beckman GS-6KR centrifuge. The supernatant was discarded and the cells were subsequently washed with LiAc/TE (10 ml, 10 mM Tris-HCl, 1 mM EDTA pH 7.5, 100 mM Li₂OOCCH₃), and resuspended into LiAc/TE (2.5 ml).

Transformation took place by mixing the prepared cells (100 µl) with freshly denatured single stranded salmon testes DNA (Lofstrand Labs, Gaithersburg, MD. USA) and transforming DNA (1 µg, vol. < 10 µl) in microfuge tubes. The mixture was mixed briefly by vortexing, then 40% PEG/TE (600 µl, 40% polyethylene glycol-4000, 10 mM Tris-HCl, 1 mM EDTA, 100 mM Li₂OOCCH₃, pH 7.5) was added. This mixture was gently mixed and incubated at 30°C while agitating for 30 minutes. The cells were then heat shocked at 42°C for 15 minutes, and the reaction vessel centrifuged in a microfuge at 12,000 rpm for 5-10 seconds, decanted and resuspended into TE (500 µl, 10 mM Tris-HCl, 1 mM EDTA pH 7.5) followed by recentrifugation. The cells were then diluted into TE (1 ml) and aliquots (200 µl) were spread onto the selective media previously prepared in 150 mm growth plates (VWR).

Alternatively, instead of multiple small reactions, the transformation was performed using a single, large scale reaction, wherein reagent amounts were scaled up accordingly.

The selective media used was a synthetic complete dextrose agar lacking uracil (SCD-Ura) prepared as described in Kaiser et al., Methods in Yeast Genetics, Cold Spring Harbor Press, Cold Spring Harbor, N.Y., USA, p. 208-210 (1994). Transformants were grown at 30°C for 2-3 days.

The detection of colonies secreting amylase was performed by including red starch in the selective growth media. Starch was coupled to the red dye (Reactive Red-120, Sigma) as per the procedure described by Biely et al., Anal. Biochem., 172:176-179 (1988). The coupled starch was incorporated into the SCD-Ura agar plates at a final concentration of 0.15% (w/v), and was buffered with potassium phosphate to a pH of 7.0 (50-100 mM final concentration).

The positive colonies were picked and streaked across fresh selective media (onto 150 mm plates) in order to obtain well isolated and identifiable single colonies. This step also ensured maintenance of the plasmid amongst the transformants. Well isolated single colonies positive for amylase secretion were detected by direct incorporation of red starch into buffered SCD-Ura agar. Positive colonies were determined by their ability to break down starch resulting in a clear halo around the positive colony visualized directly.

### 4. Isolation of DNA by PCR Amplification

When a positive colony was isolated, a portion of it was picked by a toothpick and diluted into sterile water (30 µl) in a 96 well plate. At this time, the positive colonies were either frozen and stored for subsequent analysis or immediately amplified. An aliquot of cells (5 µl) was used as a template for the PCR reaction in a 25 µl volume containing: 0.5 µl Klentaq (Clontech, Palo Alto, CA); 4.0 µl 10 mM dNTP's (Perkin Elmer-Cetus); 2.5 µl Kentaq buffer(Clontech);0.25 µl forwardoligo 1; 0.25 µl reverse oligo 2; 12.5 µl distilled water. The sequence of the forward oligonucleotide 1 was:
TGTAAAACGACGGCCAGTTAAATAGACCTGCAATTATTAATCT
[SEQ ID NO:5]
The sequence of reverse oligonucleotide 2 was:
CAGGAAACAGCTATGACCACCTGCACACCTGCAAATCCATT
[SEQ ID NO:6]

PCR was then performed as follows:

| | | | |
|---|---|---|---|
| a. | Denature | | 92°C, 5 minutes |
| b. | 3 cycles of Denature | | 92°C, 30 seconds |
| | | Anneal | 59°C, 30 seconds |
| | | Extend | 72°C, 60 seconds |
| c. | 3 cycles of Denature | | 92°C, 30 seconds |
| | | Anneal | 57°C, 30 seconds |
| | | Extend | 72°C, 60 seconds |
| d. | 25 cycles of Denature | | 92°C, 30 seconds |
| | | Anneal | 55°C, 30 seconds |
| | | Extend | 72°C, 60 seconds |
| e. | | Hold | 4°C |

The underlined regions of the oligonucleotides annealed to the ADH promoter region and the amylase region, respectively, and amplified a 307 bp region from vector pSST-AMY.0 when no insert was present. Typically, the first 18 nucleotides of the 5' end of these oligonucleotides contained annealing sites for the sequencing primers. Thus, the total product of the PCR reaction from an empty vector was 343 bp. However, signal sequence-fused cDNA resulted in considerably longer nucleotide sequences.

Following the PCR, an aliquot of the reaction (5 µl) was examined by agarose gel electrophoresis in a 1% agarose using a Tris-Borate-EDTA (TBE) buffering system as described by Sambrook et al., supra. Clones resulting in a single strong PCR product larger than 400 bp were further analyzed by DNA sequencing after purification with a 96 Qiaquick PCR clean-up column (Qiagen Inc., Chatsworth, CA).

### 5. Identification of Full-length Clone

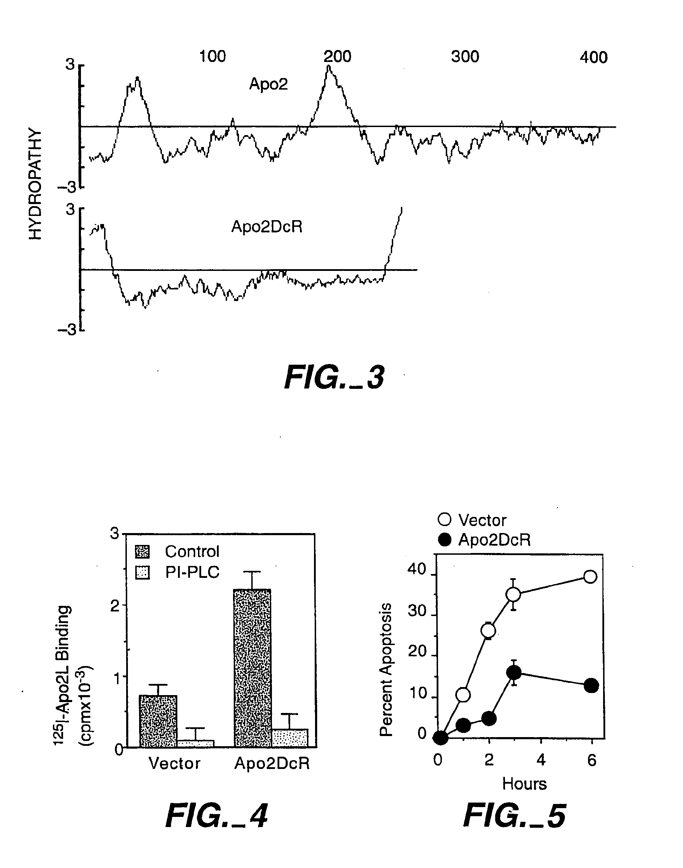

A cDNA sequence ("DNA21705") isolated in the above screen was found to have certain amino acid sequence similarity or homology with human TNFR1: 
TNFR1        81     CRECESG-SFTASENHLRHCLSCSKCRKEMG
                    *  *  *  .* . *.   *. *. *. .
DNA21705     164    CNPCTEGVDYTNASNNEPSCFPCTVCKSD--

QVEISSCTVDRDTVCGCRK (SEQ ID NO:7)
*   ****. ***** *..
QKHKSSCTMTRDTVCQCKE (SEQ ID NO:8)
 Based on the similarity, probes were generated from the sequence of DNA21705 and used to screen a human fetal lung library ("LIB25") prepared as described in paragraph 1 above. The cloning vector was pRK5B (pRK5B is a precursor of pRK5D that does not contain the SfiI site), and the cDNA size cut was less than 2800 bp. A full length clone was identified (DNA33085) (pRK5-hApo-2DcR) (also referred to as Apo2-DcR deposited as ATCC 209087. as indicated below) that contained a single open reading frame with an apparent translational initiation site at nucleotide positions 193-195 [Kozak et al., supra] and ending at the stop codon found at nucleotide positions 970-972 (Fig. 1A; SEQ ID NO:2). The predicted polypeptide precursor is 259 amino acids long and has a calculated molecular weight of approximately 27.4 kDa. Sequence analysis indicated an N-terminal signal peptide, two cysteine-rich domains, a sequence that contains four nearly identical 15 amino acid tandem repeats, and a hydrophobic C-terminal region. (Figures 2 and 3). The hydrophobic sequence at the C-terminus is preceded by a pair of small amino acids (Ala223 and Ala224); this structure and the absence of an apparent cytoplasmic domain suggests that Apo-2DcR may be a glycosylphosphatydilinositol (GPI) anchored protein [see, Moran, J. Biol. Chem., 266:1250-1257 (1991)]. Apo-2DcR contains five potential N-linked glycosylation sites. (Fig. 2)

TNF receptor family proteins are typically characterized by the presence of multiple (usually four) cysteine-rich domains in their extracellular regions -- each cysteine-rich domain being approximately 45 amino acids long and containing approximately 6, regularly spaced, cysteine residues. Based on the crystal structure of the type 1 TNF receptor, the cysteines in each domain typically form three disulfide bonds in which usually cysteines 1 and 2, 3 and 5, and 4 and 6 are paired together. Like DR4 and Apo-2 (described further below), Apo-2DcR contains two extracellular cysteine-rich pseudorepeats (Fig. 2), whereas other identified mammalian TNFR family members contain three or more such domains [Smith et al., Cell, 76:959 (1994)].

Based on an alignment analysis of the full-length sequence shown in Figure 1A (SEQ ID NO:1), Apo-2DcR shows more sequence identity to DR4 (60%) and Apo-2 (50%) than to other apoptosis-linked receptors, such as Apo-3, TNFR1, or Fas/Apo-1.

In Fig. 1B, Applicants have shown that the apparent translational initiation site may alternatively be assigned at nucleotide positions 93-95 (identified in Fig. 1B as amino acid residue -40; SEQ ID NO:4). The Apo-2DcR shown in Fig. 1B includes amino acid residues -40 to 259.

### EXAMPLE 2

### Binding of Apo-2DcR to Apo-2L and Effect of PI-PLC on Apo-2DcR Activity

To test whether Apo-2DcR binds to Apo-2L. and to assess whether Apo-2DcR is GPI-linked, binding of radioiodinated Apo-2L to Apo-2DcR-transfected 293 cells was analyzed. The effect of pre-treatment of the cells with phosphatidylinositol-specific phospholipase C (PI-PLC) on the binding was also analyzed.

Human 293 cells (ATCC CRL 1573) were plated in 100mm plates (1 x 10⁶ cells/plate) and transfected with 20 µg/plate pRK5 or pRK5 encoding the full-length Apo-2DcR (described in Example 1, ATCC deposit 209087) using calcium phosphate precipitation. After 24 hours, the cells were harvested in PBS/10mM EDTA, washed in phosphate buffered saline (PBS), resuspended in 2 ml PBS per original plate and divided into two 1 ml aliquots per transfection. PI-PLC [Treanor et al., Nature, 382:80-83 (1996)](1 µg/ml) was added to one of the two aliquots derived from each transfection, and the cells were incubated 1 hour at 37° C. The cells were washed and respuspended in 1 ml PBS containing 1% BSA (Sigma), and 0.04 ml aliquots were placed into tubes in triplicate. To these tubes was added approximately20.000cpm ¹²⁵I-Apo-2L (Apo-2L is described in Pitti et al., supra, and was radioiodinated by conventional lactoperoxidase methodology) in 0.005ml, along with 0.005 ml PBS, or 0.005 µl unlabeled Apo-2L in PBS (final concentration 0.5 µg/ml) for determination of nonspecific binding. After a 1 hour incubation at room temperature, the cells were washed in ice cold PBS. pelleted, and counted for radioactivity.

Transfection by Apo-2DcR led to a marked increase in the amount of specific Apo-2L binding, indicating that Apo-2DcR binds Apo-2L (Fig. 4). Treatment with PI-PLC caused a marked reduction in Apo-2L binding, indicating that Apo-2DcR is a GPI-anchored receptor (Fig. 4).

### EXAMPLE 3

### Inhibition of Apo-2L Function by Full-length Apo-2DcR

The absence of a cytoplasmic region in Apo-2DcR suggested that this receptor is involved in modulation, rather than in transduction of Apo-2L signaling. Thus, the effect of Apo-2DcR transfection on cellular responsiveness to Apo-2L was examined.

Human 293 cells, which express both DR4 and Apo-2 mRNA (data not shown), were plated in 100mm plates (1 x 10⁶ cells/plate)and transfected with 3 µg per plate pRK encoding green fluorescent protein (GFP; purchased from Clontech)togetherwith 20 µg/plate pRK5 or pRK5-hApo-2DcR (see Example 2) using calcium phosphate precipitation. After 18 hours, the cells were treated with PBS or with Apo-2L (Pitti et al., supra, 0.5 µg/ml) and examined over 6 hours under a fluorescence microscope equipped with Hoffman optics (which enables clear viewing of non-fixed cells on plastic). GFP-positive cells were identified by green fluorescence and scored for apoptosis by morphologic criteria such as membrane blebbing and cytoplasmic condensation.

Transfection by Apo-2DcR markedly reduced responsiveness to Apo-2L as measured by apoptosis induction (Fig. 5).

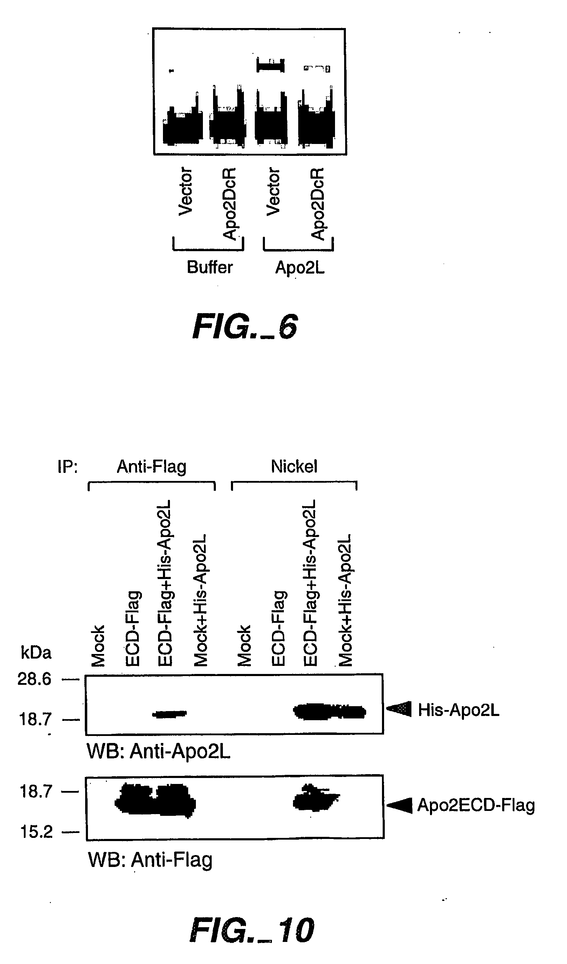

In a similar experiment, the 293 cells were transfected by pRK5 or pRKS-hApo-2DcR (20 µg/plate) and analyzed 18 hours later for activation of NF-κB by Apo-2L (0.5 µg/ml), as in Example 10 below. The results showed that Apo-2DcR inhibits Apo-2L function as measured by apoptosis induction as well as by NF-κB activation (Fig. 6).

### EXAMPLE 4

### Northern Blot Analysis

Expression of Apo-2DcR mRNA in human tissues was examined by Northern blot analysis. Human RNA blots were hybridized to a 1.2 kilobase ³²P-labelled DNA probe based on the full length Apo-2DcR cDNA: the probe was generated by digesting the pRK5-Apo-2DcRplasm id with EcoRI and purifying the Apo-2DcR cDNA insert. Human fetal RNA blot MTN (Clontech), human adult RNA blot MTN-II (Clontech) and human cancer cell line RNA blot (Clontech) were incubated with the DNA probes. Blots were incubated with the probes in hybridization buffer (5X SSPE; 2X Denhardt's solution: 100 mg/mL denatured sheared salmon sperm DNA; 50% formamide: 2% SDS) for 60 hours at 42°C. The blots were washed several times in 2X SSC; 0.05% SDS for 1 hour at room temperature, followed by a 30 minute wash in 0.1X SSC; 0.1% SDS at 50°C. The blots were developed after overnight exposure by phosphorimager analysis (Fuji).

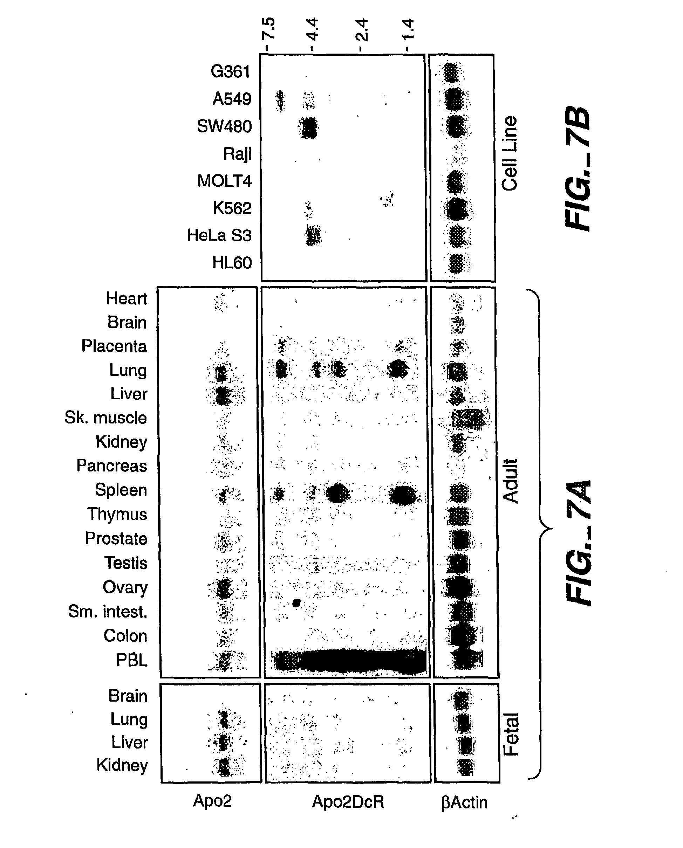

As shown in Fig. 7A. several Apo-2DcRmRNA transcriptswere detected. Relativelyhigh expression was seen in adult peripheral blood leukocytes (PBL), spleen, lung, liver and placenta. Some adult tissues that express Apo-2DcR, e.g., PBL and spleen, have been shown to express Apo-2 (Example 11 below) and DR4 [Pan et al., supra].

As shown in Fig. 7B. the Apo-2DcR message is absent from most of the human tumor cell lines examined (namely, HL60 promyelocytic leukemia, HeLa S3 cervical carcinoma, K562 chronic myelogenous leukemia, MOLT4 acute lymphoblastic leukemia, SW480 colorectal adenocarcinoma, A549 lung carcinoma, and G361 melanoma), and particularly the approximate 1.5 kB transcript which corresponds in size to the Apo-2DcR cDNA. The apparent expression of Apo-2DcR in the above-mentioned normal human tissues but not the identified tumor cell types suggests that the Apo-2DcR receptor may allow for preferential killing of cancer cells by Apo-2 ligand (possibly through protection of normal cells but not cancerous cells).

### EXAMPLE 5

### Isolation of cDNA clones Encoding Human Apo-2

An expressed sequence tag (EST) DNA database (LIFESEQ^{™}, Incyte Pharmaceuticals, Palo Alto, CA) was searched and an EST was identified which showed homology to the death domain of the Apo-3 receptor [Marsterset al., Curr. Biol., 6:750 (1996)]. Human pancreas ("LIB55") and human kidney ("LIB28") cDNA libraries (prepared as described in Example I above in pRK5 vectors), were screened by hybridization with a synthetic oligonucleotide probe: GGGAGCCGCTCATGAGGAAGTTGGGCCTCATGGACAATGAGATAAAGGTGGCTAAAGCTGAG GCAGCGGG (SEQ ID NO:9) based on the EST.

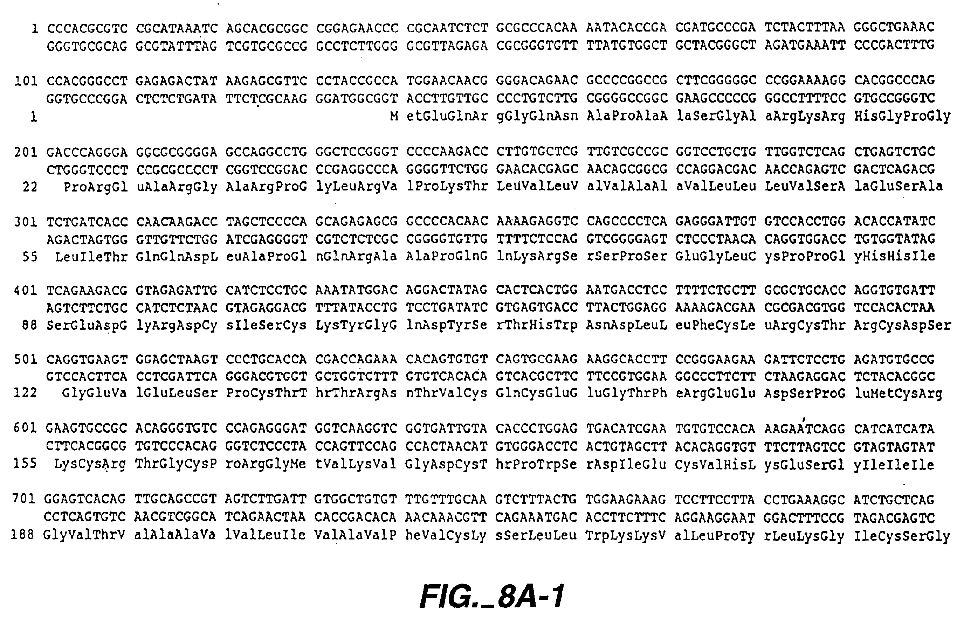

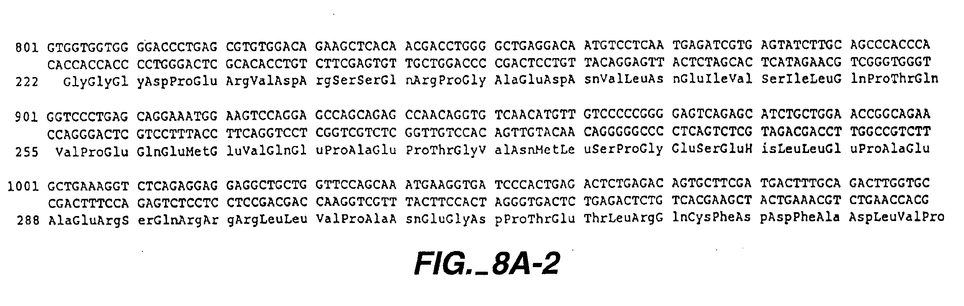

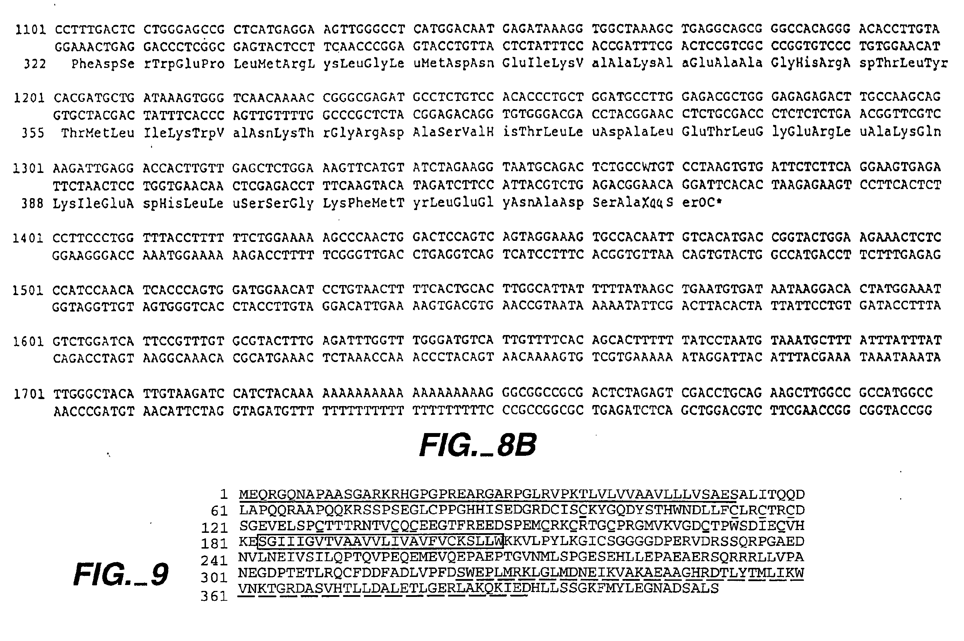

Three cDNA clones were sequenced in entirety. The overlapping coding regions of the cDNAs were identical except for codon 410 0 (using the numbering system for Fig. 8); this position encoded a leucine residue (TTG) in both pancreatic cDNAs, and a methionine residue (ATG) in the kidney cDNA, possibly due to polymorphism.

The entire nucleotide sequence of Apo-2 is shown in Figure 8 (SEQ ID NO:10). Clone 27868 (also referred to as pRK5-Apo-2 deposited as ATCC 209021, as indicated below) contains a single open reading frame with an apparent translational initiation site at nucleotide positions 140-142 [Kozak et al., supra] and ending at the stop codon found at nucleotide positions 1373-1375 (Fig. 8; SEQ ID NO:10). The predicted polypeptide precursor is 411 amino acids long, a type I transmembrane protein, and has a calculated molecular weight of approximately 45 kDa. Hydropathy analysis (not shown) suggested the presence of a signal sequence (residues 1-53), followed by an extracellular domain (residues 54-182), a transmembrane domain (residues 183-208), and an intracellular domain (residues 209-411) (Fig. 9; SEQ ID NO:11). N-terminal amino acid sequence analysis of Apo-2-IgG expressed in 293 cells showed that the mature polypeptide starts at amino acid residue 54, indicating that the actual signal sequence comprises residues 1-53.

Like DR4 and Apo-2DcR, Apo-2 contains two extracellular cysteine-rich pseudorepeats (Fig. 9), whereas other identified mammalian TNFR family members contain three or more such domains [Smith et al., Cell, 76:959 (1994)].

The cytoplasmic region of Apo-2 contains a death domain (amino acid residues 324-391 shown in Fig. 8; see also Fig. 2) which shows significantly more amino acid sequence identity to the death domain of DR4 (64%) than to the death domain of TNFR1 (30%); CD95 (19%); or Apo-3/DR3 (29%) (Fig. 2). Four out of six death domain amino acids that are required for signaling by TNFR1 [Tartaglia et al., supra] are conserved in Apo-2 while the other two residues are semi-conserved (see Fig. 2).

Based on an alignment analysis (using the ALIGN computer program) of the full-length sequence. Apo-2 shows more sequence identity to DR4 (55%) than to other apoptosis-linked receptors, such as TNFR1 (19%); CD95 (17%); or Apo-3 (also referred to as DR3, WSL-1 or TRAMP) (29%).

### EXAMPLE 6

### A. Expression of Apo-2 ECD

A soluble extracellular domain (ECD) fusion construct was prepared. An Apo-2 ECD (amino acid residues 1-184 shown in Figure 8) was obtained by PCR and fused to a C-terminal Flag epitope tag (Sigma). (The Apo-2 ECD construct included residues 183 and 184 shown in Figure 8 to provide flexibility at the junction, even though residues 183 and 184 are predicted to be in the transmembrane region). The Flag epitope-tagged molecule was then inserted into pRK5, and expressed by transient transfection into human 293 cells (ATCC CRL 1573).

After a 48 hour-incubation, the cell supernatants were collected and either used directly for co-precipitation studies (see Example 7) or subjected to purification of the Apo-2 ECD-Flag by affinity chromatography on anti-Flag agarose beads, according to manufacturer's instructions (Sigma).

### B. Expression of Ago-2 ECD as an Immunoadhesin

A soluble Apo-2 ECD immunoadhesin construct was prepared. The Apo-2 ECD (amino acids 1-184 shown in Fig. 8) was fused to the hinge and Fc region of human immunoglobulinG₁ heavy chain in pRK5 as described previously [Ashkenazi et al., Proc. Natl. Acad. Sci., 88:10535-10539(1991)]. The immunoadhesin was expressed by transient transfection into human 293 cells and purified from cell supernatants by protein A affinity chromatography, as described by Ashkenazi et al., supra.

### EXAMPLE 7

### Immunoprecipitation Assay Showing Binding Interaction Between Apo-2 and Apo-2 Ligand

To determine whether Apo-2 and Apo-2L interact or associate with each other, supernatants from mock-transfected 293 cells or from 293 cells transfected with Apo-2 ECD-Flag (described in Example 6 above) (5 ml) were incubated with 5 µg poly-histidine-tagged soluble Apo-2L [Pitti et al.. supra] for 30 minutes at room temperature and then analyzed for complex formation by a co-precipitation assay.

The samples were subjected to immunoprecipitation using 25 µl anti-Flag conjugated agarose beads (Sigma) or Nickel-conjugated agarose beads (Qiagen). After a 1.5 hour incubation at 4° C. the beads were spun down and washed four times in phosphate buffered saline (PBS). By using anti-Flag agarose, the Apo-2L was precipitated through the Flag-tagged Apo-2 ECD; by using Nickel-agarose, the Apo-2 ECD was precipitated through the His-tagged Apo-2L. The precipitated proteins were released by boiling the beads for 5 minutes in SDS-PAGEbuffer, resolved by electrophoresis on 12% polyacrylamide gels, and then detected by immunoblot with anti-Apo-2L or anti-Flag antibody (2 µg/ml) as described in Marsters et al., J. Biol. Chem., 272:14029-14032 (1997).

The results, shown in Figure 10, indicate that the Apo-2 ECD and Apo-2L can associate with each other.

The binding interaction was further analyzed by purifying Apo-2 ECD from the transfected 293 cell supernatants with anti-Flag beads (see Example 6) and then analyzing the samples on a BIACORE^{™} instrument. The BIACORE^{™} analysis indicated a dissociation constant (K_{d}) of about 1 nM. BIACORE^{™} analysis also showed that the Apo-2 ECD is not capable of binding other apoptosis-inducing TNF family members, namely, TNF-alpha (Genentech, Inc., Pennica et al., Nature, 312:724 (1984), lymphotoxin-alpha (Genentech, Inc.), or Fas/Apo-1 ligand (Alexis Biochemicals). The data thus shows that Apo-2 is a specific receptor for Apo-2L.

### EXAMPLE 8

### Induction of Apoptosis by Apo-2

Because death domains can function as oligomerization interfaces, over-expression of receptors that contain death domains may lead to activation of signaling in the absence of ligand [Frazeret al., supra, Nagata et al., supra]. To determine whether Apo-2 was capable of inducing cell death, human 293 cells or HeLa cells (ATCC CCL 2.2) were transiently transfected by calcium phosphate precipitation (293 cells) or electroporation (HeLa cells) with a pRK5 vector or pRK5-based plasmids encoding Apo-2 and/or CrmA. When applicable, the total amount of plasmid DNA was adjusted by adding vector DNA. Apoptosis was assessed 24 hours after transfection by morphology (Fig. 11A); DNA fragmentation (Fig. 11B); or by FACS analysis of phosphatydilserineexposure (Fig. 11C) as described in Marsters et al., Curr. Biol., 6:1669 (1996). As shown in Figs. 11A and 11B, the Apo-2 transfected 293 cells underwent marked apoptosis.

For samples assayed by FACS, the HeLa cells were co-transfected with pRK5-CD4 as a marker for transfection and apoptosis was determined in CD4-expressingcells; FADD was co-transfectedwith the Apo-2 plasmid; the data are means ± SEM of at least three experiments, as described in Marsters et al., Curr. Biol., 6:1669(1996). The caspase inhibitors, DEVD-fmk (Enzyme Systems) or z-VAD-fmk (Research Biochemicals Intl.) were added at 200 µM at the time of transfection. As shown in Fig. 11C, the caspase inhibitors CrmA, DEVD-fmk, and z-VAD-fmk blocked apoptosis induction by Apo-2, indicating the involvement of Ced-3-like proteases in this response.

FADD is an adaptor protein that mediates apoptosis activation by CD95, TNFR1, and Apo-3/DR3 [Nagata et al., supra], but does not appear necessary for apoptosis induction by Apo-2L [Marsters et al., supra] or by DR4 [Pan et al., supra]. A dominant-negative mutant form of FADD, which blocks apoptosis induction by CD95, TNFR1, or Apo-3/DR3 [Frazer et al., supra; Nagata et al., supra; Chinnayian et al., supra] did not inhibit apoptosis induction by Apo-2 when co-transfected into HeLa cells with Apo-2 (Fig. 11C). These results suggest that Apo-2 signals apoptosis independently of FADD. Consistent with this conclusion, a glutathione-S-transterase fusion protein containing the Apo-2 cytoplasmic region did not bind to *in vitro* transcribed and translated FADD (data not shown).

### EXAMPLE 9

### Inhibition of Apo-2L Activity by Soluble Apo-2 ECD

Soluble Apo-2L (0.5 µg/ml, prepared as described in Pitti et al., supra) was pre-incubated for 1 hour at room temperature with PBS buffer or affinity-purified Apo-2 ECD (5 µg/ml) together with anti-Flag antibody (Sigma) (1 µg/ml) and added to HeLa cells. After a 5 hour incubation, the cells were analyzed for apoptosis by FACS (as above) (Fig. 11D).

Apo-2L induced marked apoptosis in HeLa cells, and the soluble Apo-2 ECD was capable of blocking Apo-2L action (Fig. 11D), confirming a specific interaction between Apo-2L and Apo-2. Similar results were obtained with the Apo-2 ECD immunoadhesin(Fig. 11E). Dose-response analysis showed half-maximal inhibition at approximately 0.3 nM Apo-2 immunoadhesin (Fig. 11E).

### EXAMPLE 10

### Activation of NF-κB by Apo-2

An assay was conducted to determine whether Apo-2 activates NF-κB.

HeLa cells were transfected with pRK5 expression plasmids encoding full-length native sequence Apo-2, DR4 or Apo-3 and harvested 24 hours after transfection. Nuclear extracts were prepared and 1 µg of nuclear protein was reacted with a ³²P-labelled NF-κB-specific synthetic oligonucleotide probe ATCAGGGACTTTCCGCTGGGGACTTTCCG (SEQ ID NO:12) [see, also, MacKay et al., J. Immunol., 153:5274-5284 (1994)], alone or together with a 50-fold excess of unlabelled probe, or with an irrelevant ³²P-labelled synthetic oligonucleotide AGGATGGGAAGTGTGTGATATATCCTTGAT (SEQ ID NO: 13). In some samples, antibody to p65/RelA subunits of NF-κB (1 µg/ml; Santa Cruz Biotechnology) was added. DNA binding was analyzed by an electrophoretic mobility shift assay as described by Hsu et al., supra; Marsters et al., supra, and MacKay et al., supra.

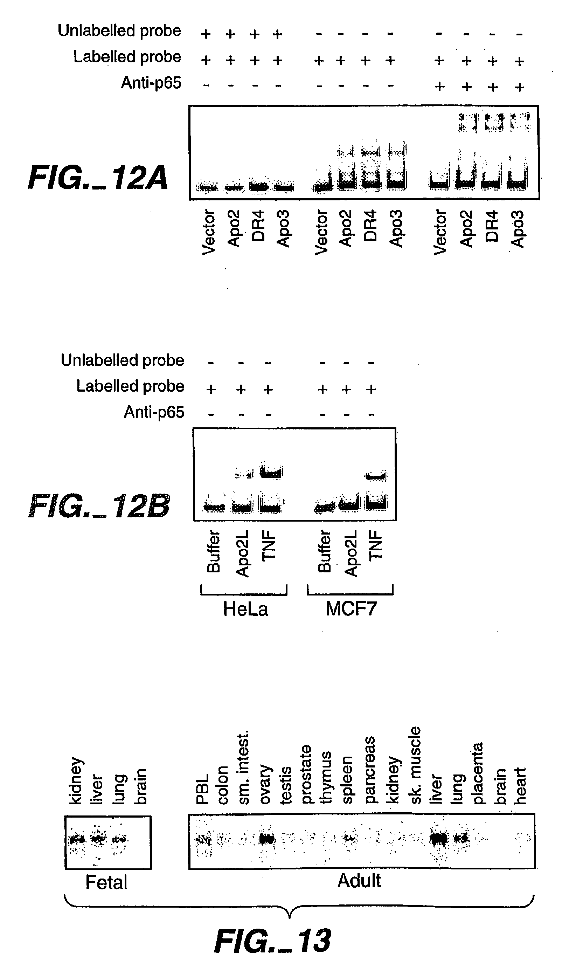

The results are shown in Fig. 12. As shown in Fig. 12A, upon transfection into HeLa cells, both Apo-2 and DR4 induced significant NF-κB activation as measured by the electrophoretic mobility shift assay; the level of activation was comparable to activation observed for Apo-3/DR3. Antibody to the p65/RelA subunit of NF-κB inhibited the mobility of the NF-κB probe, implicating p65 in the response to all 3 receptors.

An assay was also conducted to determine if Apo-2L itself can regulate NF-κB activity. HeLa cells or MCF7 cells (human breast adenocarcinoma cell line, ATCC HTB 22) were treated with PBS buffer, soluble Apo-2L (Pitti et al., supra) or TNF-alpha (Genentech, Inc., see Pennica et al., Nature, 312:724 (1984)) (1 µg/ml) and assayed for NF-κB activity as above. The results are shown in Fig. 12B. The Apo-2L induced a significant NF-κB activation in the treated HeLa cells but not in the treated MCF7 cells; the TNF-alpha induced a more pronounced activation in both cell lines. Several studies have disclosed that NF-κB activation by TNF can protect cells against TNF-induced apoptosis [Nagata, supra].

The effects of a NF-κB inhibitor, ALLN (N-acetyl-Leu-Leu-norleucinal) and a transcription inhibitor, cyclohexamide, were also tested. The HeLa cells (plated in 6-well dishes) were preincubated with PBS buffer, ALLN (Calbiochem)(40 µg/ml) or cyclohexamide(Sigma)(50 µg/ml) for 1 hour before addition of Apo-2L (1 µg/ml). After a 5 hour incubation, apoptosis was analyzed by FACS (see Fig. 12C).

The results are shown in Fig. 12C. Both ALLN and cyclohexamide increased the level of Apo-2L-induced apoptosis in the HeLa cells. The data indicates that Apo-2L can induce protective NF-κB-dependent genes. The data also indicates that Apo-2L is capable of activating NF-κB in certain cell lines and that both Apo-2 and DR4 may mediate that function.

### EXAMPLE 11

### Northern Blot Analysis

Expression of Apo-2 mRNA in human tissues was examined by Northern blot analysis. Human RNA blots were hybridized to a 4.6 kilobase ³²P-labelled DNA probe based on the full length Apo-2 cDNA; the probe was generated by digesting the pRK5-Apo-2 plasmid with EcoRI. Human fetal RNA blot MTN (Clontech)and human adult RNA blot MTN-11 (Clontech)were incubated with the DNA probes. Blots were incubated with the probes in hybridization buffer (5X SSPE; 2X Denhardt's solution; 100 mg/mL denatured sheared salmon sperm DNA: 50% formamide; 2% SDS) for 60 hours at 42°C. The blots were washed several times in 2X SSC; 0.05% SDS for 1 hour at room temperature, followed by a 30 minute wash in 0.1X SSC; 0.1% SDS at 50°C. The blots were developed after overnight exposure.

As shown in Fig. 13, a predominant mRNA transcript of approximately 4.6kb was detected in multiple tissues. Expression was relatively high in fetal and adult liver and lung, and in adult ovary and peripheral blood leukocytes (PBL), while no mRNA expression was detected in fetal and adult brain. Intermediate levels of expression were seen in adult colon, small intestine, testis, prostate, thymus, pancreas, kidney, skeletal muscle, placenta, and heart. Several adult tissues that express Apo-2, e.g., PBL, ovary, and spleen, have been shown previously to express DR4 [Pan et al., supra], however, the relative levels of expression of each receptor mRNA appear to be different.

### EXAMPLE 12

### Chromosomal Localization of the Apo-2, DR4 and Apo-2DcR genes

Chromosomal localization of the human Apo-2 gene was examined by radiation hybrid (RH) panel analysis. RH mapping was performed by PCR using a human-mouse cell radiation hybrid panel (Research Genetics) and primers based on the coding region of the Apo-2 cDNA [Gelb et al., Hum. Genet., 98:141 (1996)]. Analysis of the PCR data using the Stanford Human Genome Center Database indicates that Apo-2 is linked to the marker D8S481, with an LOD of 11.05; D8S481 is linked in turn to D8S2055, which maps to human chromosome 8p21. A similar analysis of DR4 showed that DR4 is linked to the marker D8S2127 (with an LOD of 13.00), which maps also to human chromosome 8p21. Analysis of Apo-2DcR using radiation hybrid panel examination showed that the Apo-2DcR gene is linked to the marker WI-6536, which in turn is linked to D8S298, which maps also to human chromosome 8p21 and is nested between D8S2005 and D8S2127. Thus, the human genes for three Apo-2L receptors, Apo-2, Apo-2DcR and DR4, all map to chromosome 8p21.

To Applicants' present knowledge, to date, no other member of the TNFR gene family has been located to chromosome 8p.

### EXAMPLE 13

### Preparation of Monoclonal Antibodies for Apo-2DcR

Balb/c mice (obtained from Charles River Laboratories) were immunized by injecting 0.5µg/50µl of an Apo-2DcR immunoadhesin protein (diluted in MPL-TDM adjuvant purchased from Ribi Immunochemical Research Inc., Hamilton, MT) 11 times into each hind foot pad at 3 day intervals. The Apo-2DcR immunoadhesin protein was generated by fusing an N-terminal region of Apo-2DcR (amino acids 1-165 shown in Fig. 1A) to the hinge and Fc region of human immunoglobulin G₁ heavy chain in pRK5 as described previously [Ashkenazi et al., Proc. Natl. Acad. Sci., 88:10535-10539 (1991)]. The immunoadhesin protein was expressed by transient transfection into human 293 cells and purified from cell supernatants by protein A affinity chromatography, as described by Ashkenazi et al., supra.

Three days after the final boost, popliteal lymph nodes were removed from the mice and a single cell suspension was prepared in DMEM media (obtained from Biowhitakker Corp.) supplemented with 1% penicillin-streptomycin. The lymph node cells were then fused with murine myeloma cells P3X63AgU.1 (ATCC CRL 1597) using 35% polyethylene glycol and cultured in 96-well culture plates. Hybridomas resulting from the fusion were selected in HAT medium. Ten days after the fusion, hybridoma culture supernatants were screened in an ELISA to test for the presence of monoclonal antibodies binding to the Apo-2DcR immunoadhesin protein.

In the ELISA, 96-well microtiter plates (Maxisorb; Nunc, Kamstrup, Denmark) were coated by adding 50 µl of 2 µg/ml goat anti-human IgG Fc (purchased from Cappel Laboratories) in PBS to each well and incubating at 4°C overnight. The plates were then washed three times with wash buffer (PBS containing 0.05% Tween 20). The wells in the microtiter plates were then blocked with 200 µl of 2.0% bovine serum albumin in PBS and incubated at room temperature for 1 hour. The plates were then washed again three times with wash buffer.

After the washing step, 50 µl of 0.4 µg/ml Apo-2DcR immunoadhesin protein (as described above) in assay buffer (PBS containing 0.5% BSA) was added to each well. The plates were incubated for 1 hour at room temperature on a shaker apparatus, followed by washing three times with wash buffer.

Following the wash steps, 100 µl of the hybridoma supernatants or purified antibody (using Protein G-sepharose columns) (1 µg/ml) was added to designated wells in assay buffer. 100 µl of P3X63AgU.1 myeloma cell conditioned medium was added to other designated wells as controls. The plates were incubated at room temperature for 1 hour on a shaker apparatus and then washed three times with wash buffer.

Next, 50 µl HRP-conjugatedgoat anti-mouse IgG Fc (purchased from Cappel Laboratories), diluted 1:1000 in assay buffer, was added to each well and the plates incubated for 1 hour at room temperature on a shaker apparatus. The plates were washed three times with wash buffer, followed by addition of 50 µl of substrate (TMB microwell peroxidase substrate, Kirkegaard & Perry, Gaithersburg, MD) to each well and incubation at room temperature for 10 minutes. The reaction was stopped by adding 50 µl of TMB 1-component stop solution (diethyl glycol, Kirkegaard & Perry) to each well, and absorbance at 450 nm was read in an automated microtiter plate reader.

Of the hybridoma supernatants screened in the ELISA, 47 supernatants tested positive (calculated as approximately 4 times above background). The supernatants testing positive in the ELISA were further analyzed by FACS analysis using HUMEC cells(a human microvascularendothelial cell line expressing Apo-2DcR; Cell Systems, Kirkland. WA) and PE-conjugated goat anti-mouse IgG. For this analysis. 25 µl of cells suspended (at 4 X 10⁶ cells/ml) in cell sorter buffer (PBS containing 1% FCS and 0.02% NaN₃) were added to U-bottom microtiter wells, mixed with 100 µl of culture supernatant or purified antibody (purified on Protein G-sepharose columns) (10 µg /ml) in cell sorter buffer, and incubated for 30 minutes on ice. The cells were then washed and incubated with 100 µl PE-conjugated goat anti-mouse IgG for 30 minutes at 4°C. Cells were then washed twice, resuspended in 200 µl of cell sorter buffer and then analyzed by FACScan (Becton Dickinson, Mountain View, CA). FACS analysis showed 12/35 supernatants were positive for anti-Apo-2 antibodies.

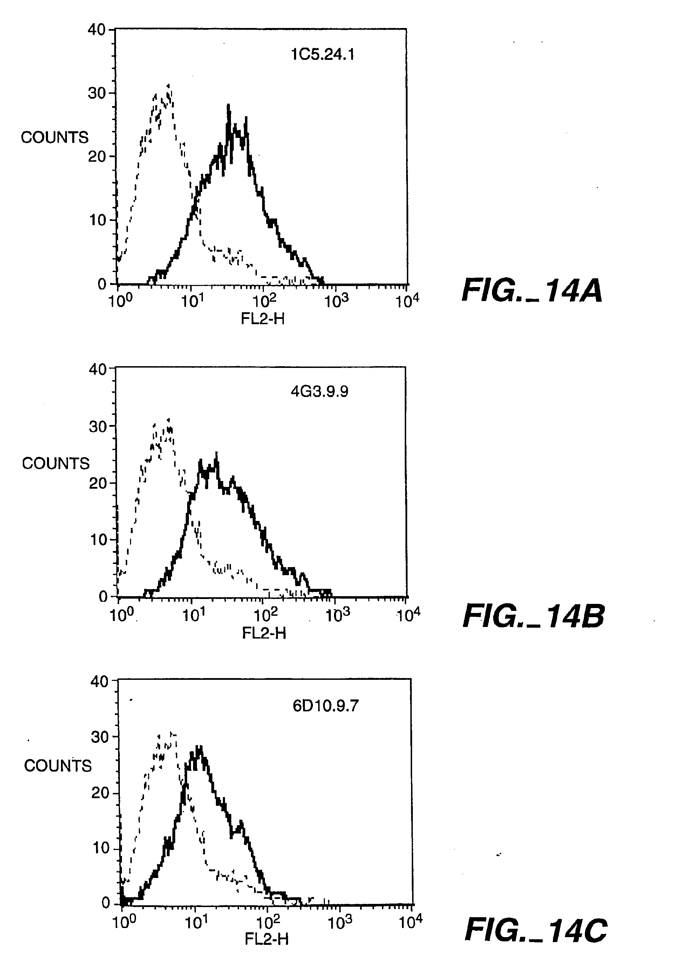

Figure 14 shows the FACS staining of HUMEC cells incubated with the Apo-2DcR antibodies, referred to as 4G3.9.9; 6D 10.9.7; and IC5.24.1. As shown in Figure 14, the respective antibodies recognize the Apo-2DcR receptor expressed in HUMEC cells.

### EXAMPLE 14

### ELISA Assay to Test Binding of Apo-2DcR Antibodies to Other Apo-2 Ligand Receptors

An ELISA was conducted to determine if the monoclonal antibodies described in Example 13 were able to bind other known Apo-2L receptors beside Apo-2DcR. Specifically, the 4G3.9.9; 6D10.9.7; and IC5.24.1 antibodies, respectively, were tested for binding to the Apo-2DcR described herein and to DR4 [Pan et al., supra], Apo-2 [described in the Examples above], and DcR2 [Marsters et al., Curr. Biol., 7:1003-1006 (1997)]. The ELISA was performed essentially as described in Example 13 above.

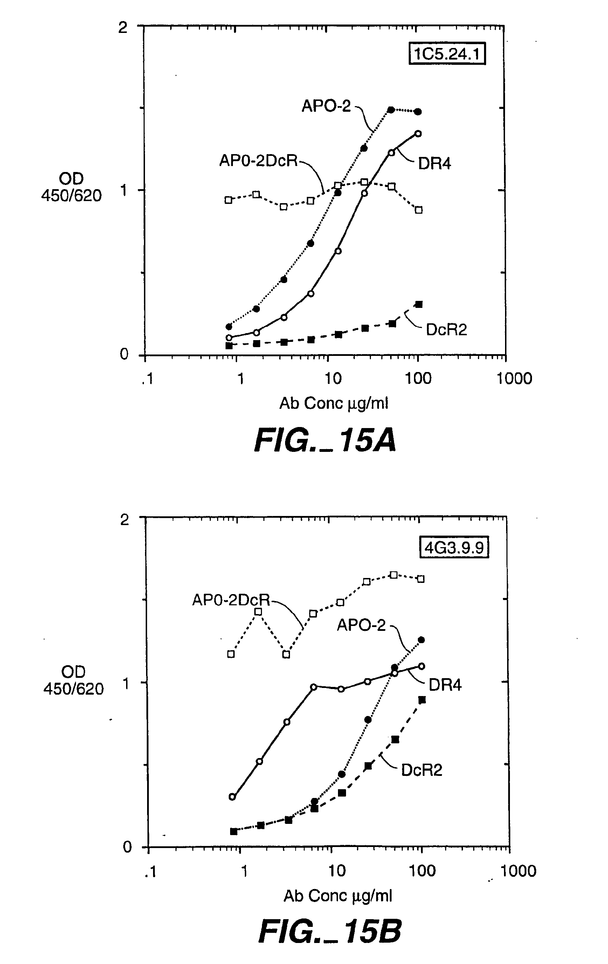

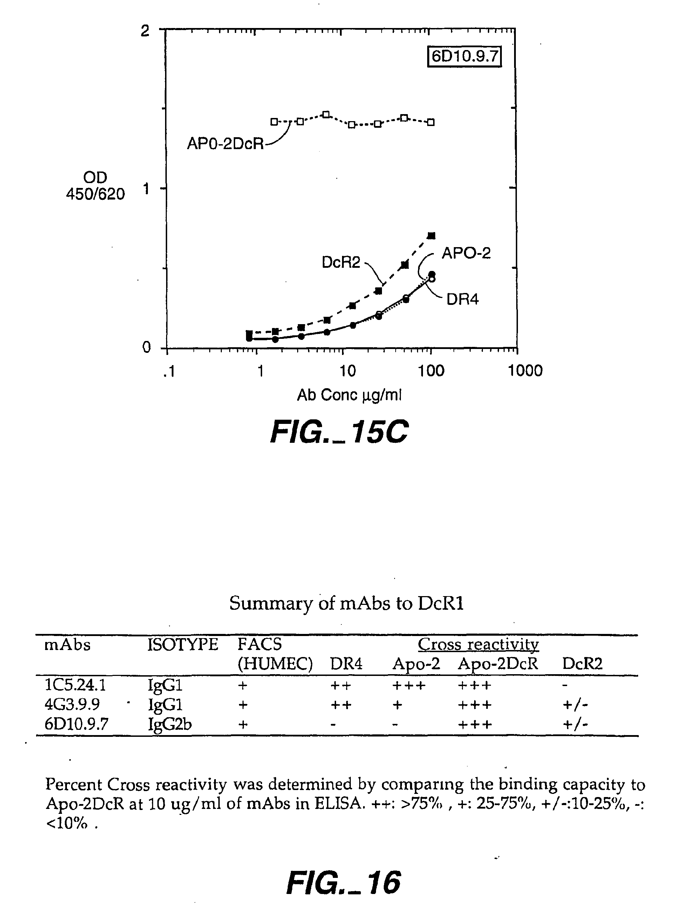

The results are shown in Figure 15. The Apo-2DcR antibody 4G3.9.9 bound to Apo-2DcR. The 4G3.9.9 antibody also showed some cross-reactivity to DR4 and Apo-2, as well as somewhat limited cross-reactivity to DcR2. The 6D10.9.7 antibody bound to Apo-2DcR and showed somewhat limited cross-reactivityto DR4, Apo-2 and DcR2. Finally, the IC5.24.1 antibody bound to Apo-2DcR and showed some cross-reactivity to DR4. The IC5.24.1 antibody exhibited somewhat less cross-reactivity to Apo-2 and DcR2. A summary of the cross-reactive properties is also provided in Figure 16.

### EXAMPLE 15

### Antibody Isotyping

The isotype of the Apo-2DcR antibodies(as described above in Examples 13 and 14) was determined by coating microtiterplates with isotype specific goat anti-mouse Ig (Fisher Biotech, Pittsburgh, PA) overnight at 4°C. The plates were then washed with wash buffer (as described in Example 13 above). The wells in the microtiter plates were then blocked with 200 µl of 2% bovine serum albumin (BSA) and incubated at room temperature for one hour. The plates were washed again three times with wash buffer. Next, 100 µl of hybridoma culture supernatant or 5 µg/ml of purified antibody was added to designated wells. The plates were incubated at room temperature for 30 minutes and then 50 µl HRP-conjugated goat anti-mouse IgG (as described above in Example 13) was added to each well. The plates were incubated for 30 minutes at room temperature. The level of HRP bound to the plate was detected using HRP substrate as described above.

The isotyping analysis showed that the 4G3.9.9 and IC5.24.1 antibodies are IgG1 antibodies. The analysis also showed that the 6D10.9.7 antibody is an IgG2b antibody. These results are also shown in Figure 16.

### Deposit of Material

The following materials have been deposited with the American Type Culture Collection, 10801 University Blvd., Manassas, Virginia USA (ATCC):

| Material | ATCC Dep. No. | Deposit Date |
|---|---|---|
| pRK5-Apo-2 | 209021 | May 8, 1997 |
| Apo2-DcR | 209087 | May 30, 1997 |
| 4G3.9.9 | --------- | --------- |
| 6D10.9.7 | --------- | --------- |
| IC5.24.1 | --------- | --------- |

This deposit was made under the provisionsofthe Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposit will be made available by ATCC under the terms of the Budapest Treaty, and subject to an agreement between Genentech, Inc. and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissionerof Patents and Trademarks to be entitled thereto according to 35 USC §122 and the Commissioner's rules pursuant thereto (including 37 CFR §1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

The foregoing written specification is considered to be sufficient to enable one skilled in the art to practice the invention. The present invention is not to be limited in scope by the construct deposited, since the deposited embodiment is intended as a single illustration of certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description and fall within the scope of the appended claims.

## Claims

**1.** An isolated Apo-2DcR polypeptide having at least about 80% amino acid sequence identity with native sequence Apo-2DcR polypeptide comprising amino acid residues 1 to 259 of Fig. 1A (SEQ ID NO: 1).

**2.** The Apo-2DcR polypeptide of claim 1, wherein:
(i) said Apo-2DcR polypeptide has at least about 90% amino acid sequence identity; or
(ii) said Apo-2DcR polypeptide has at least about 95% amino acid sequence identity.

**4.** An isolated native sequence Apo-2DcR polypeptide comprising amino acid residues 1 to 259 of Fig. 1A (SEQ ID NO: 1) or an isolated extracellular domain sequence of Apo-2DcR polypeptide comprising amino acid residues 1 to 161 of Fig. 1A (SEQ ID NO: 1).

**5.** The extracellular domain sequence of claim 4 which comprises:
(i) amino acid residues 1 to 165 of Fig. 1A (SEQ ID NO: 1); or
(ii) amino acid residues 1 to 236 of Fig. 1A (SEQ ID NO: 1); or
(iii) amino acid residues 1 to X, wherein X is any one of amino acid residues 161 to 236 of Figure 1A (SEQ ID NO: 1).

**6.** An isolated native sequence Apo-2DcR polypeptide comprising amino acid residues -40 to 259 of Fig. 1B (SEQ ID NO: 3).

**7.** A chimeric molecule comprising the Apo-2DcR polypeptide of any one of the preceding claims fused to a heterologous amino acid sequence.

**8.** The chimeric molecule of claim 7, wherein said heterologous amino acid sequence is an epitope tag sequence or an immunoglobulin sequence.

**9.** The chimeric molecule of claim 8, wherein said immunoglobulin sequence is an IgG.

**10.** The chimeric molecule of claim 9, wherein said extracellular domain sequence comprises amino acid residues 1 to 165 of Fig. 1A (SEQ ID NO:1).

**11.** An antibody which binds to the Apo-2DcR polypeptide of any one of claims 1 to 6.

**12.** The antibody of claim 11, wherein said antibody is a monoclonal antibody, a chimeric antibody, a human antibody, an IgG antibody, or a blocking antibody.

**13.** The antibody of claim 12 which comprises an antibody that, in addition to binding Apo-2DcR polypeptide, binds to another Apo-2 ligand receptor.

**14.** The antibody of claim 12 having the biological characteristics of the 4G3.9.9 monoclonal antibody, or the 6D10.9.7 monoclonal antibody, or the 1C5.24.1 monoclonal antibody.

**15.** The antibody of claim 12 wherein:
(i) the antibody binds to the same epitope as the epitope to which the 4G3.9.9 monoclonal antibody binds; or
(ii) the antibody binds to the same epitope as the epitope to which the 6D10.9.7 monoclonal antibody binds; or
(iii) the antibody binds to the same epitope as the epitope to which the 1C5.24.1 monoclonal antibody binds.

**16.** A hybridoma cell line which produces an antibody of claim 14 or claim 15.

**17.** An isolated nucleic acid comprising a nucleotide sequence encoding the Apo-2DcR polypeptide of any one of claims 1 to 6.

**18.** A vector comprising the nucleic acid of claim 17.

**19.** The vector of claim 18 operably linked to control sequences recognized by a host cell transformed with the vector.

**20.** A host cell comprising the vector of claim 18 or claim 19.

**21.** The host cell of claim 20 which is a CHO cell, a yeast cell or an *E. coli* cell.

**22.** A process of using a nucleic acid molecule encoding Apo-2DcR polypeptide to effect production of Apo-2DcR polypeptide comprising culturing the host cell of claim 20 or claim 21.

**23.** A composition comprising an Apo-2DcR polypeptide of any one of claims 1 to 6, a chimeric molecule of any one of claims 7 to 10, or an antibody of any one of claims 11 to 15 and a carrier.

**24.** A kit comprising a container and a composition contained within said container, wherein the composition includes an Apo-2DcR polypeptide of any one of claims 1 to 6, a chimeric molecule of any one of claims 7 to 10, or an antibody of any one of claims 11 to 15, and optionally instructions for using the composition in vivo or ex vivo.

**25.** A method of modulating apoptosis in mammalian cells comprising exposing said cells to an Apo-2DcR polypeptide of any one of claims 1 to 6.

**26.** The method of claim 25 wherein said cells are further exposed to Apo-2 ligand.
